# EUROPÄISCHE PATENTSCHRIFT

(11) **EP 1 797 107 B1**
(45) Veröffentlichungstag und Bekanntmachung des Hinweises auf die Patenterteilung: **06.08.2008**
(21) Anmeldenummer: 05797250.7
(22) Anmeldetag: 28.09.2005
(51) Int. Cl.: C07H 7/04

(54) **D-PYRANOSYL-SUBSTITUIERTE PHENYLE, DIESE VERBINDUNGEN ENTHALTENDE ARZNEIMITTEL, DEREN VERWENDUNG UND VERFAHREN ZU IHRER HERSTELLUNG**
D-PYRANOSYL-SUBSTITUTED PHENYLS, MEDICAMENTS CONTAINING SAID COMPOUNDS, USE THEREOF, AND METHOD FOR THE PRODUCTION THEREOF
PHENYLES D-PYRANOSYL-SUBSTITUES, AGENTS PHARMACEUTIQUES CONTENANT CES COMPOSES, LEUR UTILISATION ET PROCEDE POUR LES PREPARER

(30) Priorität: 01.10.2004 DE 102004048388
(43) Veröffentlichungstag der Anmeldung: 20.06.2007
(73) Patentinhaber: Boehringer Ingelheim International GmbH, 55216 Ingelheim (DE); Boehringer Ingelheim Pharma GmbH & Co. KG, 55216 Ingelheim (DE)
(72) Erfinder: ECKHARDT, Matthias, 88400 Biberach (DE); HIMMELSBACH, Frank, 88441 Mittelbiberach (DE); EICKELMANN, Peter, 88441 Mittelbiberach (DE); THOMAS, Leo, 88400 Biberach (DE); BARSOUMIAN, Edward Leon, Saratoga, CA 95070 (US)
(74) Vertreter: Hammann, Heinz
(86) Internationale Anmeldenummer: PCT/EP2005/010488
(87) Internationale Veröffentlichungsnummer: WO 2006/037537

(56) Entgegenhaltungen:
- WO-A-01/27128
- WO-A1-20/05092877
- WO-A2-02/083066
- US-A1- 2003 114 390

## Beschreibung

Gegenstand der vorliegenden Erfindung sind D-Pyranosyl-substituierte Phenyle der allgemeinen Formel I 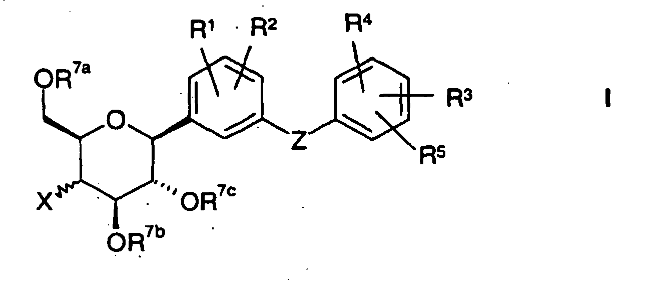 wobei die Reste R¹ bis R⁵, X, Z sowie R^{7a}, R^{7b}, R^{7c} nachfolgend definiert sind, einschließlich deren Tautomere, deren Stereoisomere, deren Gemische und deren Salze. Ein weiterer Gegenstand dieser Erfindung betrifft Arzneimittel enthaltend eine erfindungsgemäße Verbindung der Formel I sowie die Verwendung einer erfindungsgemäßen Verbindung zur Herstellung eines Arzneimittels zur Behandlung von Stoffwechselerkrankungen. Darüber hinaus sind Verfahren zur Herstellung eines Arzneimittels sowie einer erfindungsgemäßen Verbindung Gegenstand dieser Erfindung.

In der Literatur werden Verbindungen, die eine Hemmwirkung auf den natriumabhängigen Glucose-Cotransporter SGLT besitzen, zur Behandlung von Krankheiten, insbesondere von Diabetes vorgeschlagen.

Aus den internationalen Offenlegungsschriften WO 98/31697, WO 01/27128, US 2003/0114390, WO 02/083066 und WO 03/099836 sind Glucopyranosyl-substituierte Aromaten sowie deren Hersteliung und deren mögliche Aktivität als SGLT2-Inhibitoren bekannt.

In der Anmeldung WO 2004/052902 werden aromatische Fluorglycosid-Derivate wegen ihrer Wirkung auf SGLT als Antidiabetika vorgeschlagen. Bei den exemplarisch dargestellten Verbindungen handelt es sich ausschließlich um O-Phenylglycoside.

### Aufgabe der Erfindung

Der vorliegenden Erfindung liegt die Aufgabe zugrunde, neue Pyranosyl-substituierte Phenyle aufzuzeigen, insbesondere solche, die eine Aktivität bezüglich des natriumabhängigen Glucose-Cotransporters SGLT, insbesondere SGLT2 besitzen, Eine weitere Aufgabe der vorliegenden Erfindung besteht im Aufzeigen von Pyranosyl-substituierten Phenylen, die *in vitro* und/oder *in vivo* im Vergleich mit bekannten, strukturähnlichen Verbindungen eine erhöhte Hemmwirkung bezüglich des natriumabhängigen Glucose-Cotransporters SGLT2 besitzen und/oder verbesserte pharmakologische oder pharmakokinetische Eigenschaften aufweisen.

Ferner ist es eine Aufgabe der vorliegenden Erfindung, neue Arzneimittel bereit zu stellen, welche zur Prophylaxe und/oder Behandlung von Stoffwechselerkrankungen, insbesondere von Diabetes geeignet sind.

Ebenfalls eine Aufgabe dieser Erfindung ist es, ein Verfahren zur Herstellung der erfindungsgemäßen Verbindungen bereit zu stellen.

Weitere Aufgaben der vorliegenden Erfindung ergeben sich für den Fachmann unmittelbar aus den vorhergehenden und nachfolgenden Ausführungen.

### Gegenstand der Erfindung

Ein erster Gegenstand der vorliegenden Erfindung sind D-Pyranosyl-substituierte Phenyle der allgemeinen Formel I 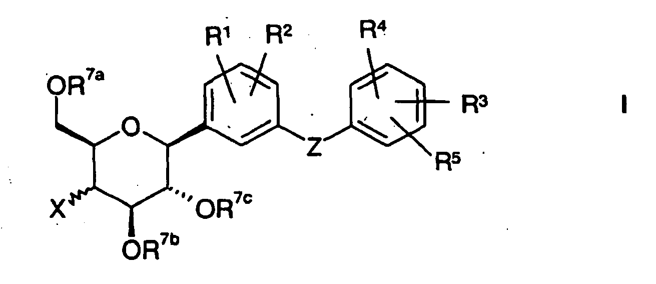 in der
- R¹: Wasserstoff, Fluor, Chlor, Brom, Iod, C₁₋₆-Alkyl, C₂₋₆-Alkinyl, C₂₋₆-Alkenyl, C₃₋₁₀-Cycloalkyl, C₃₋₁₀-Cycloalkyl-C₁-₃-alkyl, C₅₋₁₀-Cycloalkenyl, C₅₋₁₀-Cycloalkenyl-C₁₋₃-alkyl, C₁₋₄-Alkylcarbonyl, Arylcarbonyl, Heteroarylcarbonyl, Aminocarbonyl, C₁₋₄-Alkylaminocarbonyl, Di-(C₁₋₃-Alkyl)aminocarbonyl, Pyrrolidin-1-ylcarbonyl, Piperidin-1-ylcarbonyl, Morpholin-4-ylcarbonyl, Piperazin-1-ylcarbonyl, 4-(C₁₋₄-Alkyl)piperazin-1-ylcarbonyl, C₁₋₄-Alkoxycarbonyl, Amino, C₁₋₄-Alkylamino, Di-(C₁₋₃-alkyl)amino, Pyrrolidin-1-yl, Piperidin-1-yl, Morpholin-4-yl, Piperazin-1-yl, 4-(C₁₋₄-Alkyl)piperazin-1-yl, C₁₋₄-Alkylcarbonylamino, C₁₋₆-Alkyloxy, C₃₋₁₀-Cycloalkyloxy, C₅₋₁₀-Cycloalkenyloxy, Aryloxy, C₁₋₄-Alkylsulfanyl, C₁₋₄-Alkylsulfinyl, C₁₋₄-Alkylsulfonyl, C₃₋₁₀-Cycloalkylsulfanyl, C₃₋₁₀-Cycloalkylsulfinyl, C₃₋₁₀-Cycloalkylsulfonyl, C₅₋₁₀-Cycloalkenylsulfanyl, C₅₋₁₀-Cycloalkenylsulfinyl, C₅₋₁₀-Cycloalkenylsulfonyl, Arylsulfanyl, Arylsulfinyl, Arylsulfonyl, Hydroxy, Cyan oder Nitro bedeutet,
wobei Alkyl-, Alkenyl-, Alkinyl-, Cycloalkyl- und Cycloalkenyl-Reste teilweise oder vollständig fluoriert oder ein- oder zweifach mit gleichen oder verschiedenen Substituenten ausgewählt aus Chlor, Hydroxy, C₁₋₃-Alkoxy und C₁₋₃-Alkyl substituiert sein können, und
wobei in Cycloalkyl- und Cycloalkenyl-Resten eine oder zwei Methylengruppen unabhängig voneinander durch O, S, CO, SO oder SO₂ ersetzt sein können, und
wobei in N-Heterocycloalkyl-Resten eine Methylengruppe durch CO oder SO₂ ersetzt sein kann, und
- R²: Wasserstoff, Fluor, Chlor, Brom, Hydroxy, C₁₋₄-Alkyl, C₁₋₄-Alkoxy, Cyan oder Nitro, wobei Alkyl-Reste ein- oder mehrfach mit Fluor substituiert sein können, oder
für den Fall, dass R¹ und R² an zwei miteinander benachbarte C-Atome des Phenylrings gebunden sind, können R¹ und R² derart miteinander verbunden sein, dass R¹ und R² zusammen eine C₃₋₅-Alkylen-, C₃₋₅-Alkenylen- oder Butadienylen-Brücke bilden, die teilweise oder vollständig fluoriert oder ein- oder zweifach mit gleichen oder verschiedenen Substituenten ausgewählt aus Chlor, Hydroxy, C₁₋₃-Alkoxy und C₁₋₃-Alkyl substituiert sein kann, und in der eine oder zwei Methylengruppen unabhängig voneinander durch O, S, CO, SO, SO₂ oder NR^{N} ersetzt sein können, und in der im Falle einer Butadienylen-Brücke eine oder zwei Methingruppen durch ein N-Atom ersetzt sein können,
- R³: Wasserstoff, Fluor, Chlor, Brom, Iod, C₁₋₆-Alkyl, C₂₋₆-Alkinyl, C₂₋₆-Alkenyl, C₃₋₁₀-Cycloalkyl, C₃₋₁₀-Cycloalkyl-C₁₋₃-alkyl, C₅₋₁₀-Cycloalkenyl, C₅₋₁₀-Cycloalkenyl-C₁₋₃-alkyl, Aryl, Heteroaryl, C₁₋₄-Alkylcarbonyl, Arylcarbonyl, Heteroarylcarbonyl, Aminocarbonyl, C₁₋₄-Alkylaminocarbonyl, Di-(C₁₋₃-Alkyl)aminocarbonyl, Pyrrolidin-1-ylcarbonyl, Piperidin-1-ylcarbonyl, Morpholin-4-ylcarbonyl, Piperazin-1-ylcarbonyl, 4-(C₁₋₄-Alkyl)piperazin-1-ylcarbonyl, Hydroxycarbonyl, C₁₋₄-Alkoxycarbonyl, C₁₋₄-Alkylamino, Di-(C₁₋₃-alkyl)amino, Pyrrolidin-1-yl, Piperidin-1-yl, Morpholin-4-yl, Piperazin-1-yl, 4-(C₁₋₄-Alkyl)piperazin-1-yl, C₁₋₄-Alkylcarbonylamino, Arylcarbonylamino, Heteroarylcarbonylamino, C₁₋₄-Alkylsulfonylamino, Arylsulfonylamino, C₁₋₆-Alkoxy, C₃₋₇-Cycloalkyloxy, C₅₋₇-Cycloalkenyloxy, Aryloxy, Heteroaryloxy, C₁₋₄-Alkylsulfanyl, C₁₋₄-Alkylsulfinyl, C₁₋₄-Alkylsulfonyl, C₃₋₁₀-Cycloalkylsulfanyl, C₃₋₁₀-Cycloalkylsulfinyl, C₃₋₁₀-Cycloalkylsulfonyl, C₅₋₁₀-Cycloalkenylsulfanyl, C₅₋₁₀-Cycloalkenylsulfinyl, C₅₋₁₀-Cycloalkenylsulfonyl, Arylsulfanyl, Arylsulfinyl, Arylsulfonyl, Amino, Hydroxy, Cyan oder Nitro,
wobei Alkyl-, Alkenyl-, Alkinyl-, Cycloalkyl- und Cycloalkenyl-Reste teilweise oder vollständig fluoriert oder ein- oder zweifach mit gleichen oder verschiedenen Substituenten ausgewählt aus Chlor, Hydroxy, C₁₋₃-Alkoxy und C₁₋₃-Alkyl substituiert sein können, und
wobei in Cycloalkyl- und Cycloalkenyl-Resten eine oder zwei Methylengruppen unabhängig voneinander durch O, S; CO, SO oder SO₂ ersetzt sein können, und
wobei in N-Heterocycloalkyl-Resten eine Methylengruppe durch CO oder SO₂ ersetzt sein kann, und
- R⁴: Wasserstoff, Fluor, Chlor, Brom, Iod, Cyan, Nitro, C₁₋₃-Alkyl, C₁₋₃-Alkoxy, durch 1 bis 3 Fluoratome substituiertes Methyl- oder Methoxy, oder
für den Fall, dass R³ und R⁴ an zwei miteinander benachbarte C-Atome des Phenylrings gebunden sind, können R³ und R⁴ derart miteinander verbunden sein, dass R³ und R⁴ zusammen eine C₃₋₅-Alkylen-, C₃₋₅-Alkenylen- oder Butadienylen-Brücke bilden, die teilweise oder vollständig fluoriert oder ein- oder zweifach mit gleichen oder verschiedenen Substituenten ausgewählt aus Chlor, Hydroxy, C₁₋₃-Alkoxy und C₁₋₃-Alkyl substituiert sein kann, und in der eine oder zwei Methylengruppen unabhängig voneinander durch O, S, CO, SO, SO₂ oder NR^{N} ersetzt sein können, und in der im Falle einer Butadienylen-Brücke eine oder zwei Methingruppen durch ein N-Atom ersetzt sein können,
- R⁵: Wasserstoff, Fluor, Chlor, Brom, Iod, Cyan, Nitro, C₁₋₃-Alkyl, C₁₋₃-Alkoxy, durch 1 bis 3 Fluoratome substituiertes Methyl- oder Methoxy, und
- R^{N}: H, C₁₋₄-Alkyl oder C₁₋₄-Alkylcarbonyl,
- L: unabhängig voneinander ausgewählt aus der Gruppe bestehend aus Fluor, Chlor, Brom, Iod, C₁₋₃-Alkyl, Difluormethyl, Trifluormethyl, C₁₋₃-Alkoxy, Difluormethoxy, Trifluormethoxy und Cyan,
- R^{7a}, R^{7b}, R^{7c}: unabhängig voneinander eine Bedeutung ausgewählt aus der Gruppe Wasserstoff, (C₁₋₁₈-Alkyl)carbonyl, (C₁₋₁₈-Alkyl)oxycarbonyl, Arylcarbonyl und Aryl-(C₁₋₃-alkyl)carbonyl besitzen,
- X: Wasserstoff, Fluor, Chlor, Brom, Iod, C₁₋₆-Alkyl, C₂₋₆-Alkinyl, C₂₋₆-Alkenyl, C₃₋₇-Cycloalkyl-C₁₋₃-alkyl, C₅₋₇-cycloalkenyl-C₁₋₃-alkyl, Aryl-C₁₋₃-alkyl, Heteroaryl-C₁₋₃-alkyl, C₁₋₆-Alkoxy, C₂₋₆-Alkenyloxy, C₂₋₆-Alkinyloxy, C₃₋₇-Cycloalkyloxy, C₅₋₇-Cycloalkenyloxy, Aryloxy, Heteroaryloxy, C₃₋₇-Cycloalkyl-C₁₋₃-alkyloxy, C₅₋₇-Cycloalkenyl-C₁₋₃-alkyloxy, Aryl-C₁₋₃-alkyloxy, Heteroaryl-C₁₋₃-alkyloxy, Aminocarbonyl, C₁₋₄-Alkylaminocarbonyl-, Di-(C₁₋₃-Alkyl)aminocarbonyl, Hydroxycarbonyl, C₁₋₄-Alkoxycarbonyl, Aminocarbonyl-C₁₋₃-alkyl-, C₁₋₄-Alkylaminocarbonyl-C₁₋₃-alkyl, Di-(C₁₋₃-Alkyl)aminocarbonyl-C₁₋₃-alkyl, Hydroxycarbonyl-C₁₋₃-alkyl, C₁₋₄-Alkoxycarbonyl-C₁₋₃-alkyl, C₃₋₇-Cycloalkyloxy-C₁₋₃-alkyl, C₅₋₇-Cycloalkenyloxy-C₁₋₃-alkyl, Aryloxy-C₁₋₃-alkyl, Heteroaryloxy-C₁₋₃-alkyl, C₁₋₄-Alkylsulfonyloxy, Arylsulfonyloxy, Aryl-C₁₋₃-alkyl-sulfonyloxy oder Cyan,
wobei eine unmittelbar mit dem Pyranosering verbundene Methylengruppe durch NR^{N}, S, CO, SO oder SO₂ ersetzt sein kann, und
wobei Alkyl-, Alkenyl-, Alkinyl-, Cycloalkyl- und Cycloalkenyl-Reste teilweise oder vollständig fluoriert oder ein- oder zweifach mit gleichen oder verschiedenen Substituenten ausgewählt aus Chlor, Cyan, Hydroxy, C₁₋₃-Alkoxy, C₁₋₃-Alkylsulfanyl, -NH₂, -NHR^{N}, -NR^{N}(C₁₋₃-Alkyl) und C₁₋₃-Alkyl substituiert sein können, und
wobei in Cycloalkyl- und Cycloalkenyl-Resten eine oder zwei Methylengruppen unabhängig voneinander durch O, S, CO, SO oder SO₂ ersetzt sein können, und
- Z: Sauerstoff, Methylen, Dimethylmethylen, 1,1-Cyclopropylen, Difluormethylen oder Carbonyl bedeutet;
wobei unter den bei der Definition der vorstehend genannten Reste erwähnten Arylgruppen Phenyl- oder Naphthylgruppen zu verstehen sind, welche unabhängig voneinander ein- oder zweifach mit gleichen oder verschiedenen Resten L substituiert sein können; und
unter den bei der Definition der vorstehend erwähnten Reste erwähnten Heteroarylgruppen eine Pyrrolyl-, Furanyl-, Thienyl-, Imidazolyl-, Pyridyl-, Indolyl-, Benzofuranyl-, Benzothiophenyl-, Chinolinyl- oder Isochinolinylgruppe zu verstehen ist,
oder eine Pyrrolyl-, Furanyl-, Thienyl-, Imidazolyl- oder Pyridylgruppe zu verstehen ist, in der eine oder zwei Methingruppen durch Stickstoffatome ersetzt sind,
oder eine Indolyl-, Benzofuranyl-, Benzothiophenyl-, Chinolinyl- oder Isochinolinylgruppe zu verstehen ist, in der eine bis drei Methingruppen durch Stickstoffatome ersetzt sind,
wobei die vorstehend erwähnten Heteroarylgruppen unabhängig voneinander ein- oder zweifach mit gleichen oder verschiedenen Resten L substituiert sein können;
wobei unter dem bei der Definition der vorstehend erwähnten Reste erwähnten N-Heterocycloalkyl-Rest ein gesättigter carbocyclischer Ring, der eine Imino-Gruppe im Ring aufweist, zu verstehen ist, der eine weitere Imino-Gruppe oder ein O- oder S-Atom im Ring aufweisen kann, und
wobei, soweit nichts anderes erwähnt wurde, die vorstehend erwähnten Alkylgruppen geradkettig oder verzweigt sein können,
deren Tautomere, deren Stereoisomere, deren Gemische und deren Salze, insbesondere deren physiologisch verträglichen Salze.

Die erfindungsgemäßen Verbindungen der allgemeinen Formel I und ihre physiologisch verträglichen Salze weisen wertvolle pharmakologische Eigenschaften auf, insbesondere eine Hemmwirkung auf den natriumabhängigen Glucose-Cotransporter SGLT, insbesondere SGLT2. Ferner können erfindunsgemäße Verbindungen eine Hemmwirkung auf den natriumabhängigen Glucose-Cotransporter SGLT1 aufweisen. Verglichen mit einer möglichen Hemmwirkung auf SGLT1 hemmen die erfindungsgemäßen Verbindungen vorzugsweise selektiv SGLT2.

Gegenstand der vorliegenden Erfindung sind auch die physiologisch verträglichen Salze der erfindungsgemäßen Verbindungen mit anorganischen oder organischen Säuren.

Daher ist die Verwendung der erfindungsgemäßen Verbindungen, einschließlich der physiologisch verträglichen Salze, als Arzneimittel ebenfalls ein Gegenstand dieser Erfindung.

Ein weiterer Gegenstand dieser Erfindung sind Arzneimittel, enthaltend mindestens eine erfindungsgemäße Verbindung oder ein erfindungsgemäßes physiologisch verträgliches Salz neben gegebenenfalls einem oder mehreren inerten Trägerstoffen und/oder Verdünnungsmitteln.

Ebenfalls ein Gegenstand dieser Erfindung ist die Verwendung mindestens einer erfindungsgemäßen Verbindung oder eines physiologisch verträglichen Salzes solch einer Verbindung zur Herstellung eines Arzneimittels, das zur Behandlung oder Prophylaxe von Erkrankungen oder Zuständen geeignet ist, die durch Inhibierung des natriumabhängigen Glucose-Cotransporters SGLT, insbesondere SGLT2 beeinflussbar sind.

Ein weiterer Gegenstand dieser Erfindung ist die Verwendung mindestens einer erfindungsgemäßen Verbindung zur Herstellung eines Arzneimittels, das zur Behandlung von Stoffwechselerkrankungen geeignet ist.

Ein weiterer Gegenstand dieser Erfindung ist die Verwendung mindestens einer erfindungsgemäßen Verbindung zur Herstellung eines Arzneimittels zur Inhibition des natriumabhängigen Glucose-Cotransporters SGLT, insbesondere SGLT2.

Ferner ist ein Verfahren zur Herstellung eines erfindungsgemäßen Arzneimittels Gegenstand dieser Erfindung, dadurch gekennzeichnet, dass auf nicht-chemischem Wege eine erfindungsgemäße Verbindung in einen oder mehrere inerte Trägerstoffe und/oder Verdünnungsmittel eingearbeitet wird.

Gegenstand der vorliegenden Erfindung ist auch ein Verfahren zur Herstellung der erfindungsgemäßen Verbindungen der allgemeinen Formel I, dadurch gekennzeichnet, dass
a) zur Herstellung von Verbindungen der allgemeinen Formel I, die wie vor- und nachstehend definiert ist,
   eine Verbindung der allgemeinen Formel II 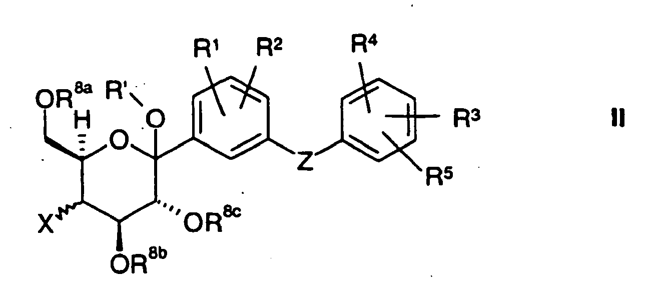 in der
   - R': H, C₁₋₄-Alkyl, (C₁₋₁₈-Alkyl)carbonyl, (C₁₋₁₈-Alkyl)oxycarbonyl, Arylcarbonyl oder Aryl(C₁₋₃-alkyl)-carbonyl bedeutet, worin die Alkyl- oder Arylgruppen ein- oder mehrfach mit Halogen substituiert sein können;
   - R^{8a}, R^{8b}, R^{8c}: unabhängig voneinander eine zuvor und nachstehend für die Reste R^{7a}, R^{7b}, R^{7c} angegebenen Bedeutungen aufweisen, eine Benzyl-Gruppe oder eine R^{a}R^{b}R^{c}Si-Gruppe oder eine Ketal- oder Acetalgruppe bedeuten, wobei jeweils zwei benachbarte Reste R^{8a}, R^{8b}, R^{8c} eine cyclische Ketal- oder Acetalgruppe oder mit zwei Sauerstoffatomen des Pyranose-Rings einen substituierten 2,3-Oxydioxan-Ring, insbesondere einen 2,3-Dimethyl-2,3-di(C₁₋₃-alkoxy)-1,4-dioxan-Ring, bilden können, und wobei Alkyl-, Aryl- und/oder Benzylgruppen ein- oder mehrfach mit Halogen, C₁₋₃-Alkyl oder C₁₋₃-Alkoxy substituiert sein können und Benzyl-Gruppen auch mit einer Di-(C₁₋₃-alkyl)amino-Gruppe substituiert sein können; und
   - R^{a}, R^{b}, R^{c}: unabhängig voneinander C₁₋₄-Alkyl, Aryl oder Aryl-C₁₋₃-alkyl bedeuten, worin die Aryl- oder Alkylgruppen ein- oder mehrfach mit Halogen substituiert sein können;
   wobei unter den bei der Definition der vorstehend genannten Reste erwähnten Arylgruppen Phenyl- oder Naphthylgruppen, vorzugsweise Phenylgruppen zu verstehen sind;
   und in der die Reste X und R¹ bis R⁵ und die Brücke Z wie vor- und nachstehend definiert sind;
   mit einem Reduktionsmittel in Gegenwart einer Säure umgesetzt wird, wobei die eventuell vorhandenen Schutzgruppen gleichzeitig oder nachträglich abgespalten werden; oder
b) zur Herstellung von Verbindungen der allgemeinen Formel I, in der R^{7a}, R^{7b} und R^{7c} Wasserstoff bedeuten,
   in einer Verbindung der allgemeinen Formel III 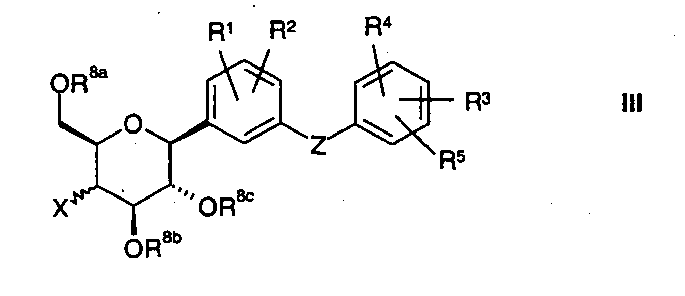 in der X, Z, R^{8a}, R^{8b}, R^{8c} sowie R¹ bis R⁵ wie zuvor und nachstehend definiert sind, wobei mindestens einer der Reste R^{8a}, R^{8b} und R^{8c} nicht Wasserstoff bedeutet,
   die nicht Wasserstoff bedeutenden Reste R^{8a}, R^{8b} bzw. R^{8c} entfernt werden, insbesondere hydrolysiert werden; und
   erforderlichenfalls ein bei den vorstehend beschriebenen Umsetzungen gemäß Verfahren a) oder b) verwendeter Schutzrest wieder abgespalten wird und/oder
   gewünschtenfalls eine so erhaltene Verbindung der allgemeinen Formel I selektiv an einer Hydroxygruppe derivatisiert oder diese substituiert wird und/oder
   gewünschtenfalls eine so erhaltene Verbindung der allgemeinen Formel I in ihre Stereoisomere aufgetrennt wird und/oder
   gewünschtenfalls eine so erhaltene Verbindung der allgemeinen Formel I in ihre Salze, insbesondere für die pharmazeutische Anwendung in ihre physiologisch verträglichen Salze, überführt wird.

### Detailierte Beschreibung der Erfindung

Sofern nicht anders angegeben besitzen die Gruppen, Reste und Substituenten, insbesondere R¹ bis R⁵, X, Z, L, R^{N}, R^{7a}, R^{7b}, R^{7c} die zuvor und nachfolgend angegebenen Bedeutungen.

Kommen Reste, Substituenten oder Gruppen in einer Verbindung mehrfach vor, so können diese eine gleiche oder verschiedene Bedeutungen aufweisen.

Die erfindungsgemäßen Verbindungen können β-D-glucopyranosyl-konfiguriert (Formel IA) oder β-D-galactopyranosyl-konfiguriert (Formel IB) sein.

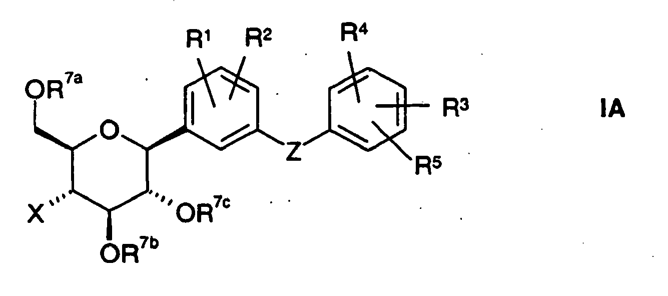

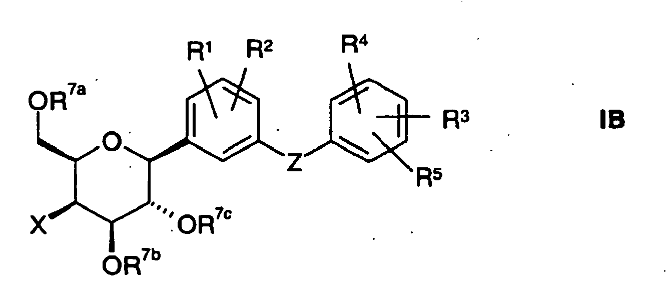

Im Hinblick auf eine Inhibierung von SGLT2 und vorzugsweise eine höhere Selektivität der Hemmwirkung auf SGLT2 verglichen mit SGLT1 sind Verbindungen der Formel IA bevorzugt.

Der Rest R³ steht vorzugsweise in meta- oder para-Position zur -Z-Brücke. Daher können erfindungsgemäße Verbindungen der allgemeinen Formel IA vorzugsweise gemäß der folgenden Formeln IA.1 und IA.2, insbesondere der Formel IA.2, beschrieben werden:

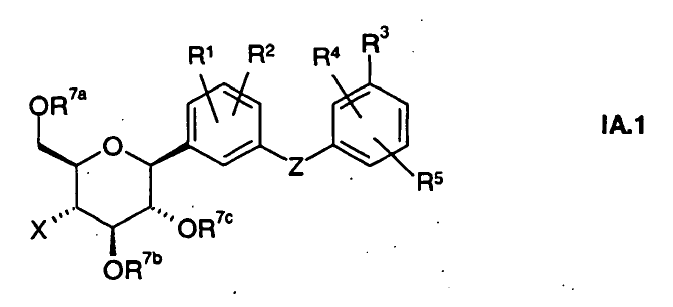

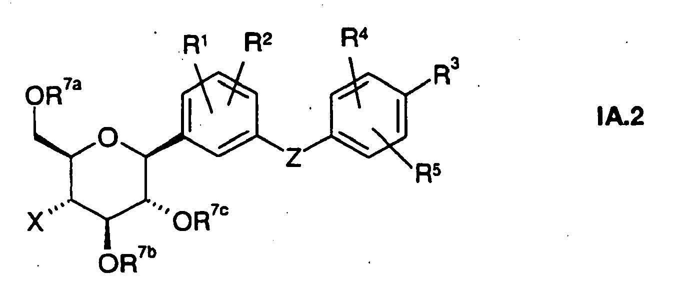

Die vorstehend und nachfolgend verwendete, beispielsweise in den Gruppen X, R¹ und R³ vorkommende Bezeichnung Aryl bedeutet vorzugsweise Phenyl. Gemäß der allgemeinen Definition und sofern nichts anderes angegeben ist, kann die Aryl-Gruppe, insbesondere die Phenylgruppe, ein- oder zweifach mit gleichen oder verschiedenen Resten L substituiert sein.

Die vorstehend und nachfolgend verwendete, beispielsweise in den Gruppen X, R¹ und R³ vorkommende Bezeichnung Heteroaryl bedeutet vorzugsweise Pyridinyl, Pyrimidinyl, Pyridazinyl, Pyrazinyl, Triazinyl, Imidazolyl, Pyrazolyl, Triazolyl, Tetrazolyl, Oxazolyl, Oxadiazolyl, Thiazolyl oder Thiadiazolyl. Gemäß der allgemeinen Definition und sofern nichts anderes angegeben ist, kann die Heteroaryl-Gruppe ein- oder zweifach mit gleichen oder verschiedenen Resten L substituiert sein.

Bevorzugt bedeutet R¹ Wasserstoff, Fluor, Chlor, Brom, Iod, C₁₋₆-Alkyl, C₂₋₆-Alkinyl, C₂₋₆-Alkenyl, C₃₋₇-Cycloalkyl, C₅₋₇-Cycloalkenyl, C₁₋₄-Alkylcarbonyl, Aminocarbonyl, C₁₋₄-Alkylaminocarbonyl, Di-(C₁₋₃-Alkyl)aminocarbonyl, C₁₋₄-Alkoxycarbonyl, C₁₋₄₋Alkylamino, Di-(C₁₋₃-alkyl)amino, Pyrrolidin-1-yl, Piperidin-1-yl, Morpholin-4-yl, C₁₋₄-Alkylcarbonylamino, C₁₋₆-Alkyloxy, C₃₋₇-Cycloalkyloxy, C₅₋₇-Cycloalkenyloxy, C₁₋₄-Alkylsulfanyl, C₁₋₄-Alkylsulfonyl, C₃₋₇-Cycloalkylsulfanyl, C₃₋₇-Cycloalkylsulfonyl, C₅₋₇-Cycloalkenylsulfanyl, C₅₋₇-Cycloalkenylsulfonyl, Hydroxy und Cyan,
wobei Alkyl-, Alkenyl-, Alkinyl-, Cycloalkyl- und Cycloalkenyl-Reste teilweise oder vollständig fluoriert oder ein- oder zweifach mit gleichen oder verschiedenen Substituenten ausgewählt aus Chlor, Hydroxy, C₁₋₃-Alkoxy und C₁₋₃-Alkyl substituiert sein können; und
wobei in Cycloalkyl- und Cycloalkenyl-Resten eine oder zwei Methylengruppen unabhängig voneinander durch O, S, CO, SO oder SO₂ ersetzt sein können, und
wobei in N-Heterocycloalkyl-Resten eine Methylengruppe durch CO oder SO₂ ersetzt sein kann.

Bedeutet die Gruppe R¹ einen Cycloalkyl- oder Cycloalkenyl-Rest, in dem eine oder zwei Methylengruppen unabhängig voneinander durch O, S, CO, SO oder SO₂ ersetzt sind, so sind bevorzugte Bedeutungen des Rests R¹ ausgewählt aus der Gruppe bestehend aus Tetrahydrofuranyl, Tetrahydrofuranonyl, Tetrahydrothienyl, Tetrahydropyranyl, Tetrahydropyranonyl, Dioxanyl und Trioxanyl.

Bedeutet die Gruppe R¹ einen N-Heterocycloalkyl-Rest, in dem eine Methylengruppe durch CO oder SO₂ substituiert ist, so sind bevorzugte Bedeutungen des Rests R¹ ausgewählt aus der Gruppe bestehend aus Pyrrolidinon, Piperidinon, Piperazinon und Morpholinon.

Besonders bevorzugt bedeutet R¹ Wasserstoff, Fluor, Chlor, Brom, Iod, C₁₋₆-Alkyl, C₂₋₆-Alkinyl, C₂₋₆-Alkenyl, C₃₋₇-Cycloalkyl, C₅₋₇-Cycloalkenyl, C₁₋₆-Alkyloxy, C₃₋₇-Cycloalkyloxy oder Cyan, wobei in Cycloalkyl- und Cycloalkenylgruppen eine oder zwei Methyleneinheiten unabhängig voneinander durch O oder CO ersetzt und Alkyl-, Alkenyl- und Alkinyl-Reste teilweise oder vollständig fluoriert sein können.

Beispiele der ganz besonders bevorzugten R¹ sind Wasserstoff, Fluor, Chlor, Brom, Methyl, Ethyl, Isopropyl, Trifluormethyl, Ethinyl, Methoxy, Cyclopentyloxy und Cyan.

Der Rest R³ bedeutet vorzugsweise Wasserstoff, Fluor, Chlor, Brom, C₁₋₆-Alkyl, C₂₋₆-Alkinyl, C₂₋₆-Alkenyl, C₃₋₇-Cycloalkyl, C₃₋₇-Cycloalkyl-methyl, C₅₋₇-Cycloalkenyl, C₃₋₇Cycloalkenyl-methyl, Aryl, Heteroaryl, C₁₋₄-Alkylcarbonyl, Aminocarbonyl, C₁₋₄-Alkylaminocarbonyl, Di-(C₁₋₃-Alkyl)-aminocarbonyl, C₁₋₄-Alkoxycarbonyl, Di-(C₁₋₃-alkyl)amino, Pyrrolidin-1-yl, Piperidin-1-yl, Morpholin-4-yl, C₁₋₄-Alkylcarbonylamino, C₁₋₆-Alkoxy, C₃₋₇-Cycloalkyloxy, C₅₋₇-Cycloalkenyloxy, Aryloxy, Heteroaryloxy, C₁₋₄-Alkylsulfanyl, C₁₋₄-Alkylsulfonyl, C₃₋₇-Cycloalkylsulfanyl, C₃₋₇-Cycloalkylsulfonyl, C₅₋₇-Cycloalkenylsulfanyl, C₅₋₇-Cycloalkenylsulfonyl, Hydroxy und Cyan,
wobei Alkyl-, Alkenyl-, Alkinyl-, Cycloalkyl- und Cycloalkenyl-Reste teilweise oder vollständig fluoriert oder ein- oder zweifach mit gleichen oder verschiedenen Substituenten ausgewählt aus Chlor, Hydroxy, C₁₋₃-Alkoxy und C₁₋₃-Alkyl substituiert sein können, und
wobei in Cycloalkyl- und Cycloalkenyl-Resten eine oder zwei Methylengruppen unabhängig voneinander durch O, S, CO, SO oder SO₂ ersetzt sein können, und
wobei in N-Heterocycloalkyl-Resten eine Methylengruppe durch CO oder SO₂ ersetzt sein kann,
wobei die Begriffe Aryl und Heteroaryl wie zuvor definiert sind und Aryl- und Heteroaryl-Gruppen unabhängig voneinander ein- oder zweifach mit gleichen oder verschiedenen Resten L substituiert sein können.

Bedeutet die Gruppe R³ einen Cycloalkyl- oder Cycloalkenyl-Rest, in dem eine oder zwei Methylengruppen unabhängig voneinander durch O, S, CO, SO oder SO₂ ersetzt sind, so sind bevorzugte Bedeutungen der Gruppe R³ ausgewählt aus der Gruppe bestehend aus Tetrahydrofuranyl, Tetrahydrofuranonyl, Tetrahydrothienyl, Tetrahydropyranyl, Tetrahydropyranonyl, Dioxanyl und Trioxanyl.

Bedeutet die Gruppe R³ einen N-Heterocycloalkyl-Rest, in dem eine Methylengruppe durch CO oder SO₂ ersetzt ist, so sind bevorzugte Bedeutungen des Rests R³ ausgewählt aus der Gruppe bestehend aus Pyrrolidinon, Piperidinon, Piperazinon und Morpholinon.

Besonders bevorzugte R³ sind C₁₋₆-Alkyl, C₂₋₆-Alkinyl, C₁₋₄-Alkyloxy, C₃₋₇-Cycloalkyl, C₃₋₇-Cycloalkyloxy und Hydroxy, wobei in den Cycloalkylgruppen eine oder zwei Methyleneinheiten unabhängig voneinander durch O oder CO ersetzt und Alkylreste teilweise oder vollständig fluoriert sein können.

Ganz besonders bevorzugte Reste R³ sind Methyl, Ethyl, Ethinyl, Isopropyl, Methoxy, Ethoxy, Isopropyloxy, Difluormethoxy, Cyclopentyloxy, Tetrahydrofuran-3-yloxy und Hydroxy.

Die Gruppe X besitzt eine der zuvor und nachfolgend angeführten Definitionen, wobei X keine Hydroxy-Gruppe und vorzugsweise keine Gruppe bedeutet, die unter physiologischen Bedingungen zu einer signifikanten Menge in eine Hydroxy-Gruppe umgewandelt wird. Insbesondere ist die Gruppe X keine Alkylcarbonyloxy-, (Het)Arylcarbonyloxy-, Alkyloxycarbonyloxy- oder (Het)Aryloxycarbonyloxy-Gruppe, wobei (Het)Aryl eine Aryl- oder Heteroarylgruppe bezeichnet.

Die Gruppe X bedeutet vorzugsweise Wasserstoff, Fluor, Chlor, C₁₋₆-Alkyl, C₂₋₆-Alkinyl, C₂₋₆-Alkenyl, C₃₋₇-Cycloalkyl-C₁₋₃-alkyl, C₅₋₇-Cycloalkenyl-C₁₋₃-alkyl, Aryl-C₁₋₃-alkyl, Heteroaryl-C₁₋₃-alkyl, C₁₋₆-Alkoxy, C₂₋₆-Alkenyloxy, C₂₋₆-Alkinyloxy, C₃₋₇-Cycloalkyloxy, C₅₋₇-Cycloalkenyloxy, Aryloxy, Heteroaryloxy, C₃₋₇-Cycloalkyl-C₁₋₃-alkyloxy, C₅₋₇-Cycloalkenyl-C₁₋₃-alkyloxy, Aryl-C₁₋₃-alkyloxy, Heteroaryl-C₁₋₃-alkyloxy, C₃₋₇-Cycloalkyloxy-C₁₋₃-alkyl, C₅₋₇-Cycloalkenyloxy-C₁₋₃-alkyl, Aryloxy-C₁₋₃-alkyl, Heteroaryloxy-C₁₋₃-alkyl, Aminocarbonyl, C₁₋₄-Alkylaminocarbonyl, C₁₋₄-Alkylcarbonylamino-, N-(C₁₋₅-Alkyl)-N-(C₁₋₄-alkylcarbonyl)-amino, Hydroxycarbonyl, C₁₋₄-Alkoxycarbonyl, C₁₋₆-Alkylsulfonyl, Aminocarbonyl-C₁₋₃-alkyl, C₁₋₄-Alkylaminocarbonyl-C₁₋₃-alkyl, Di-(C₁₋₃-Alkyl)aminocarbonyl-C₁₋₃-alkyl, C₁₋₄-Alkoxycarbonyl-C₁₋₃alkyl, Amino, C₁₋₅-Alkylamino, N-(C₁₋₅-Alkyl)-N-(C₁₋₃-alkyl)-amino, Mercapto oder Cyan,
wobei Alkyl-, Alkenyl-, Alkinyl-, Cycloalkyl- und Cycloalkenyl-Reste teilweise oder vollständig fluoriert oder ein- oder zweifach mit gleichen oder verschiedenen Substituenten ausgewählt aus Chlor, Cyan, Hydroxy, C₁₋₃-Alkoxy, C₁₋₃-Alkylsulfanyl, -NH₂, -NHA^{N}, -NR^{N}(C₁₋₃-Alkyl) und C₁₋₃-Alkyl substituiert sein können, und
wobei in den vorstehend genannten Cycloalkyl- und Cycloalkenyl-Resten eine oder zwei Methylengruppen unabhängig voneinander durch O, S, CO, SO oder SO₂ ersetzt sein können, und
wobei die Begriffe Aryl und Heteroaryl wie zuvor definiert sind und Aryl- und Heteroaryl-Gruppen unabhängig voneinander ein- oder zweifach mit gleichen oder verschiedenen Resten L substituiert sein können.

Die erfindungsgemäßen Verbindungen lassen sich formal entsprechend der unterschiedlichen Bedeutungen der Gruppe X in verschiedene Ausführungsformen aufteilen:
Gemäß einer ersten Ausführungsform bedeutet X vorzugsweise Wasserstoff, Chlor, Cyan, C₁₋₆-Alkyl, C₂₋₆-Alkinyl, C₂₋₆-Alkenyl, C₃₋₇-Cycloalkyl-C₁₋₃-alkyl, C₅₋₇-Cycloalkenyl-C₁₋₃-alkyl, Aryl-C₁₋₃-alkyl, Heteroaryl-C₁₋₃-alkyl, Aminocarbonyl, C₁₋₄-Alkylaminocarbonyl, Hydroxycarbonyl, C₁₋₄-Alkoxycarbonyl, C₁₋₆-Alkylsulfonyl, C₃₋₇-Cycloalkyloxy-C₁₋₃-alkyl, C₅₋₇-Cycloalkenyloxy-C₁₋₃-alkyl, Aryloxy-C₁₋₃-alkyl und Heteroaryloxy-C₁₋₃-alkyl,
wobei Alkyl-, Alkenyl-, Alkinyl-, Cycloalkyl- und Cycloalkenyl-Reste teilweise oder vollständig fluoriert oder ein- oder zweifach mit gleichen oder verschiedenen Substituenten ausgewählt aus Chlor, Cyan, Hydroxy, C₁₋₃-Alkylsulfanyl, NHR^{N}, C₁₋₃-Alkyl-NR^{N}, C₁₋₃-Alkoxy und C₁₋₃-Alkyl substituiert sein können, und wobei Methyl-Reste teilweise oder vollständig fluoriert oder einfach mit Chlor substituiert sein können, und
wobei in den vorstehend genannten Cycloalkyl- und Cycloalkenyl-Resten eine oder zwei Methylengruppen unabhängig voneinander durch O, S, CO, SO oder SO₂ ersetzt sein können, und
wobei die Begriffe Aryl und Heteroaryl wie zuvor definiert sind und Aryl- und Heteroaryl-Gruppen unabhängig voneinander ein- oder zweifach mit gleichen oder verschiedenen Resten L substituiert sein können.

Bedeutet die Gruppe X einen Cycloalkyl- oder Cycloalkenyl-Rest, in dem eine oder zwei Methylengruppen unabhängig voneinander durch O, S, CO, SO oder SO₂ ersetzt sind, so sind bevorzugte Bedeutungen der Gruppe X ausgewählt aus der Gruppe bestehend aus Tetrahydrofuranyl, Tetrahydrofuranonyl, Tetrahydrothienyl, Tetrahydropyranyl, Tetrahydropyranonyl, Dioxanyl und Trioxanyl.

Besonders bevorzugte Reste der Gruppe X sind Wasserstoff, Chlor, Cyan, C₁₋₆-Alkyl, C₂₋₆-Alkinyl, C₂₋₆-Alkenyl, C₁₋₆-Alkylsulfonyl, Hydroxycarbonyl, C₁₋₄-Alkoxycarbonyl, Aminocarbonyl und C₁₋₄-Alkylaminocarbonyl,
wobei Alkyl-Reste ein- oder mehrfach fluoriert oder einfach mit Hydroxy oder Cyan substituiert sein können.

Ganz besonders bevorzugte Reste X sind Wasserstoff, Chlor, Cyan, Methyl, Ethyl, Propyl, Hydroxymethyl, Prop-2-enyl, Prop-2-inyl, Methylsulfonyl, Aminocarbonyl, Methylaminocarbonyl, Hydroxycarbonyl und Methoxycarbonyl.

Eine Auswahl der ganz besonders bevorzugten Gruppen X ist Wasserstoff, Chlor, Methyl, Hydroxymethyl und Ethyl.

Gemäß einer zweiten Ausführungsform bedeutet X vorzugsweise C₁₋₆-Alkoxy, C₂₋₆-Alkenyloxy, C₂₋₆-Alkinyloxy, C₃₋₇-Cycloalkyloxy, C₅₋₇-Cycloalkenyloxy, Aryloxy, Aryl-C₁₋₃-alkyloxy oder Heteroaryloxy,
wobei die vorstehend genannten Alkoxy-, Cycloalkyl- und Cycloalkenyl-Reste teilweise oder vollständig fluoriert oder ein- oder zweifach mit gleichen oder verschiedenen Substituenten ausgewählt aus Hydroxy, C₁₋₃-Alkoxy und C₁₋₃-Alkyl substituiert sein können, und
wobei in den vorstehend genannten Cycloalkyl- und Cycloalkenyl-Resten eine oder zwei Methylengruppen unabhängig voneinander durch O, S, CO, SO oder SO₂ ersetzt sein können, und
wobei die Begriffe Aryl und Heteroaryl wie zuvor definiert sind und Aryl- und Heteroaryl-Gruppen unabhängig voneinander ein- oder zweifach mit gleichen oder verschiedenen Resten L substituiert sein können.

Gemäß dieser Ausführungsform bevorzugte Bedeutungen des Rests X sind C₁₋₆-Alkyloxy, C₂₋₆-Alkenyloxy, C₂₋₆-Alkinyloxy, C₃₋₇-Cycloalkyloxy, Aryl-C₁₋₃-alkyloxy und Aryloxy, wobei unter Aryl eine Phenyl- oder Naphthylgruppe, insbesondere Phenyl zu verstehen ist, welche ein- oder zweifach mit gleichen oder verschiedenen Substituenten L substituiert sein kann.

Besonders bevorzugte Bedeutungen des Rests X sind hierbei Methoxy und Ethoxy.

Gemäß einer dritten Ausführungsform bedeutet X vorzugsweise Mercapto, C₁₋₅-Alkylsulfanyl, C₂₋₅-Alkenylsulfanyl, C₂₋₅-Alkinyisulfanyl, C₃₋₇-Cycloalkylsulfanyl, C₅₋₇-Cycloalkenylsulfanyl, Arylsulfanyl oder Heteroarylsulfanyl,
wobei die vorstehend genannten Alkoxy-, Cycloalkyl- und Cycloalkenyl-Reste teilweise oder vollständig fluoriert oder ein- oder zweifach mit gleichen oder verschiedenen Substituenten ausgewählt aus Hydroxy, C₁₋₃-Alkoxy und C₁₋₃-Alkyl substituiert sein können, und
wobei in den vorstehend genannten Cycloalkyl- und Cycloalkenyl-Resten eine oder zwei Methylengruppen unabhängig voneinander durch O, S, CO, SO oder SO₂ ersetzt sein können, und
wobei die Begriffe Aryl und Heteroaryl wie zuvor definiert sind und Aryl- und Heteroaryl-Gruppen unabhängig voneinander ein- oder zweifach mit gleichen oder verschiedenen Resten L substituiert sein können.

Gemäß dieser Ausführungsform bevorzugte Bedeutungen des Rests X sind Mercapto, C₁₋₅-Alkylsulfanyl, C₂₋₅-Alkenylsulfanyl, C₂₋₅-Alkinylsulfanyl, C₃₋₇-Cycloalkylsulfanyl und Arylsulfanyl, wobei unter Aryl eine Phenyl- oder Naphthylgruppe, insbesondere Phenyl zu verstehen ist, welche ein- oder zweifach mit gleichen oder verschiedenen Substituenten L substituiert sein kann.

Besonders bevorzugte Bedeutungen des Rests X sind hierbei Mercapto, Methylsulfanyl und Ethylsulfanyl.

Gemäß einer vierten Ausführungsform bedeutet X vorzugsweise Amino, C₁₋₅-Alkylamino, N-(C₁₋₅-Alkyl)-N-(C₁₋₃-alkyl)-amino, C₁₋₄-Alkylcarbonylamino, N-(C₁₋₅-Alkyl)-N-(C₁₋₄-alkylcarbonyl)-amino, C₂₋₅-Alkenylamino, C₂₋₅-Alkinylamino, C₃₋₇-Cycloalkylamino, C₅₋₇-Cycloalkenylamino, Arylamino oder Heteroarylamino,
wobei die vorstehend genannten Alkoxy-, Cycloalkyl- und Cycloalkenyl-Reste teilweise oder vollständig fluoriert oder ein- oder zweifach mit gleichen oder verschiedenen Substituenten ausgewählt aus Hydroxy, C₁₋₃-Alkoxy und C₁₋₃-Alkyl substituiert sein können, und
wobei in den vorstehend genannten Cycloalkyl- und Cycloalkenyl-Resten eine oder zwei Methylengruppen unabhängig voneinander durch O, S, CO, SO oder SO₂ ersetzt sein können, und
wobei die Begriffe Aryl und Heteroaryl wie zuvor definiert sind und Aryl- und Heteroaryl-Gruppen unabhängig voneinander ein- oder zweifach mit gleichen oder verschiedenen Resten L substituiert sein können.

Gemäß dieser Ausführungsform bevorzugte Bedeutungen des Rests X sind Amino, C₁₋₅-Alkylamino, N-(C₁₋₅-Alkyl)-N-(C₁₋₃-alkyl)-amino, C₁₋₄-Alkylcarbonylamino, N-(C₁₋₅-Alkyl)-N-(C₁₋₄-alkylcarbonyl)-amino und Arylamino, wobei unter Aryl eine Phenyl- oder Naphthylgruppe, insbesondere Phenyl zu verstehen ist, welche ein- oder zweifach mit gleichen oder verschiedenen Substituenten L substituiert sein kann.

Besonders bevorzugte Bedeutungen des Rests X sind hierbei Amino, Methylamino, Dimethylamino und Methylcarbonylamino.

Gemäß einer fünften Ausführungsform bedeutet X Fluor.

Gemäß einer sechsten Ausführungsform bedeutet X vorzugsweise Brom, Iod, C₁₋₆-Alkylsulfonyloxy, Arylsulfonyloxy oder Aryl-C₁₋₃-alkyl-sulfonyloxy,
wobei die vorstehend genannten Alkyl-Reste teilweise oder vollständig fluoriert oder ein- oder zweifach chloriert sein können und wobei die vorstehend genannten Aryl-Gruppen ein- oder zweifach mit gleichen oder verschiedenen Resten L substituiert sein können. L ist vorzugsweise ausgewählt aus der Gruppe Fluor, Chlor, Brom, Iod, C₁₋₃-Alkyl, Difluormethyl, Trifluormethyl und Cyan:
Die Verbindungen gemäß dieser sechsten Ausführungsform eignen sich über ihre beschriebene pharmazeutische Wirkung hinaus insbesondere als Zwischenprodukte in der Synthese von Verbindungen mit SGLT, vorzugsweise SGLT2 inhibierender Wirkung, insbesondere in der Synthese weiterer erfindungsgemäßer Verbindungen. Im Fall der Verwendung als Zwischenprodukt zur Synthese von Verbindungen der allgemeinen Formel I können die hier beschriebenen Reste X auch mit der gegenüber dem gewünschten Produkt invertierten Stereochemie an den Pyranosering angeknüpft sein.

Besonders bevorzugte Reste X gemäß dieser sechsten Ausführungsform sind Brom, Iod, C₁₋₄-Alkylsulfonyloxy, Phenylsulfonyloxy oder Phenylmethylsulfonyloxy, wobei die vorstehend genannten Alkyl-Reste teilweise oder vollständig fluoriert sein können und wobei die vorstehend genannten Phenyl-Gruppen ein- oder zweifach mit gleichen oder verschiedenen Resten L substituiert sein können. L ist vorzugsweise ausgewählt ist aus der Gruppe Fluor, Chlor, Brom und Methyl.

Ganz besonders bevorzugt ist hierbei X in der Bedeutung Trifluormethylsulfonyloxy oder Iod.

Diejenigen erfindungsgemäßen Verbindungen sind bevorzugt, die eine Gruppe X gemäß der zuvor beschriebenen ersten, zweiten, dritten, vierten und fünften Ausführungsform aufweisen, insbesondere die eine Gruppe X gemäß der darin als bevorzugt angegebenen Bedeutungen aufweisen. Ganz besonders bevorzugt sind hierbei die erste, zweite, vierte und fünfte Ausführungsform.

Sind in den Resten oder Gruppen X, R¹ oder R³ Cycloalkyl- oder Cycloalkenyl-Ringe vorhanden, in denen zwei Methylengruppen durch O oder S ersetzt sind oder durch CO, SO oder SO₂ ersetzt sind, so sind diese Methylengruppen vorzugsweise nicht unmittelbar miteinander verbunden. Sind jedoch zwei Methylengruppen durch O und CO ersetzt, so können diese unmittelbar miteinander verbunden sein, so dass eine Carboxy-Gruppe gebildet wird. Für den Fall, dass X, R¹ oder R³ eine Cycloalkyl- oder Cycloalkenyl-Gruppe mit einer oder zwei erfindungsgemäß ersetzten Methylengruppen ist, so bedeutet die betreffende Gruppe X, R¹ bzw. R³ vorzugsweise eine Cycloalkyl- oder Cycloalkenyl-Gruppe, in der eine Methylengruppe durch O, S, CO, SO oder SO₂ substituiert oder eine Ethylengruppe durch -O-CO- oder -CO-O- substituiert ist.

Nachfolgend werden Bedeutungen weiterer Reste und Substituenten angegeben, die gemäß der allgemeinen Formel 1, insbesondere der Formeln IA und IB als auch gemäß der zuvor beschriebenen Ausführungsformen als bevorzugt anzusehen sind:
Bevorzugte Bedeutungen des Rests R² sind Wasserstoff, Fluor, Chlor, Brom, Methyl, Hydroxy, Methoxy, Ethoxy, Trifluormethoxy, Cyan, Nitro und durch 1 bis 3 Fluoratome substituiertes Methyl.

Besonders bevorzugte Bedeutungen des Rests R² sind Wasserstoff, Fluor, Hydroxy, Methoxy, Ethoxy und Methyl, insbesondere Wasserstoff und Methyl.

Für den Fall, dass R¹ und R² an zwei miteinander benachbarte C-Atome des Phenylrings gebunden sind, können R¹ und R² derart miteinander verbunden sein, dass R¹ und R² zusammen vorzugsweise eine C₃₋₄-Alkylen-Brücke bilden, in der eine oder zwei Methyleneinheiten unabhängig voneinander durch O, NR^{N} oder CO ersetzt sein können oder eine Butadienylen-Brücke, in der eine Methin-Gruppe durch ein Stickstoffatom ersetzt sein kann, wobei die aufgeführten Brückengruppen teilweise oder vollständig fluoriert oder ein- oder zweifach mit gleichen oder verschiedenen Substituenten ausgewählt aus Chlor, Hydroxy, C₁₋₃-Alkoxy und C₁₋₃-Alkyl substituiert sein können. Bevorzugt bilden hierbei die miteinander verbundenen Reste R¹ und R² zusammen mit dem Phenylring, mit dem diese verbunden sind, ein bicyclisches Ringsystem ausgewählt aus Indan, Dihydroindol, Dihydrobenzofuran, Tetrahydrochinolin, Dihydrochinolinon, Tetrahydroisochinolin, Dihydroisochinolinon, Tetrahydronaphthalin, Naphthalin, Chinolin oder Isochinolin.

Bevorzugte Bedeutungen des Rests R⁴ sind Wasserstoff und Fluor, insbesondere Wasserstoff.

Für den Fall, dass R³ und R⁴ an zwei unmittelbar miteinander benachbarte C-Atome des Phenylrings gebunden sind, können R³ und R⁴ derart miteinander verbunden sein, dass R³ und R⁴ zusammen vorzugsweise eine C₃₋₄-Alkylen-Brücke bilden, in der ein oder zwei Methyleneinheiten unabhängig voneinander durch O, NR^{N} oder CO ersetzt sein können oder eine Butadienylen-Brücke, in der eine Methin-Gruppe durch ein Stickstoffatom ersetzt sein kann, wobei die aufgeführten Brückengruppen teilweise oder vollständig fluoriert oder ein- oder zweifach mit gleichen oder verschiedenen Substituenten ausgewählt aus Chlor, Hydroxy, C₁₋₃-Alkoxy und C₁₋₃-Alkyl substituiert sein können. Bevorzugt bilden hierbei die miteinander verbundenen Reste R³ und R⁴ zusammen mit dem Phenylring, mit dem diese verbunden sind, ein bicyclisches Ringsystem ausgewählt aus Indan, Dihydroindol, Dihydrobenzofuran, Tetrahydrochinolin, Dihydrochinolinon, Tetrahydroisochinolin, Dihydroisochinolinon, Tetrahydronaphthalin, Naphthalin, Chinolin oder Isochinolin.

Bevorzugte Bedeutungen des Rests R⁵ sind Wasserstoff und Fluor, insbesondere Wasserstoff.

Bevorzugte Bedeutungen des Rests Z sind Sauerstoff und Methylen, insbesondere Methylen.

Die Substituenten R^{7a}, R^{7b}, R^{7c} bedeuten unabhängig voneinander vorzugsweise Wasserstoff, (C₁₋₈-Alkyl)oxycarbonyl-, (C₁₋₁₈-Alkyl)carbonyl, Benzoyl, insbesondere Wasserstoff oder (C₁₋₆-Alkyl)oxycarbonyl-, (C₁₋₈-Alkyl)carbonyl, besonders bevorzugt Wasserstoff, Methoxycarbonyl, Ethoxycarbonyl, Methylcarbonyl oder Ethylcarbonyl. Ganz besonders bevorzugt bedeuten R^{7a}, R^{7b} und R^{7c} Wasserstoff.

Die Verbindungen der Formel I, in denen R^{7a}, R^{7b} und R^{7c} eine erfindungsgemäße, von Wasserstoff verschiedene Bedeutung aufweisen, beispielsweise C₁₋₈-Alkylcarbonyl, eignen sich bevorzugt als Zwischenprodukte bei der Synthese von Verbindungen der Formel I in denen R^{7a}, R^{7b} und R^{7c} Wasserstoff bedeuten.

Die Substituenten L sind unabhängig voneinander vorzugsweise ausgewählt aus der Gruppe bestehend aus Fluor, Chlor, Brom, C₁₋₃-Alkyl, Difluormethyl, Trifluormethyl, C₁₋₃-Alkoxy, Difluormethoxy, Trifluormethoxy und Cyan, besonders bevorzugt aus der Gruppe bestehend aus Fluor, Chlor, Methyl, Trifluormethyl, Methoxy und Difluormethoxy. Falls der Substituent L mit einem N-Atom verbunden ist, sind bevorzugte Bedeutungen L ausgewählt aus C₁₋₃-Alkyl, Difluormethyl und Trifluormethyl.

Besonders bevorzugte Verbindungen der allgemeinen Formel IA sind ausgewählt aus der Gruppe der Formeln IA.2a bis IA.2d, insbesondere der Formel IA.2c: 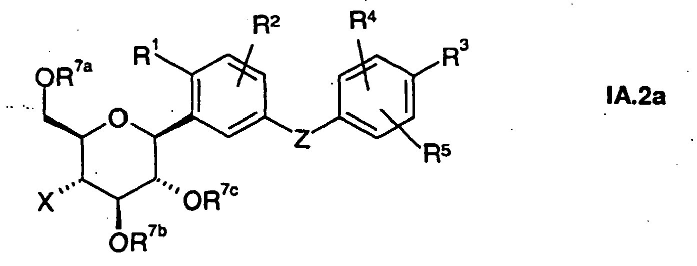 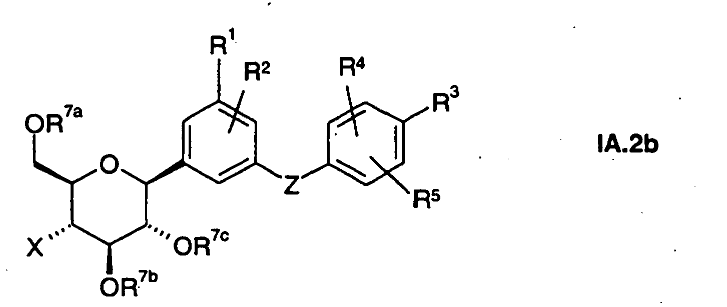 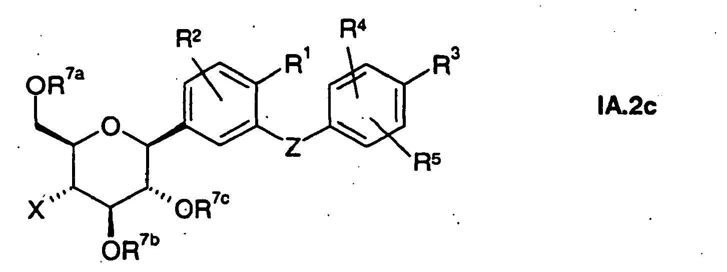 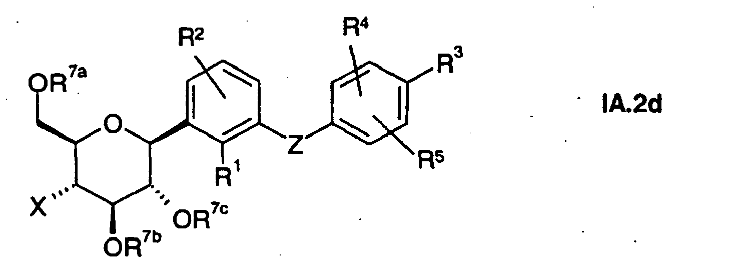 in denen R¹ bis R⁵, X, Z, R^{7a}, R^{7b}, R^{7c} wie zuvor definiert sind.

Ganz besonders bevorzugt sind diejenigen Verbindungen der Formel I, insbesondere der Formel IA oder IB und bezüglich der Formel IA insbesondere der Formeln IA.2a, IA.2b, IA.2c und IA.2d, insbesondere der Formel IA.2c, in denen die Reste R¹ bis R⁵, X, Z, R^{7a} R^{7b}, R^{7c} die zuvor als bevorzugt angegebenen Bedeutungen aufweisen, insbesondere in denen
- R¹: Wasserstoff, Fluor, Chlor, Brom, C₁₋₈-Alkyl, C₂₋₆-Alkinyl, C₂₋₆-Alkenyl, C₃₋₇-Cycloalkyl, C₅₋₇-Cycloalkenyl, C₁₋₆-Alkyloxy, C₃₋₇-Cycloalkyloxy oder Cyan bedeutet, wobei in Cycloalkyl- und Cycloalkenylgruppen ein oder zwei Methyleneinheiten unabhängig voneinander durch O oder CO ersetzt und Alkyl-, Alkenyl- und Alkinyl-Reste teilweise oder vollständig fluoriert sein können, besonders bevorzugt Wasserstoff, Fluor, Chlor, Brom, Methyl, Ethyl, Isopropyl, Trifluormethyl, Ethinyl, Methoxy, Cyclopentyloxy oder Cyan bedeutet, und
- R³: Wasserstoff, C₁₋₆-Alkyl, C₂₋₆-Alkinyl, C₁₋₄-Alkyloxy, C₃₋₇-Cycloalkyl, C₃₋₇-Cycloalkyloxy oder Hydroxy bedeutet, wobei in den Cycloalkylgruppen ein oder zwei Methyleneinheiten unabhängig voneinander durch O oder CO ersetzt und Alkylreste teilweise oder vollständig fluoriert sein können; besonders bevorzugt Methyl, Ethyl, Ethinyl, Isopropyl, Methoxy, Ethoxy, Isopropyloxy, Difluormethoxy, Cyclopentyloxy, Tetrahydro-furan-3-yloxy oder Hydroxy bedeutet, und
- X: gemäß einer ersten Ausführungsform Wasserstoff, Chlor, Cyan, C₁₋₆-Alkyl, C₂₋₆-Alkinyl, C₂₋₆-Alkenyl, C₁₋₄-Alkylcarbonyl, Hydroxycarbonyl, C₁₋₄-Alkoxycarbonyl, Aminocarbonyl, C₁₋₄-Alkylaminocarbonyl oder C₁₋₆-Alkylsulfonyl bedeutet, wobei Alkyl-Reste ein- oder mehrfach fluoriert oder einfach mit Hydroxy oder Cyan substituiert sein können; besonders bevorzugt Wasserstoff, Chlor, Cyan, Methyl, Ethyl, Hydroxymethyl, Prop-2-enyl, Prop-2-inyl, Methylsulfonyl, Aminocarbonyl, Methylaminocarbonyl, Hydroxycarbonyl oder Methoxycarbonyl bedeutet; oder
gemäß einer zweiten Ausführungsform C₁₋₅-Alkyloxy, C₂₋₅-Alkenyloxy, C₂₋₅-Alkinyloxy, C₃₋₇-Cycloalkyloxy, Aryl-C₁₋₃-alkyloxy oder Aryloxy bedeutet, wobei unter Aryl eine Phenyl- oder Naphthylgruppe, insbesondere Phenyl zu verstehen ist, welche ein- oder zweifach mit gleichen oder verschiedenen Substituenten L substituiert sein kann; besonders bevorzugt Ethoxy oder Methoxy bedeutet, oder
gemäß einer dritten Ausführungsform Mercapto, C₁₋₅-Alkylsulfanyl, C₂₋₅-Alkenylsulfanyl, C₂₋₅-Alkinylsulfanyl, C₃₋₇-Cycloalkylsulfanyl oder Arylsulfanyl bedeutet, wobei unter Aryl eine Phenyl- oder Naphthylgruppe, insbesondere Phenyl zu verstehen ist, welche ein- oder zweifach mit gleichen oder verschiedenen Substituenten L substituiert sein kann; besonders bevorzugt Mercapto, Ethylsulfanyl oder Methylsulfanyl bedeutet, oder
gemäß einer vierten Ausführungsform Amino, C₁₋₅-Alkylamino, N-(C₁₋₅-Alkyl)-N-(C₁₋₃-alkyl)-amino, C₁₋₄-Alkylcarbonylamino, N-(C₁₋₅-Alkyl)-N-(C₁₋₄-alkylcarbonyl)-amino oder Arylamino bedeutet, wobei unter Aryl eine Phenyl- oder Naphthylgruppe, insbesondere Phenyl zu verstehen ist, welche ein- oder zweifach mit gleichen oder verschiedenen Substituenten L substituiert sein kann; besonders bevorzugt Amino, Methylamino, Dimethylamino oder Methylcarbonylamino bedeutet, oder
gemäß einer fünften Ausführungsform Fluor bedeutet; und
- R²: Wasserstoff, Fluor, Chlor, Brom, Methyl, Hydroxy, Methoxy, Ethoxy, Trifluormethoxy, Cyan, Nitro oder durch 1 bis 3 Fluoratome substituiertes Methyl bedeutet, besonders bevorzugt Wasserstoff, Fluor, Hydroxy, Methoxy, Ethoxy oder Methyl, insbesondere Wasserstoff oder Methyl bedeutet, und
- R⁴: Wasserstoff oder Fluor, insbesondere Wasserstoff bedeutet, und
- R⁵: Wasserstoff oder Fluor, insbesondere Wasserstoff bedeutet, und
- Z: Sauerstoff oder Methylen, insbesondere Methylen bedeutet, und
- R^{7a}, R^{7b}, R^{7c}: unabhängig voneinander Wasserstoff, (C₁₋₈-Alkyl)oxycarbonyl-, (C₁₋₁₈-Alkyl)carbonyl oder Benzoyl, insbesondere Wasserstoff oder (C₁₋₆-Alkyl)oxycarbonyl-, (C₁₋₈-Alkyl)carbonyl bedeuten, besonders bevorzugt Wasserstoff, Methoxycarbonyl, Ethoxycarbonyl, Methylcarbonyl oder Ethylcarbonyl, ganz besonders bevorzugt Wasserstoff bedeuten, und
- L: unabhängig voneinander Fluor, Chlor, Brom, C₁₋₃-Alkyl, Difluormethyl, Trifluormethyl, C₁₋₃-Alkoxy, Difluormethoxy, Trifluormethoxy und Cyan bedeuten und falls der Substituent L mit einem N-Atom verbunden ist C₁₋₃-Alkyl, Difluormethyl oder Trifluormethyl bedeutet;
einschließlich deren Tautomere, deren Stereoisomere, deren Gemische und deren Salze, insbesondere deren physiologisch verträglichen Salze.

Gemäß einer Variante der zuvor angeführten Ausführungsformen sind diejenigen Verbindungen auch bevorzugt, in denen die Phenylgruppe, die den Substituenten R³ trägt, mindestens einen weiteren, von Wasserstoff verschiedenen Substituenten R⁴ und/oder R⁵ aufweist. Nach dieser Variante sind diejenigen Verbindungen auch bevorzugt, die einen Substituenten R⁴ in der Bedeutung Fluor aufweisen.

Der Phenylrest, der den Substituenten R³ trägt, ist vorzugsweise maximal zweifach fluoriert.

Besonders bevorzugte Verbindungen der allgemeinen Formel I sind ausgewählt aus der Gruppe:
(a) 1-Chlor-2-(4-methoxy-benzyl)-4-(4-O-methyl-β-D-glucopyranos-1-yl)-benzol,
(b) 1-Chlor-2-(4-methoxy-benzyl)-4-(4-desoxy-β-D-glucopyranos-1-yl)-benzol,
(c) 1-Chlor-2-(4-methoxy-benzyl)-4-(4-O-ethyl-β-D-glucopyranos-1-yl)-benzol,
(d) 1-Chlor-2-(4-methoxy-benzyl)-4-(4-desoxy-4-fluor-β-D-glucopyranos-1-yl)-benzol,
(e) 1-Chlor-2-(4-methoxy-benzyl)-4-(4-desoxy-4-fluor-β-D-galactopyranos-1-yl)-benzol,
einschließlich deren Tautomere, deren Stereoisomere und deren Gemische.

Im folgenden werden Begriffe, die zuvor und nachfolgend zur Beschreibung der erfindungsgemäßen Verbindungen verwendet werden, näher definiert.

Die Bezeichnung Halogen bezeichnet ein Atom ausgewählt aus der Gruppe bestehend aus F, Cl, Br und I, insbesondere F, Cl und Br.

Die austauschbar verwendeten Bezeichnungen "teilweise oder vollständig fluoriert sein kann" und "ein oder mehrfach fluoriert sein kann" bedeuten, dass die so bezeichnete Gruppe nicht fluoriert ist oder einen oder mehrere Fluor-Substituenten aufweist, wobei dies auch die vollständige Fluorierung der bezeichneten Gruppe mit einschließt.

Die Bezeichnung C₁₋ₙ-Alkyl, wobei n einen Wert von 1 bis 18 besitzen kann, bedeutet eine gesättigte, verzweigte oder unverzweigte Kohlenwasserstoffgruppe mit 1 bis n C-Atomen. Beispiele solcher Gruppen umfassen Methyl, Ethyl, n-Propyl, iso-Propyl, Butyl, iso-Butyl, sec-Butyl, tert-Butyl, n-Pentyl, iso-Pentyl, neo-Pentyl, tert-Pentyl, n-Hexyl, iso-Hexyl, etc..

Die Bezeichnung Methylen bedeutet eine -CH₂-Gruppe und die Bezeichnung Methin bedeutet eine CH-Gruppe.

Die Bezeichnung "Butadienylen" bedeutet die Gruppe

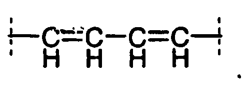

Der Begriff C₂₋ₙ-Alkinyl, wobei n einen Wert von 3 bis 6 besitzt, bezeichnet eine verzweigte oder unverzweigte Kohlenwasserstoffgruppe mit 2 bis n C-Atomen und einer C≡C-Dreifachbindung. Beispiele solcher Gruppen umfassen Ethinyl, 1-Propinyl, 2-Propinyl, 1-Butinyl, 2-Butinyl, 3-Butinyl, 1-Pentinyl, 2-Pentinyl, 3-Pentinyl, 4-Pentinyl, 1-Hexinyl, 2-Hexinyl, 3-Hexinyl, 4-Hexinyl-, 5-Hexinyl, 4-Methyl-2-pentinyl, etc..

Der Begriff C₁₋ₙ-Alkoxy oder C₁₋ₙ-Alkyloxy bezeichnet eine C₁₋ₙ-Alkyl-O-Gruppe, worin C₁₋ₙ-Alkyl wie oben definiert ist. Beispiele solcher Gruppen umfassen Methoxy, Ethoxy, n-Propoxy, iso-Propoxy, n-Butoxy, iso-Butoxy, sec-Butoxy, tert-Butoxy, n-Pentoxy, iso-Pentoxy, neo-Pentoxy, tert-Pentoxy, n-Hexoxy, iso-Hexoxy etc..

Der Begriff C₁₋ₙ-Alkylcarbonyl bezeichnet eine C₁₋ₙ-Alkyl-C(=O)-Gruppe, worin C₁₋ₙ-Alkyl wie oben definiert ist. Beispiele solcher Gruppen umfassen Methylcarbonyl, Ethylcarbonyl, n-Propylcarbonyl, iso-Propylcarbonyl, n-Butylcarbonyl, iso-Butylcarbonyl, sec-Butylcarbonyl, tert-Butylcarbonyl, n-Pentylcarbonyl, iso-Pentylcarbonyl, neo-Pentylcarbonyl, tert-Pentylcarbonyl, n-Hexylcarbonyl, iso-Hexylcarbonyl, etc..

Der Begriff C₃₋ₙ-Cycloalkyl bezeichnet eine gesättigte mono-, bi-, tri- oder spirocarbocyclische Gruppe mit 3 bis n C-Atomen. Beispiele solcher Gruppen umfassen Cyclopropyl, Cyclobutyl, Cyclopentyl, Cyclohexyl, Cycloheptyl, Cyclooctyl, Cyclononyl, Cyclododecyl, Decalinyl, Bicyclo[3.2.1.]octyl, Spiro[4.5]decyl, Norpinyl, Norbonyl, Norcaryl, Adamantyl, etc.. Vorzugsweise umfasst der Begriff C₃₋₇-Cycloalkyl gesättigte monocyclische Gruppen.

Der Begriff C₃₋ₙ-Cycloalkyloxy bezeichnet eine C₃₋ₙ-Cycloalkyl-O-Gruppe, worin C₃₋ₙ-Cycloalkyl wie oben definiert ist. Beispiele solcher Gruppen umfassen Cyclopropyloxy, Cyclobutyloxy, Cyclopentyloxy, Cyclohexyloxy, Cycloheptyloxy, etc..

Der Begriff C₅₋ₙ-Cycloalkenyl bezeichnet eine C₅₋ₙ-Cycloalkyl-Gruppe, die wie oben definiert ist und zusätzlich mindestens eine ungesättigte C=C-Doppelbindung hat.

Der Begriff C₃₋ₙ-Cycloalkylcarbonyl bezeichnet eine C₃₋ₙ-Cycloalkyl-C(=O)-Gruppe, worin C₃₋ₙ-Cycloalkyl wie oben definiert ist.

Der Begriff Tri-(C₁₋₄-alkyl)silyl umfasst Silyl-Gruppen, die gleiche oder zwei oder drei verschiedene Alkylgruppen aufweisen.

Der Begriff Di-(C₁₋₃-alkyl)amino umfasst Amino-Gruppen, die gleiche oder zwei verschiedene Alkylgruppen aufweisen.

Der Begriff N-Heterocycloalkyl bezeichnet einen gesättigten carbocyclischen Ring, der eine Imino-Gruppe im Ring aufweist, und der zusätzlich eine weitere Imino-Gruppe oder ein O- oder S-Atom im Ring aufweisen kann. Beispiele solcher N-Heterocycloalkyl-Gruppen sind Pyrrolidin, Piperidin, Piperazin und Morpholin.

Falls in Gruppen, beispielsweise in X, R¹ oder R³, vorkommende Alkyl-Reste substituiert, beispielsweise fluoriert, sein können, so umfasst dies nicht nur Alkyl-Reste in den Gruppen die unmittelbar Alkyl bedeuten, sondern auch in anderen, Alkyl-Reste aufweisenden Bedeutungen, wie beispielsweise Alkoxy, Alkylcarbonyl, Alkoxyalkyl, etc.. So umfasst beispielsweise X, R¹ und R³ in der Bedeutung Alkoxy, wobei Alkylreste teilweise oder vollständig fluoriert sein können, auch Difluormethoxy und Trifluormethoxy.

Die vorstehend und nachfolgend verwendete Schreibweise, bei der in einer Phenyl-gruppe eine Bindung eines Substituenten zur Mitte des Phenylrings hin dargestellt ist, bedeutet, sofern nicht anders angegeben, dass dieser Substituent an jede freie, ein H-Atom tragende Position des Phenylrings gebunden sein kann.

Die erfindungsgemäßen Verbindungen sind unter Anwendung im Prinzip bekannter Syntheseverfahren erhältlich. Bevorzugt werden die Verbindungen nach den im folgenden näher erläuterten erfindungsgemäßen Herstellungsverfahren erhalten.

Die nachfolgenden Beschreibungen bevorzugter Synthesemöglichkeiten beziehen sich auf Endprodukte in einer β-D-Glucopyranosyl-Konfiguration, die durch die Formel IA beschrieben wird. Die Synthese der entsprechenden Vertreter der β-D-Galactoyranosyl-Konfiguration, die durch die Formel IB beschrieben wird, ergibt sich für den Fachmann in analoger Anwendung, weshalb auf nähere Ausführungen und Syntheseschemata hierzu der Übersichtlichkeit halber verzichtet wird.

Die erfindungsgemäßen Tetrahydropyran-Derivate der Formel IIA können aus D-Gluconolacton oder eines Derivats davon durch Addition der gewünschten Aryl-Verbindung in Form einer Organometallverbindung (Schema 1) aufgebaut werden.

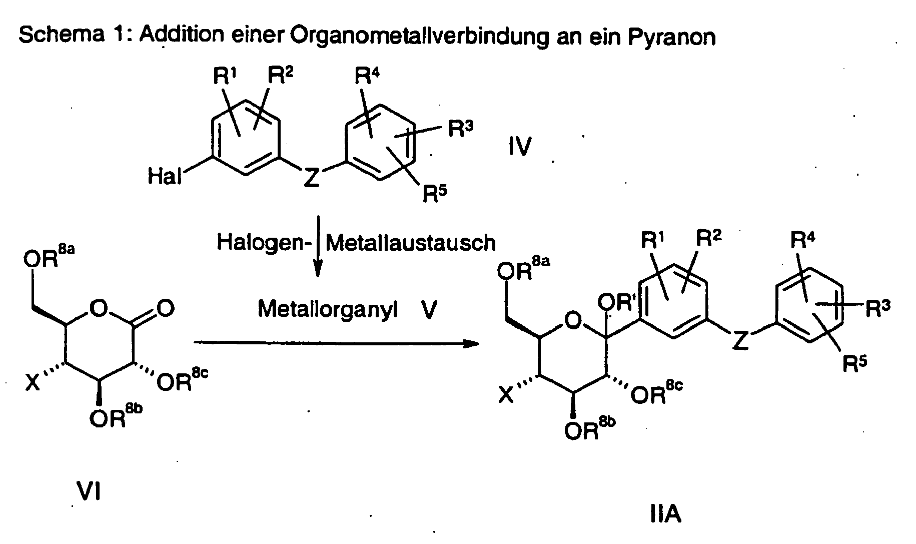

Die Reaktion gemäß Schema 1 wird vorzugsweise ausgehend von einer Halogen-Benzylbenzol-Verbindung der allgemeinen Formel IV, in der Hal Chlor-, Brom- oder Iod bedeutet, durchgeführt. Ausgehend vom Halogenaromaten IV kann die entsprechende Organometallverbindung (V) entweder über einen so genannten Halogen-Metall-Austausch oder über eine Insertion des Metalls in die Kohlenstoff-Halogen-Bindung hergestellt werden. Der Halogen-Metallaustausch mit Brom- oder Iod-substituierten Aromaten kann beispielweise mit einer Organolithiumverbindung wie z.B. n-, sec- oder tert-Butyllithium durchgeführt werden und liefert dabei den entsprechenden lithiierten Aromaten. Die analoge Magnesiumverbindung kann ebenfalls über einen Halogen-Metallaustausch mit einer geeigneten Grignard-Verbindung wie z.B. Isopropylmagnesiumbromid oder Diisopropylmagnesium generiert werden. Die Reaktionen werden vorzugsweise zwischen 0 und -100°C, besonders bevorzugt zwischen -30 und -80°C, in einem inerten Lösungsmittel oder Gemischen daraus, wie beispielsweise Diethylether, Tetrahydrofuran, Toluol, Hexan oder Methylenchlorid, durchgeführt. Die so erhaltenen Magnesium- bzw. Lithium-Verbindungen können gegebenenfalls mit Metallsalzen, wie z.B. Certrichlorid, zu weiteren zur Addition geeigneten Organometallverbindungen (V) ummetalliert werden. Alternativ kann die Organometallverbindung (V) auch durch Insertion eines Metalls in die Kohlenstoff-Halogen-Bindung des Halogenaromaten IV dargestellt werden. Hierzu eignen sich Metalle wie z.B. Lithium oder Magnesium. Die Addition der Organometallverbindung V an das Gluconolacton bzw. Derivaten davon der Formel VI erfolgt vorzugsweise bei Temperaturen zwischen 0 und -100°C, besonders bevorzugt bei -30 bis -80°C, in einem inerten Lösungsmittel oder Gemischen daraus unter Erhalt der Verbindung der Formel IIA. Als Lösungsmittel eignen sich z.B. Diethylether, Toluol, Methylenchlorid, Hexan, Tetrahydrofuran oder Gemische daraus. Die Reaktionen können ohne weitere Hilfsmittel oder im Fall von reaktionsträgen Kupplungspartnem in Gegenwart von Lewis-Säuren wie z.B. BF₃*OEt₂ oder Me₃SiCl durchgeführt werden (siehe M. Schlosser, Organometallics in Synthesis, John Wiley & Sons, Chichester/New York/Brisbane/Toronto/Singapore, 1994). Hierbei bevorzugte Bedeutungen der Gruppen R^{8a}, R^{8b} und R^{8c} sind Benzyl, substituiertes Benzyl, Trialkylsilyl, besonders bevorzugt Trimethylsilyl, Triisopropylsilyl, 4-Methoxybenzyl und Benzyl. Wenn zwei benachbarte Reste der Gruppe bestehend aus R^{8a}, R^{8b} und R^{8c} miteinander verknüpft sind, sind diese beiden Reste bevorzugt Bestandteil eines Benzylidenacetals, 4-Methoxybenzylidenacetals, Isopropylacetals oder stellen eine 2,3-Dimethoxy-butylengruppe dar, die über die 2 und 3-Position des Butans mit den benachbarten Sauerstoffatomen des Pyranoserings verknüpft ist. Der Rest R' bedeutet vorzugsweise Wasserstoff oder C₁₋₄-Alkyl, besonders bevorzugt Wasserstoff, Methyl oder Ethyl. Der Rest R' wird nach der Addition der metallorganischen Verbindung V oder eines Derivats davon an das Gluconolacton VI eingeführt. Dazu wird die Reaktionslösung mit einem Alkohol wie z.B. Methanol oder Ethanol oder Wasser in Gegenwart einer Säure wie z.B. Methansulfonsäure, Toluolsulfonsäure, Schwefelsäure oder Salzsäure behandelt.

Die Synthese von Halogenaromaten der Formel IV und Pyranosederivate der Formel VI kann unter Anwendung von Standardtransformationen in der Organischen Chemie oder zumindest von aus der Fachliteratur bekannten Methoden in der organischen Synthese durchgeführt werden (siehe u.a. J. March, Advanced Organic Reactions, Reactions, Mechanisms, and Structure, 4. Edition, John Wiley & Sons, Chichester/New York/Brisbane/Toronto/Singapore, 1992 und darin zitierte Literatur).
Zur Synthese der Glucosederivate eignen sich neben Glucosederivaten selbst z.B. Galactosederivate, die in 4-Position mit austauschbaren Resten X_{AG} substituiert sind (Schema 2). Hierbei wird der neue Rest X bevorzugt durch eine S_{N}2- oder S_{N}2-artige Reaktion eingebracht. Der Austausch kann sowohl an einem geeigneten Galactose-Derivat als auch am vollständigen Gerüst mit Arylgruppe (die auch als Aglycon bezeichnet wird) durchgeführt werden. X_{AG} stellt in diesen Reaktionen eine Abgangsgruppe wie z.B. Trifluormethylsulfonyl, Tosyl, Mesyl, Iod oder Brom dar und wird durch ein C-, N-, O- oder S-Nucleophil, das bevorzugt als Anion eingebracht wird, unter Inversion der Stereochemie ausgetauscht. Die Abgangsgruppe kann aber auch in situ in Gegenwart des Nucleophils, wie z.B. in Mitsunobu-Reaktionen, gebildet werden. Neben nucleophilen Substitutionen können radikalische Substitutionen, z.B. zum Einbringen eines Wasserstoffs (z.B. Barton-McCombie-Reaktion), oder auch Übergangsmetall-katalysierte Substitutionen, wie z.B. mit Palladium- oder Nickelkatalysatoren, zur Anwendung kommen.

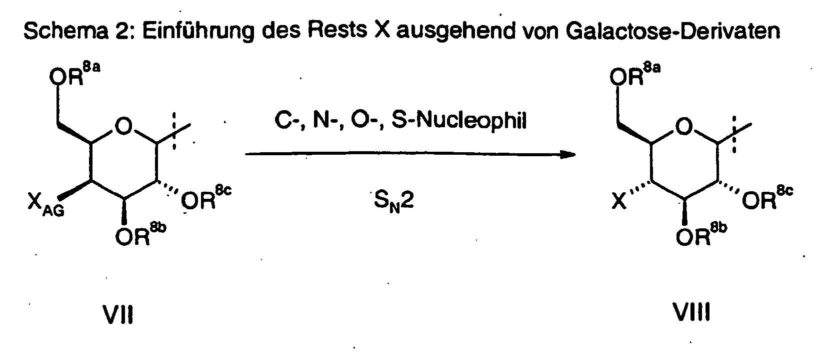

Zur Herstellung von Verbindungen der allgemeinen Formel IA wird gemäß dem erfindungsgemäßen Verfahren a) eine Verbindung der allgemeinen Formel IIA. 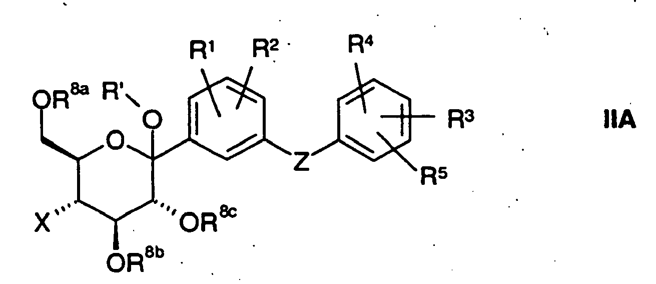 in der X, Z und R', R¹ bis R⁵ wie zuvor definiert sind und
R^{8a}, R^{8b} und R^{8c} wie zuvor definiert sind und beispielsweise unabhängig voneinander Acetyl, Pivaloyl, Benzoyl, tert-Butoxycarbonyl, Benzyloxycarbonyl, Trialkylsilyl, Benzyl oder substituiertes Benzyl bedeuten,
mit einem Reduktionsmittel in Gegenwart einer Säure umgesetzt.

Für die Umsetzung eignen sich als Reduktionsmittel beispielsweise Silane, wie Triethyl-, Tripropyl-, Triisopropyl- oder Diphenylsilan, Natriumborhydrid, Natriumcyanoborhydrid, Zinkborhydrid, Boran, Lithiumaluminiumhydrid, Diisobutylaluminiumhydrid oder Samariumiodid. Die Reduktionen finden vorzugsweise in Gegenwart einer geeigneten Säure, wie z.B. Salzsäure, Toluolsulfonsäure, Trifluoressigsäure, Essigsäure, Bortrifluoridetherat, Trimethylsilyltriflat, Titantetrachlorid, Zinntetrachlorid, Scandiumtriflat oder Zinkiodid statt. In Abhängigkeit vom Reduktionsmittel und der Säure kann die Reaktion in einem Lösungsmittel, wie beispielsweise Methylenchlorid, Chloroform, Acetonitril, Toluol, Hexan, Diethylether, Tetrahydrofuran, Dioxan, Ethanol, Wasser oder Gemischen daraus bei Temperaturen zwischen -60°C und 120°C durchgeführt werden. Ein besonders geeignete Reagenzienkombination besteht beispielsweise aus Triethylsilan und Bortrifluorid-Etherat, die zweckmäßigerweise in Acetonitril oder Dichlormethan bei Temperaturen von -60°C bis 60°C zum Einsatz kommt. Des Weiteren kann Wasserstoff in Gegenwart eines Übergangsmetallkatalysators, wie z.B. Palladium auf Kohle oder Raney-Nickel, in Lösungsmitteln wie Tetrahydrofuran, Ethylacetat, Methanol, Ethanol, Wasser oder Essigsäure, für die dargestellte Transformation angewendet werden.

Alternativ werden zur Herstellung von Verbindungen der allgemeinen Formel IA gemäß dem erfindungsgemäßen Verfahren b) in einer Verbindung der allgemeinen Formel IIIA 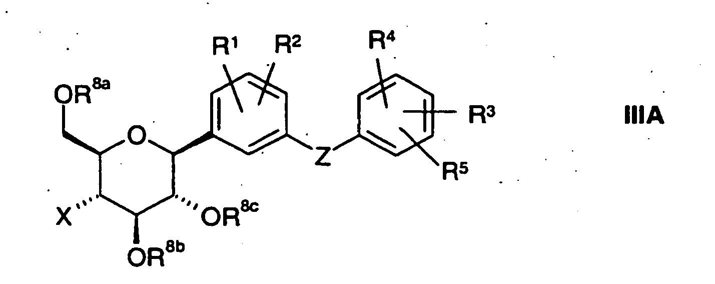 in der X, Z und R¹ bis R⁵ wie zuvor definiert sind und
R^{8a} bis R^{8c} eine der zuvor definierten Schutzgruppen, wie z.B. eine Acyl-, Arylmethyl-, Acetal-, Ketal- oder Silylgruppe bedeuten, die Schutzgruppen abgespalten.

Die Abspaltung eines verwendeten Acyl-, Acetal- oder Ketal-Schutzrestes erfolgt beispielsweise hydrolytisch in einem wässrigen Lösungsmittel, z.B. in Wasser, Isopropanol/Wasser, Essigsäure/Wasser, Tetrahydrofuran/Wasser oder Dioxan/Wasser, in Gegenwart einer Säure wie Trifluoressigsäure, Salzsäure oder Schwefelsäure oder im Fall von Acyl-Resten in Gegenwart einer Alkalibase wie Lithiumhydroxid, Natriumhydroxid oder Kaliumhydroxid bei Temperaturen zwischen 0 und 120°C, vorzugsweise bei Temperaturen zwischen 10 und 100°C. Die Abspaltung eines Trifluoracetylrestes erfolgt vorzugsweise durch Behandlung mit einer Säure wie Salzsäure gegebenenfalls in Gegenwart eines Lösungsmittels wie Essigsäure bei Temperaturen zwischen 50 und 120°C oder durch Behandlung mit Natronlauge gegebenenfalls in Gegenwart eines Lösungsmittels wie Tetrahydrofuran oder Methanol bei Temperaturen zwischen 0 und 50°C.

Die Abspaltung eines Trimethylsilylrestes erfolgt beispielsweise in Wasser, einem wässrigen Lösemittelgemisch oder einem niederen Alkohol wie Methanol oder Ethanol in Gegenwart einer Base wie Lithiumhydroxid, Natriumhydroxid, Kaliumcarbonat oder Natriummethylat. In wässrigen oder alkoholischen Lösungsmitteln eignen sich ebenfalls Säuren, wie z.B. Salzsäure, Trifluoressigsäure oder Essigsäure. Zur Abspaltung in organischen Lösungsmitteln, wie beispielsweise Diethylether, Tetrahydrofuran oder Dichlormethan, eignen sich auch Fluoridreagenzien, wie z.B. Tetrabutylammoniumfluorid.

Die Abspaltung eines Benzyl-, Methoxybenzyl- oder Benzyloxycarbonylrestes erfolgt vorteilhaft hydrogenolytisch, z.B. mit Wasserstoff in Gegenwart eines Katalysators wie Palladium/Kohle in einem geeigneten Lösungsmittel wie Methanol, Ethanol, Essigsäureethylester oder Eisessig, gegebenenfalls unter Zusatz einer Säure wie Salzsäure bei Temperaturen zwischen 0 und 100°C, vorzugsweise jedoch bei Raumtemperaturen zwischen 20 und 60°C, und bei einem Wasserstoffdruck von 1 bis 7 bar, vorzugsweise jedoch von 3 bis 5 bar. Die Abspaltung eines 2,4-Dimethoxybenzylrestes erfolgt jedoch vorzugsweise in Trifluoressigsäure in Gegenwart von Anisol.

Die Abspaltung eines tert.-Butyl- oder tert.-Butyloxycarbonylrestes erfolgt vorzugsweise durch Behandlung mit einer Säure wie Trifluoressigsäure oder Salzsäure oder durch Behandlung mit Jodtrimethylsilan gegebenenfalls unter Verwendung eines Lösungsmittels wie Methylenchlorid, Dioxan, Methanol oder Diethylether.

Bei den vorstehend beschriebenen Umsetzungen können gegebenenfalls vorhandene reaktive Gruppen wie Ethinyl-, Hydroxy-, Amino-, Alkylamin- oder Iminogruppen während der Umsetzung durch übliche Schutzgruppen geschützt werden, welche nach der Umsetzung wieder wie u.a. oben beschrieben abgespalten werden.

Beispielsweise kommt als Schutzrest für eine Ethinylgruppe die Trimethylsilyl- oder Triisopropylsilylgnrppe in Betracht.

Beispielsweise kommen als Schutzrest für eine Hydroxygruppe die Trimethylsityl-, Acetyl-, Trityl-, Benzyl- oder Tetrahydropyranylgruppe in Betracht.

Als Schutzreste für eine Amino-, Alkylamino- oder Iminogruppe kommen beispielsweise die Formyl-, Acetyl-, Trifluoracetyl-, Ethoxycarbonyl-, tert.-Butoxycarbonyl-, Benzyloxycarbonyl-, Benzyl-, Methoxybenzyl- oder 2,4-Dimethoxybenzylgruppe in Betracht.

Des Weiteren können die so erhaltenen Verbindungen der allgemeinen Formel I selektiv an einer Hydroxygruppe derivatisiert oder die Hydroxygruppe selbst substituiert werden.

Ferner können die erhaltenen Verbindungen der allgemeinen Formel I, wie bereits eingangs erwähnt wurde, in ihre Enantiomeren und/oder Diastereomeren aufgetrennt werden. So können beispielsweise cis-/trans-Gemische in ihre cis- und trans-Isomere, und Verbindungen mit mindestens einem optisch aktiven Kohlenstaffatom in ihre Enantiomeren aufgetrennt werden.

So lassen sich beispielsweise die erhaltenen cis-/trans-Gemische durch Chromatographie in ihre cis- und trans-Isomeren, die erhaltenen Verbindungen der allgemeinen Formel I, welche in Racematen auftreten, nach an sich bekannten Methoden (siehe Allinger N. L. und Eliel E. L. in "Topics in Stereochemistry", Vol. 6, Wiley Interscience, 1971) in ihre optischen Antipoden und Verbindungen der allgemeinen Formel I mit mindestens 2 asymmetrischen Kohlenstoffatomen auf Grund ihrer physikalisch-chemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch Säulentrennung an chiralen Phasen oder durch Umkristallisieren aus einem optisch aktiven Lösungsmittel oder durch Umsetzen mit einer, mit der racemischen Verbindung Salze oder Derivate wie z.B. Ester oder Amide bildenden optisch aktiven Substanz, insbesondere Säuren und ihre aktivierten Derivate oder Alkohole, und Trennen des auf diese Weise erhaltenen diastereomeren Salzgemisches oder Derivates, z.B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren sind z.B. die D- und L-Formen von Weinsäure oder Dibenzoylweinsäure, Di-O-Tolylweinsäure, Äpfelsäure, Mandelsäure, Camphersulfonsäure, Glutaminsäure, Asparaginsäure oder Chinasäure. Als optisch aktiver Alkohol kommt beispielsweise (+)- oder (-)-Menthol und als optisch aktiver Acylrest in Amiden beispielsweise (+)-oder (-)-Menthyloxycarbonyl in Betracht.

Des Weiteren können die erhaltenen Verbindungen der Formel I in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Methansulfonsäure, Phosphorsäure, Fumarsäure, Bernsteinsäure, Milchsäure, Zitronensäure, Weinsäure oder Maleinsäure in Betracht.

Weiterhin können die erhaltenen Verbindungen in Gemische, beispielsweise in 1:1 oder 1:2 Gemische mit Aminosäuren, insbesondere mit alpha-Aminosäuren wie Prolin oder Phenylalanin, übergeführt werden, die besonders günstige Eigenschaften wie hohe Kristallinität aufweisen können.

Die als Ausgangsstoffe verwendeten Verbindungen der allgemeinen Formeln II und III sind teilweise literaturbekannt oder können nach an sich literaturbekannten Verfahren sowie in Analogie zu den in den Beispielen beschriebenen Verfahren, gegebenenfalls unter zusätzlicher Einführung von Schutzresten, erhalten werden.

Die erfindungsgemäßen Verbindungen sind vorteilhaft auch nach den in den nachfolgenden Beispielen beschriebenen Verfahren zugänglich, wobei diese hierzu auch mit dem Fachmann beispielsweise aus der Literatur bekannten Verfahren, insbesondere den in den WO 98/31697, WO 01/27128, WO 02/083066, WO 03/099836, WO 2004/063209 und WO 2004/052902 beschriebenen Verfahren, kombiniert werden können.

Wie bereits eingangs erwähnt, weisen die erfindungsgemäßen Verbindungen der allgemeinen Formel I und ihre physiologisch verträglichen Salze wertvolle pharmakologische Eigenschaften auf, insbesondere eine Hemmwirkung auf den natriumabhängigen Glucose-Cotransporter SGLT. Im Hinblick auf eine Inhibierung von SGLT2 und vorzugsweise eine höhere Selektivität der Hemmwirkung auf SGLT2 verglichen mit SGLT1 sind Verbindungen der Formel IA bevorzugt.

### Die biologischen Eigenschaften der neuen Verbindungen können wie folgt geprüft werden:

Die Fähigkeit der Substanzen die SGLT-2 Aktivität zu hemmen, kann in einem Versuchsaufbau gezeigt werden, in dem eine CHO-K1 Zelllinie (ATCC No. CCL 61) oder alternativ eine HEK293 Zelllinie (ATCC No. CRL-1573), die stabil mit einem Expressionsvektor pZeoSV (Invitrogen, EMBL accession number L36849) transfiziert ist, der die cDNA für die kodierende Sequenz des humanen Natrium Glucose Cotransporters 2 (Genbank Acc. No.NM_003041) enthält (CHO-hSGLT2 bzw. HEK-hSGLT2). Diese Zelllinien transportieren Natrium-abhängig ¹⁴C-markiertes alpha-Methyl-Glucopyranosid (¹⁴C-AMG, Amersham) in das Zellinnere.

Der SGLT-2 Assay wird wie folgt durchgeführt: CHO-hSGLT2 Zellen werden in Ham's F12 Medium (BioWhittaker) mit 10% fötalem Kälberserum und 250 µg/ml Zeocin (Invitrogen), HEK293-hSGLT2 Zellen in DMEM Medium mit 10% fötalem Kälberserum und 250 µg/ml Zeocin (Invitrogen) kultiviert. Die Zellen werden von den Kulturflaschen durch zweimaliges Waschen mit PBS und anschließende Behandlung mit Trypsin/EDTA abgelöst. Nachzugabe von Zellkulturmedium werden die Zellen abzentrifugiert, in Kulturmedium resuspendiert und in einem Casy-cellcounter gezählt. Anschließend werden 40.000 Zellen pro Loch in eine weiße, Poly-D-Lysin beschichtete 96-Loch Platte ausgesät und über Nacht bei 37°C, 5% CO₂ inkubiert. Die Zellen werden zweimal mit 250µl Assaypuffer (Hanks Balanced Salt Solution, 137 mM NaCl, 5,4 mM KCl, 2,8 mM CaCl₂, 1,2 mM MgSO₄ und 10 mM HEPES (pH7,4), 50µg/ml Gentamycin) gewaschen. In jedes Loch werden dann 250 µl Assaypuffer und 5 µl Testverbindung hinzugegeben und für weitere 15 Minuten im Brutschrank inkubiert. Als Negativkontrolle werden 5 µl 10% DMSO eingesetzt. Durch Zugabe von 5µl ¹⁴C-AMG (0.05µCi) in jedes Loch wird die Reaktion gestartet. Nach einer 2 stündigen Inkubation bei 37°C, 5% CO₂ werden die Zellen wiederum mit 250 µl PBS (20°C) gewaschen und anschließend durch Zugabe von 25 µl 0.1 N NaOH lysiert (5 min. bei 37°C). Pro Loch werden 200 µl MicroScint20 (Packard) hinzugefügt und für weitere 20 min bei 37°C inkubiert. Nach dieser Inkubation wird die Radioaktivität des aufgenommenen ¹⁴C-AMG in einem Topcount (Packard) mittels eines ¹⁴C-Szintillationsprogramms gemessen.

Zur Bestimmung Aktivität gegenüber dem humanen SGLT1 wird ein analoger Test aufgebaut, in dem die cDNA für hSGLT1 (Genbank Acc. No. NM000343) statt der hSGLT2 cDNA in CHO-K1 bzw. HEK293 Zellen exprimiert wird.

Alternativ kann für hSGLT1 und hSGLT2 auch die Messung des zellulären Membranpotentials zur biologischen Testung von Substanzen herangezogen werden. Hierzu können die weiter oben beschriebenen Zellmodelle angewendet werden. Für den Test werden 10.000 Zellen pro Loch einer poly-D-Lysin beschichteten schwarzen 384-Loch-Platte mit durchsichtigem Boden in Kulturmedium ausgesät und 16 Stunden bei 37°C, 5% CO₂ inkubiert. Anschließend werden die Zellen zweimal mit glucosefreiem HBSS Puffer (12,67 mol/l CaCl₂, 4,93 mmol/l MgCl₂, 4,07 mmol/l MgSO₄, 4,41 mmol/l KH₂PO₄; pH 7,4) gewaschen und mit 20µl HBSS überschichtet. Nach Zugabe von 20 µl Ladepuffer (Membrane Potential Assay Kit Explorer R8126, Molecular Devices GmbH, Ismaning) und 20 µl der zu testenden Substanz in geeigneter Konzentration wird für weitere 30 min. bei 37°C, 5% CO₂ inkubiert. Die Messung erfolgt im Fluorescent Imaging Plate Reader (Molecular Devices GmbH, Ismaning) bei 485 nm Anregungswellenlänge und wird durch Zugabe von 20µl Stimulationspuffer (140 mM NaCl und 120 mM Glucose) gestartet. Die durch den Glucose-induzierten Na⁺-Einstrom verursachte Depolarisation der Zelle kann als Fluoreszenzänderung gemessen und quantifiziert werden.

Die erfindungsgemäßen Verbindungen der allgemeinen Formel I können beispielsweise EC50-Werte unter 1000 nM, insbesondere unter 200 nM, besonders bevorzugt unter 50 nM aufweisen.

Im Hinblick auf die Fähigkeit, die SGLT Aktivität zu hemmen, sind die erfindungsgemäßen Verbindungen der allgemeinen Formel I und ihre entsprechenden pharmazeutisch akzeptablen Salze prinzipiell geeignet, alle diejenigen Zustände oder Krankheiten zu behandeln und/oder vorbeugend zu behandeln, die durch eine Hemmung der SGLT Aktivität, insbesondere der SGLT-2 Aktivität beeinflusst werden können. Daher sind erfindungsgemäße Verbindungen insbesondere zur Prophylaxe oder Behandlung von Krankheiten, insbesondere Stoffwechselerkrankungen, oder Zuständen wie Diabetes mellitus Typ 1 und Typ 2, diabetische Komplikationen (wie z.B. Retinopathie, Nephropathie oder Neuropathien, diabetischer Fuß, Ulcus, Makroangiopathien), metabolische Azidose oder Ketose, reaktiver Hypoglykämie, Hyperinsulinämie, Glukosestoffwechselstörung, Insulinresistenz, Metabolischem Syndrom, Dyslipidämien unterschiedlichster Genese, Atherosklerose und verwandte Erkrankungen, Adipositas, Bluthochdruck, chronisches Herzversagen, Ödeme, Hyperurikämie geeignet. Darüber hinaus sind diese Substanzen geeignet, die beta-Zelldegeneration wie z.B. Apoptose oder Nekrose von pankreatischen beta-Zellen zu verhindern. Die Substanzen sind weiter geeignet, die Funktionalität von pankreatischen Zellen zu verbessern oder wiederherzustellen, daneben die Anzahl und Größe von pankreatischen beta-Zellen zu erhöhen. Die erfindungsgemäßen Verbindungen sind ebenfalls als Diuretika oder Antihypertensiva einsetzbar und zur Prophylaxe und Behandlung des akuten Nierenversagens geeignet.

Ganz besonders sind die erfindungsgemäßen Verbindungen, einschließlich deren physiologisch verträglichen Salze, zur Prophylaxe oder Behandlung von Diabetes, insbesondere Diabetes mellitus Typ 1 und Typ 2, und/oder diabetischen Komplikationen geeignet.

Die zur Erzielung einer entsprechenden Wirkung bei der Behandlung oder Prophylaxe erforderliche Dosierung hängt üblicherweise von der zu verabreichenden Verbindung, vom Patienten, von der Art und Schwere der Krankheit oder des Zustandes und der Art und Häufigkeit der Verabreichung ab und liegt im Ermessen des zu behandelnden Arztes. Zweckmäßigerweise kann die Dosierung bei intravenöser Gabe im Bereich von 1 bis 100 mg, vorzugsweise 1 bis 30 mg, und bei oraler Gabe im Bereich von 1 bis 1000 mg, vorzugsweise 1 bis 100 mg, jeweils 1 bis 4 x täglich, liegen. Hierzu lassen sich die erfindungsgemäß hergestellten Verbindungen der Formel I, gegebenenfalls in Kombination mit anderen Wirksubstanzen, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragees, Kapseln, Pulver, Lösungen, Suspensionen oder Zäpfchen einarbeiten.

Die erfindungsgemäßen Verbindungen können auch in Kombination mit anderen Wirkstoffen, insbesondere zur Behandlung.und/oder Prophylaxe der zuvor angegebenen Krankheiten und Zustände verwendet werden. Für solche Kombinationen kommen als weitere Wirksubstanzen insbesondere solche in Betracht, die beispielsweise die therapeutische Wirksamkeit eines erfindungsgemäßen SGLT-Antagonisten im Hinblick auf eine der genannten Indikationen verstärken und/oder die eine Reduzierung der Dosierung eines erfindungsgemäßen SGLT-Antagonisten erlauben. Zu den zu einer solchen Kombination geeigneten Therapeutika gehören z.B. Antidiabetika, wie etwa Metformin, Sulfonylharnstoffe (z.B. Glibenclamid, Tolbutamid, Glimepiride), Nateglinide, Repaglinide, Thiazolidindione (z.B. Rosiglitazone, Pioglitazone), PPAR-gamma-Agonisten (z.B. Gl 262570) und -Antagonisten, PPARgamma/alpha Modulatoren (z.B: KRP 297), alpha-Glucosidasehemmer (z.B. Acarbose, Voglibose), DPPIV Inhibitoren (z.B. LAF237, MK-431), alpha2-Antagonisten, Insulin und Insulinanaloga, GLP-1 und GLP-1 Analoga (z.B. Exendin-4) oder Amylin. Daneben sind weitere als Kombinationspartner geeignete Wirkstoffe Inhibitoren der Proteintyrosinphosphatase 1, Substanzen, die eine deregulierte Glucoseproduktion in der Leber beeinflussen, wie z.B. Inhibitoren der Glucose-6-phosphatase, oder der Fructose-1,6-bisphosphatase, der Glycogenphosphorylase, Glucagonrezeptor Antagonisten und Inhibitoren der Phosphoenolpyruvatcarboxykinase, der Glykogensynthasekinase oder der Pyruvatdehydrokinase, Lipidsenker, wie etwa HMG-CoA-Reduktasehemmer (z.B. Simvastatin, Atorvastatin), Fibrate (z.B. Bezafibrat, Fenofibrat), Nikotinsäure und deren Derivate, PPARalpha Agonisten, PPAR-delta Agonisten, ACAT Inhibitoren (z.B. Avasimibe) oder Cholesterolresorptionsinhibitoren wie zum Beispiel Ezetimibe, gallensäurebindende Substanzen wie zum Beispiel Colestyramin, Hemmstoffe des ilealen Gallensäuretransportes, HDL-erhöhende Verbindungen wie zum Beispiel Inhibitoren von CETP oder Regulatoren von ABC1 oder Wirkstoffe zur Behandlung von Obesitas, wie etwa Sibutramin oder Tetrahydrolipstatin, Dexfenfluramin, Axokine, Antagonisten des Cannabinoid1 Rezeptors, MCH-1 Rezeptorantagonisten, MC4 Rezeptor Agonisten, NPY5 oder NPY2 Antagonisten oder β3-Agonisten wie SB-418790 oder AD-9677 ebenso wie Agonisten des 5HT2c Rezeptors.

Daneben ist eine Kombination mit Medikamenten zur Beeinflussung des Bluthochdrucks, des chronischen Herzversagens oder der Atherosklerose wie z.B. A-II Antagonisten oder ACE Inhibitoren, ECE-Inhibitoren, Diuretika, β-Blocker, Ca-Antagonisten, zentral wirksamen Antihypertensiva, Antagonisten des alpha-2-adrenergen Rezeptors, Inhibitoren der neutralen Endopeptidase, Thrombozytenaggregationshemmer und anderen oder Kombinationen daraus geeignet. Beispiele von Angiotensin II Rezeptor Antagonisten sind Candesartan Cilexetil, Kalium Losartan, Eprosartan Mesylat, Valsartan, Telmisartan, Irbesartan, EXP-3174, L-158809, EXP-3312, Olmesartan, Medoxomil, Tasosartan, KT-3-671, GA-0113, RU-64276, EMD-90423, BR-9701, etc.. Angiotensin II Rezeptor Antagonisten werden vorzugsweise zur Behandlung oder Prophylaxe von Bluthochdruck und diabetischen Komplikationen verwendet, oft in Kombination mit einem Diuretikum wie Hydrochlorothiazide.

Zur Behandlung oder Prophylaxe der Gicht ist eine Kombination mit Harnsäuresynthese Inhibitoren oder Urikosurika geeignet.

Zur Behandlung oder Prophylaxe diabetischer Komplikationen kann eine Kombination mit GABA-Rezeptor-Antagonisten, Na-Kanal-Blockem, Topiramat, Protein-Kinase C Inhibitoren, advanced glycation endproduct Inhibitoren oder Aldose Reduktase Inhibitoren erfolgen.

Die Dosis für die zuvor angeführten Kombinationspartner beträgt hierbei zweckmäßigerweise 1/5 der üblicherweise empfohlenen niedrigsten Dosierung bis zu 1/1 der normalerweise empfohlenen Dosierung.

Daher betrifft ein weiterer Gegenstand dieser Erfindung die Verwendung einer erfindungsgemäßen Verbindung oder eines physiologisch verträglichen Salzes solch einer Verbindung in Kombination mit mindestens einem der zuvor als Kombinationspartner beschriebenen Wirkstoffe zur Herstellung eines Arzneimittels, das zur Behandlung oder Prophylaxe von Erkrankungen oder Zuständen geeignet ist, die durch Inhibierung des natriumabhängigen Glucose-Cotransporters SGLT beeinflussbar sind. Hierbei handelt es sich vorzugsweise um eine Stoffwechselerkrankung, insbesondere eine der zuvor angeführten Erkrankungen oder Zustände, ganz besonders Diabetes oder diabetischer Komplikationen.

Die Verwendung der erfindungsgemäßen Verbindung, oder eines physiologisch verträglichen Salzes hiervon, in Kombination mit einem weiteren Wirkstoff kann zeitgleich oder zeitlich versetzt, insbesondere aber zeitnah erfolgen. Bei einer zeitgleichen Verwendung werden beide Wirkstoffe dem Patienten zusammen verabreicht; bei einer zeitlich versetzten Verwendung werden beide Wirkstoffe dem Patienten in einem Zeitraum von kleiner gleich 12, insbesondere kleiner gleich 6 Stunden nacheinander verabreicht.

Folglich betrifft ein weiterer Gegenstand dieser Erfindung ein Arzneimittel, das eine erfindungsgemäße Verbindung oder ein physiologisch verträgliches Salz solch einer Verbindung sowie mindestens einen der zuvor als Kombinationspartner beschriebenen Wirkstoffe neben gegebenenfalls einem oder mehreren inerten Trägerstoffen und/oder Verdünnungsmitteln aufweist.

So weist beispielsweise ein erfindungsgemäßes Arzneimittel eine Kombination aus einer erfindungsgemäßen Verbindung der Formel I oder eines physiologisch verträglichen Salzes solch einer Verbindung sowie mindestens einem Angiotensin II Rezeptor Antagonisten neben gegebenenfalls einem oder mehreren inerten Trägerstoffen und/oder Verdünnungsmitteln auf.

Die erfindungsgemäße Verbindung, oder eines physiologisch verträglichen Salzes, und der damit zu kombinierende weitere Wirkstoff können zusammen in einer Darreichungsform, beispielsweise einer Tablette oder Kapsel, oder getrennt in zwei gleichen oder verschiedenen Darreichungsformen, beispielsweise als sogenanntes kit-of-parts, vorliegen.

Vorstehend und nachfolgend werden in Strukturformeln H-Atome von Hydroxylgruppen nicht in jedem Fall explizit dargestellt. Die nachfolgenden Beispiele sollen die vorliegende Erfindung näher erläutern ohne diese zu beschränken:

### Herstellung der Ausgangsverbindungen:

### Beispiel I

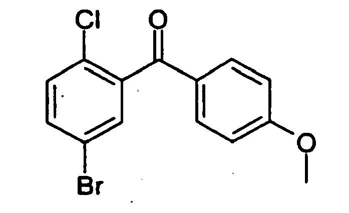

### (5-Brom-2-chlor-phenyl)-(4-methoxy-phenyl)-methanon

Zu einer Mischung von 100 g 5-Brom-2-chlor-benzoesäure in 500 ml Dichlormethan werden 38,3 ml Oxalylchlorid und 0,8 ml Dimethylformamid gegeben. Das Reaktionsgemisch wird 14 h gerührt, danach filtriert und von allen flüchtigen Bestandteilen im Rotationsverdampfer getrennt. Der Rückstand wird in 150 ml Dichlormethan gelöst, die Lösung auf -5°C abgekühlt, und es werden 46,5 g Anisol zugegeben. Danach werden 51,5 g Aluminiumtrichlorid portionsweise so zugegeben, dass die Temperatur nicht über 5°C steigt. Die Lösung wird noch 1 h bei 1-5°C gerührt und anschließend auf Eis gegossen. Die organische Phase wird abgetrennt und die wässrige noch drei Mal mit Dichlormethan extrahiert. Die vereinigten organischen Phasen werden mit wässriger 1 M Salzsäure, zwei Mal mit 1 M Natronlauge und mit gesättigter Natriumchlorid-Lösung gewaschen. Danach wird die organische Phase getrocknet, das Lösungsmittel entfernt und der Rückstand in Ethanol umkristallisiert.
Ausbeute: 86,3 g (64% der Theorie)
Massenspektrum (ESI⁺): m/z = 325/327/329 (Brom+Chlor) [M+H]⁺

### Beispiel II

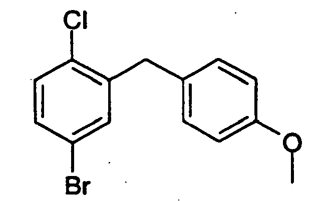

### 4-Brom-1-chlor-2-(4-methoxy-benzyl)-benzol

Eine Lösung von 86,2 g (5-Brom-2-chlor-phenyl)-(4-methoxy-phenyl)-methanon und 101,5 ml Triethylsilan in 75 ml Dichlormethan und 150 ml Acetonitril wird auf 10°C abgekühlt. Dann werden unter Rühren 50,8 ml Bortrifluoriddiethyletherat so zugegeben, dass die Temperatur nicht über 20°C steigt. Die Lösung wird 14 h bei Raumtemperatur gerührt, bevor noch einmal 9 ml Triethylsilan und 4,4 ml Bortrifluoriddiethyletherat zugegeben werden. Die Lösung wird weitere 3 h bei 45-50°C gerührt und dann auf Raumtemperatur abgekühlt. Es wird eine Lösung von 28 g Kaliumhydroxid in 70 ml Wasser zugesetzt und 2 h gerührt. Danach wird die organische Phase abgetrennt und die wässrige noch drei Mal mit Diisopropylether extrahiert. Die vereinten organischen Phasen werden zwei Mal mit 2 M Kalilauge und einmal mit wässriger Natriumchlorid-Lösung gewaschen und anschließend über Natriumsulfat getrocknet. Nach Entfernen des Lösungsmittels wird der Rückstand in Ethanol verrührt, wieder abgetrennt und bei 60°C getrocknet.
Ausbeute: 50,0 g (61 % der Theorie)
Massenspektrum (ESI⁺): m/z = 310/312/314 (Brom+Chlor) [M+H]⁺

### Beispiel III

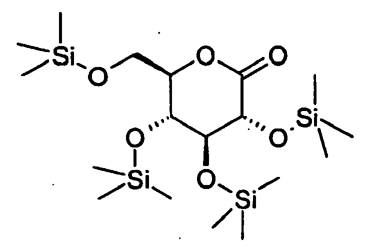

### 2,3,4,6-Tetrakis-O-(trimethylsilyl)-D-glucopyranon

Eine Lösung von 20 g D-Glucono-1,5-lacton und 98,5 ml N-Methylmorpholin in 200 ml Tetrahydrofuran wird auf -5°C abgekühlt. Dann werden 85 ml Trimethylsilylchlorid so zugetropft, dass die Temperatur nicht über 5°C steigt. Die Lösung wird danach 1 h bei Raumtemperatur, 5 h bei 35°C und noch einmal 14 h bei Raumtemperatur gerührt. Nach Zugabe von 300 ml Toluol wird die Lösung im Eisbad abgekühlt, und es werden 500 ml Wasser so zugegeben, dass die Temperatur nicht über 10°C steigt. Die organische Phase wird anschließend abgetrennt und jeweils einmal mit wässriger Natriumdihydrogenphosphatlösung, Wasser und gesättigter wässriger Natriumchloridlösung gewaschen. Das Lösungsmittel wird entfernt, der Rückstand in 250 ml Toluol aufgenommen und das Lösungsmittel erneut vollständig entfernt.
Ausbeute: 52,5 g (ca. 90% rein)
Massenspektrum (ESI⁺): m/z = 467 [M+H]⁺

### Beispiel IV

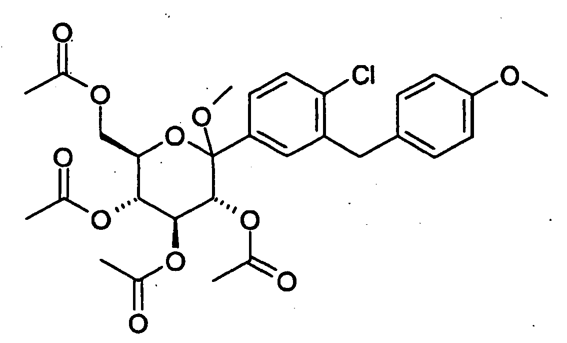

### 1-Chlor-4-(2,3,4,6-tetra-O-acetyl-1-methoxy-D-glucopyranos-1-yl)-2-(4-methoxybenzyl)-benzol

Eine Lösung von 1,0 g 4-Brom-1-chlor-2-(4-methoxy-benzyl)-benzol in 14 ml trockenem Diethylether wird unter Argon auf -80°C abgekühlt. Zu der gekühlten Lösung werden 4,0 ml einer 1,7 M Lösung von tert-Butyllithium in Pentan langsam getropft, und dann wird die Lösung 30 min bei -80°C gerührt. Diese Lösung wird nun über eine Umdrücknadel, die mit Trockeneis gekühlt wird, zu einer -80°C-kalten Lösung von 1,61 g 2,3,4,6-Tetrakis-O-(trimethylsilyl)-D-glucopyranon in 10 ml Diethylether getropft. Die resultierende Lösung wird 4 h bei -78°C gerührt. Danach wird eine Lösung von 0,4 ml Methansulfonsäure in 12 ml Methanol zugegeben und die Lösung 16 h bei Raumtemperatur gerührt. Die Lösung wird anschließend mit Ethyldiisopropylamin neutralisiert und eingeengt. Der Rückstand wird in Toluol aufgenommen und erneut eingeengt. Dann wird der Rückstand in 8 ml Toluol gelöst, und 3,4 ml Ethyldiisopropylamin werden zur Lösung gegeben. Die Lösung wird im Eisbad abgekühlt, und danach werden 1,4 ml Acetanhydrid und 0,04 g 4-Dimethylaminopyridin zugegeben. Die Lösung wird 6 h bei Raumtemperatur gerührt und dann mit wässriger Natriumhydrogencarbonatlösung versetzt. Die organische Phase wird abgetrennt und die wässrige mit Ethylacetat extrahiert. Nach Trocknen der vereinten organischen Extrakte über Natriumsulfat und Entfernen des Lösungsmittels wird der Rückstand über Kieselgel chromatografiert (Cyclohexan/Ethylacetat 6:1->1:1).
Ausbeute: 1,55 g (85% der Theorie)
Massenspektrum (ESI⁺): m/z = 610/612 (Chlor) [M+NH₄]⁺

### Beispiel V

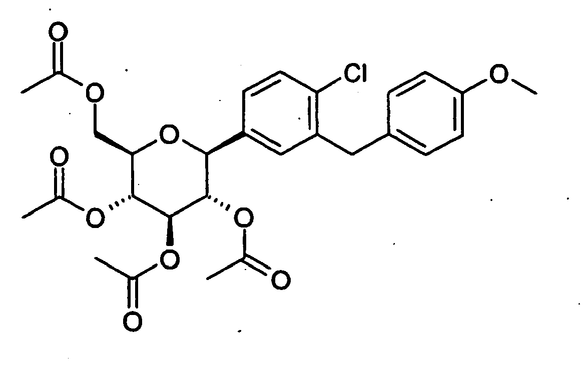

### 1-Chlor-4-(2,3,4,6-tetra-O-acetyl-β-D-glucopyranos-1-yl)-2-(4-methoxy-benzyl)-benzol

Eine Lösung von 1,44 g 1-Chlor-4-(2,3,4,6-tetra-O-acetyl-1-methoxy-D-glucopyranosyl)-2-(4-methoxybenzyl)-benzol in 20 ml Acetonitril und 44 µl Wasser wird im Eisbad abgekühlt. Dann werden 1,2 ml Triethylsilan und 0,26 ml Bortrifluoriddiethyletherat zugegeben. Die Lösung wird 1 h im Eisbad und danach bei Raumtemperatur gerührt. Nach 3 und 5 h werden noch einmal jeweils 0,72 ml Triethylsilan und 0,15 ml Bortrifluoriddiethyletherat zugegeben. Nach weiteren 12 h Rühren bei Raumtemperatur wird wässrige Natriumhydrogencarbonatlösung zugesetzt, 0,5 h gerührt und dann mit Ethylacetat extrahiert. Die organische Phase wird über Natriumsulfat getrocknet, konzentriert und über Kieselgel chromatografiert (Cyclohexan/Ethylacetat 8:1->1:1). Sollte das Produkt danach noch nicht isomerenrein sein, kann das Produkt durch Umkristallisation in Ethanol von Isomeren gereinigt werden.
Ausbeute: 1,12 g (82% der Theorie)
Massenspektrum (ESI⁺): m/z = 580/582 (Chlor) [M+NH₄]⁺

### Beispiel VI

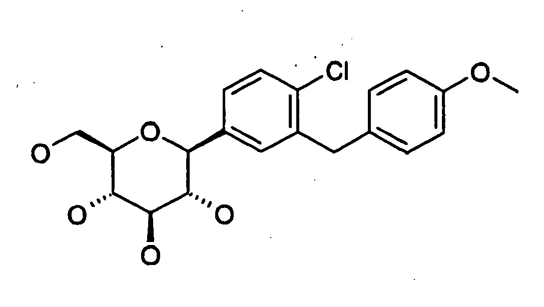

### 1-Chlor-2-(4-methoxy-benzyl)-4-(1-β-D-glucopyranosyl)-benzol

Zu einer Lösung von 1,00 g 1-Chlor-4-(2,3,4,6-tetra-O-acetyl-β-D-glucopyranos-1-yl)-2-(4-methoxy-benzyl)-benzol in 20 ml Methanol werden 2 ml 4 M Kaliumhydroxidlösung gegeben. Die Lösung wird 8 h bei Raumtemperatur gerührt und dann mit 1 M Salzsäure neutralisiert. Die Lösung wird vom Methanol befreit, mit wässriger Natriumchloridlösung versetzt und mit Ethylacetat extrahiert. Die organische Phase wird über Natriumsulfat getrocknet und das Lösungsmittel entfernt. Der Rückstand wird über Kieselgel gereinigt (Dichlormethan/Methanol 1:0->3:1).
Ausbeute: 0,64 g (91 % der Theorie)
Massenspektrum (ESI⁺): m/z = 412/414 (Chlor) [M+ NH₄]⁺

### Beispiel VII

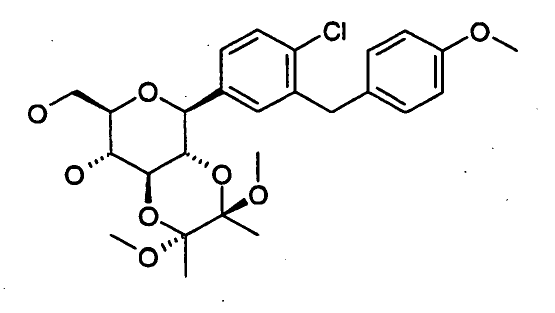

### 1-Chlor-2-(4-methoxy-benzyl)-4-[2,3-O-(2,3-dimethoxy-buta-2,3-diyl)-β-D-glucopyranos-1-yl]-benzol

Zu einer 60°C-warmen Lösung von 1,0 g 1-Chlor-2-(4-methoxy-benzyl)-4-(1-β-D-glucopyranosyl)-benzol in 14 ml Methanol werden nacheinander 0,49 ml Butan-2,3-dion, 1,2 ml Orthoameisensäuremethylester und 0,64 ml Bortrifluoriddiethyletherat gegeben. Die Lösung wird 4 h bei 60°C gerührt und danach auf Raumtemperatur abgekühlt. Bei Raumtemperatur werden 3 ml Triethylamin zugegeben, und die Lösung wird noch 0,5 h gerührt. Danach wird die Lösung eingeengt und der Rückstand über Kieselgel gereinigt (Cyclohexan/Ethylacetat 4:1->1:1).
Ausbeute: 0,70 g (54% der Theorie), daneben wird noch das 3,4-geschützte Glucosederivat (0,54 g, 42% der Theorie) isoliert.
Massenspektrum (ESI⁺): m/z = 526/528 (Chlor) [M+NH₄]⁺

### Beispiel VIII

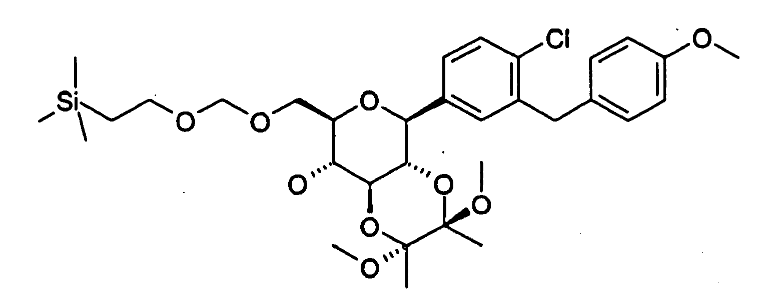

### 1-Chlor-2-(4-methoxy-benzyl)-4-[2,3-O-(2,3-dimethoxy-buta-2,3-diyl)-6-O-(2-trimethylsilylethoxymethyl)-β-D-glucopyranos-1-yl]-benzol

Zu einer Lösung von 2,08 g 1-Chlor-2-(4-methoxy-benryl)-4-[2,3-O-(2,3-dimethoxy-buta-2,3-diyl)-β-D-glucopyranos-1-yl]-benzol und 0,8 ml Ethyldiisopropylamin in 15 ml Dichlormethan werden 0,72 ml 2-Trimethylsilylethoxymethylchlorid gegeben. Nach 8 h Rühren bei Raumtemperatur werden noch einmal 0,3 ml Ethyldiisopropylamin und 0,2 ml 2-Trimethylsilylethoxymethylchlorid zugegeben und weitere 5 h bei Raumtemperatur gerührt. Dann wird die Reaktionslösung mit Wasser versetzt und mit Dichlormethan extrahiert. Die vereinigten organischen Extrakte werden über Natriumsulfat getrocknet, und das Lösungsmittel wird entfernt. Der Rückstand wird über Kieselgel chromatografiert (Cyclohexan/Ethylacetat 1:0->3:2).
Ausbeute: 1,50 g (58% der Theorie)
Massenspektrum (ESI⁺): m/z = 656/658 (Chlor) [M+NH₄]⁺

### Beispiel IX

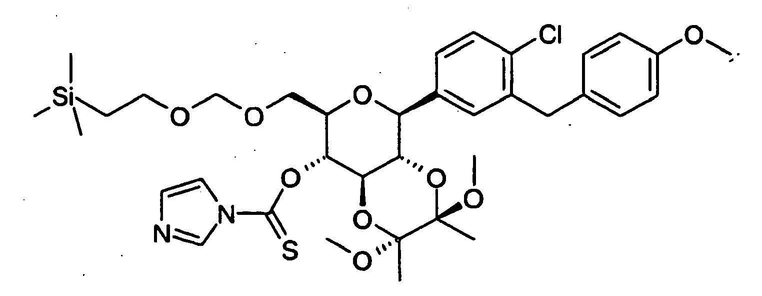

### 1-Chlor-2-(4-methoxy-benzyl)-4-[2,3-O-(2,3-dimethoxy-buta-2,3-diyl)-4-O-(imidazo)-1-yl-thiocarbonyl)-6-O-(2-trimethylsilylethoxymethyl)-β-D-glucopyranos-1-yl]-benzol

Eine Lösung von 0,3 g 1-Chlor-2-(4-methoxy-benryl)-4-[2,3-O-(2,3-dimethoxy-buta-2,3-diyl)-6-O-(2-trimethylsilylethoxymethyl)-β-D-glucopyranos-1-yl]-benzol und 0,21 g Thiocarbonyldiimidazol in 5 ml Toluol wird 5 h bei 90°C gerührt. Dann wird die Reaktionslösung mit Wasser versetzt und mit Ethylacetat extrahiert. Die vereinigten organischen Extrakte werden über Natriumsulfat getrocknet, und das Lösungsmittel wird entfernt. Der Rückstand wird über Kieselgel chromatografiert (Cyclohexan/Ethylacetat 1:0->1:1).
Ausbeute: 0,22 g (63% der Theorie)
Massenspektrum (ESI⁺): m/z = 749/751 (Chlor) [M+H]⁺

### Beispiel X

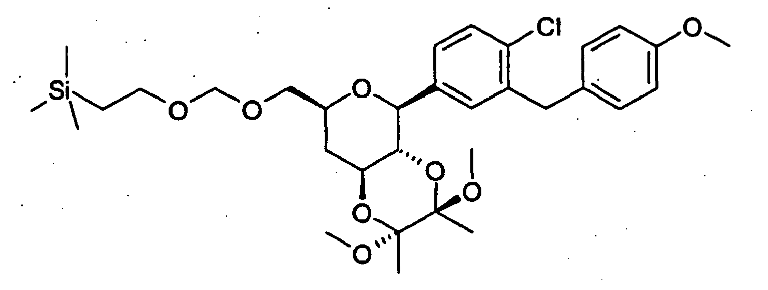

### 1-Chlor-2-(4-methoxy-benzyl)-4-[2,3-O-(2,3-dimethoxy-buta-2,3-diyl)-4-desoxy-6-O-(2-trimethylsilylethoxymethyl)-β-D-glucopyranos-1-yl]-benzol

Eine Lösung von 0,22 g 1-Chlor-2-(4-methoxy-benzyl)-4-[2,3-O-(2,3-dimethoxy-buta-2,3-diyl)-4-O-(imidazol-1-yl-thiocarbonyl)-6-O-(2-trimethylsilylethoxymethyl)-β-D-glucopyranos-1-yl]-benzol in 1,4 ml Tris(trimethylsilyl)silan und 3 ml Toluol wird mit Argon gespült. Dann werden 17 mg Azobisisobutyronitril zugegeben, und die Lösung wird über Nacht bei 120°C gerührt. Nach dem Abkühlen auf Raumtemperatur wird Methanol zugegeben und die Lösung eingeengt. Zum Rückstand wird 1 M Salzsäure gegeben und mit Ethylacetat extrahiert. Die vereinigten organischen Extrakte werden über Natriumsulfat getrocknet, und das Lösungsmittel wird entfernt. Der Rückstand wird über Kieselgel chromatografiert (Cyclohexan/Ethylacetat 1:0->3:2).
Ausbeute: 0,15 g (80% der Theorie)
Massenspektrum (ESI⁺): m/z = 640/642 (Chlor) [M+H]⁺

### Beispiel XI

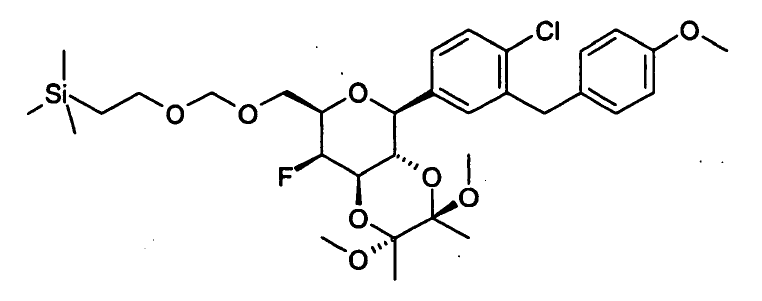

### 1-Chlor-2-(4-methoxy-benzyl)-4-[2,3-O-(2,3-dimethoxy-buta-2,3-diyl)-4-desoxy-4-fluor-6-O-(2-trimethylsilylethoxymethyl)-β-D-galactopyranos-1-yl]-benzol

Zu einer auf -30°C gekühlten Lösung von 0,2 g 1-Chlor-2-(4-methoxy-benzyl)-4-[2,3-O-(2,3-dimethoxy-buta-2,3-diyl)-6-O-(2-trimethylsilylethoxymethyl)-β-D-glucopyranos-1-yl]-benzol in 2 ml Dichlormethan werden 0,3 ml [Bis(2-methoxyethyl)amino]schwefeltrifluorid gegeben. Die Lösung wird langsam auf Raumtemperatur erwärmen gelassen und dann noch 16 h bei Raumtemperatur gerührt. Die Lösung wird dann im Eisbad abgekühlt, mit Dichlormethan verdünnt und mit wässriger Natriumhydrogencarbonatlösung versetzt. Die organische Phase wird abgetrennt und die organische mit Dichlormethan extrahiert. Die vereinigten organischen Phasen werden über Natriumsulfat getrocknet, und das Lösungsmittel wird entfernt. Der Rückstand wird über Kieselgel chromatografiert (Cyclohexan/Ethylacetat 1:0->2:3).
Ausbeute: 40 mg (20% der Theorie)
Massenspektrum (ESI⁺): m/z = 658/660 (Chlor) [M+NH₄]⁺

### Herstellung der Endverbindungen:

### Beispiel 1

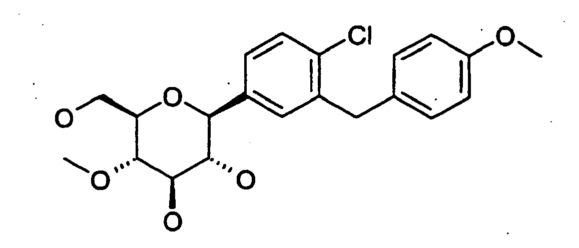

### 1-Chlor-2-(4-methoxy-benzyl)-4-(4-O-methyl-β-D-glucopyranos-1-y1)-benzol

Zu einer Lösung von 0,35 g 1-Chlor-2-(4-methoxy-benzyl)-4-[2,3-O-(2,3-dimethoxy-buta-2,3-diyl)-β-D-glucopyranos-1-yl]-benzol in 2,5 ml Dimethylformamid werden 0,14 g Silber(I)oxid und 70 µl Methyliodid gegeben. Das Gemisch wird 48 h bei Raumtemperatur gerührt und dann mit Ethylacetat verdünnt. Danach wird das Gemisch über Celite filtriert, Wasser zugegeben und mit Ethylacetat extrahiert. Nach Trocknen über Natriumsulfat wird das Lösungsmittel entfernt und der Rückstand über Kieselgel chromatografiert (Cyclhexan/Ethylacetat 5:1->1:1). Das danach erhaltene 1-Chlor-2-(4-methoxy-benzyl)-4-[2,3-O-(2,3-dimethoxy-buta-2,3-diyl)-4-O-methyl-β-D-glucopyranos-1-yl]-benzol wird in 2,5 ml eines Trifluoressigsäure/Wasser-Gemischs (80:20) aufgenommen und die Lösung 1 h bei Raumtemperatur gerührt. Die Lösung wird mit Wasser verdünnt und mit 4 M Kalilauge alkalisch gestellt. Die wässrige Lösung wird mit Ethylacetat extrahiert, die vereinten Extrakte über Natriumsulfat getrocknet und das Lösungsmittel entfernt. Der Rückstand wird über Kieselgel chromatografiert (Dichlormethan/Methanol 1:0->8:1).
Ausbeute: 0,12 g (43% der Theorie)
Massenspektrum (ESI⁺): m/z = 426/428 (Chlor) [M+NH₄]⁺

### Beispiel 2

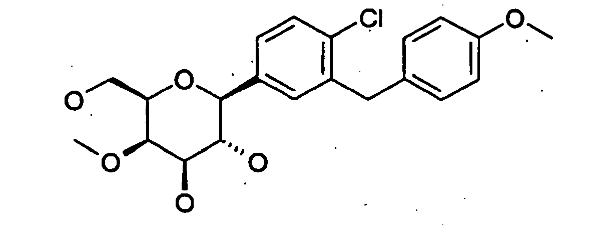

### 1-Chlor-2-(4-methoxy-benzyl)-4-(4-O-methyl-β-D-galactopyranos-1-yl)-benzol

Ausgehend von 1-Chlor-2-(4-methoxy-benzyl)-4-[2,3-O-(2,3-dimethoxy-buta-2,3-diyl)-β-D-galactopyranos-1-yl]-benzol, welches analog den vorherigen Beispielen erhältlich ist, wird nach Schützung der 6-OH-Funktion mit einer säurelabilen Schutzgruppe, wie z.B. Methoxymethyl oder 2-Trimethylsilylethoxymethyl, und nach der in Beispiel 1 beschriebenen Umsetzung 1-Chlor-2-(4-methoxy-benzyl)-4-(4-O-methyl-β-D-galactopyranos-1-yl)-benzol erhalten.

### Beispiel 3

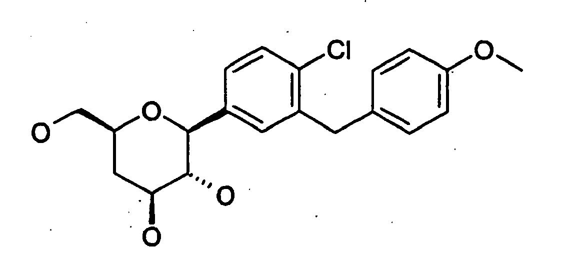

### 1-Chlor-2-(4-methoxy-benzyl)-4-(4-desoxy-β-D-glucopyranos-1-yl)-benzol

Eine Lösung von 0,15 g 1-Chlor-2-(4-methoxy-benzyl)-4-[2,3-O-(2,3-dimethoxy-buta-2,3-diyl)-4-desoxy-6-O-(2-trimethylsilylethoxymethyl)-β-D-glucopyranos-1-yl]-benzol in einem Trifluoressigsäure/Wasser-Gemisch (80:20) wird 30 min bei Raumtemperatur gerührt. Die Lösung wird dann mit Wasser verdünnt und mit 4 M Kalilauge alkalisch gestellt. Die wässrige Lösung wird mit Ethylacetat extrahiert, die vereinten Extrakte über Natriumsulfat getrocknet und das Lösungsmittel entfernt. Der Rückstand wird über Kieselgel chromatografiert (Dichlormethan/Methanol 1:0->4:1). '
Ausbeute: 0,12 g (43% der Theorie)
Massenspektrum (ESI⁺): m/z = 396/398 (Chlor) [M+NH₄]⁺

Die folgende Verbindung kann analog Beispiel 3 erhalten werden:

### (4) 1-Chlor-2-(4-methoxy-benzyl)-4-(4-desoxy-4-fluor-β-D-galactopyranos-1-yl)-benzol

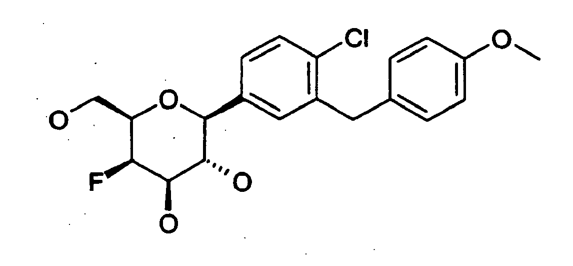

Analog den vorstehend genannten Beispielen und anderen literaturbekannten Verfahren werden auch folgende Verbindungen hergestellt:

| Bsp. | Struktur | Bsp. | Struktur |
|---|---|---|---|
| (5) | 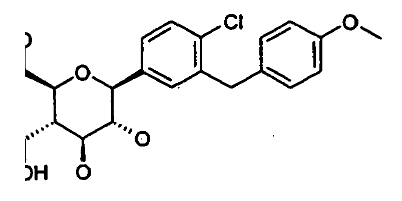 | (6) | 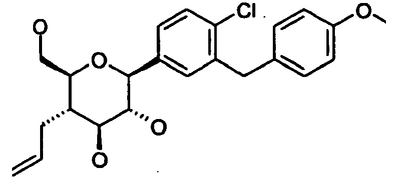 |
| (7) | 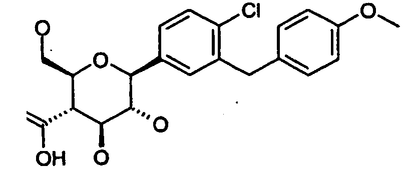 | (8) | 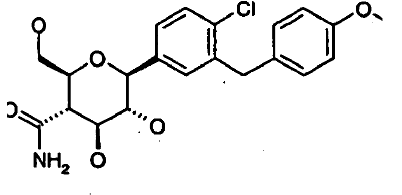 |
| (9) | 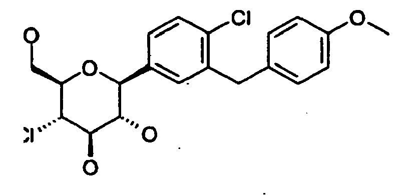 | (10) | 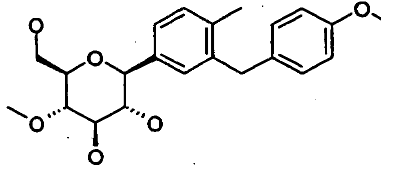 |
| (11) | 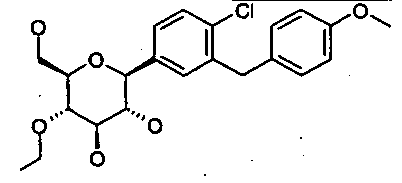 | (12) | 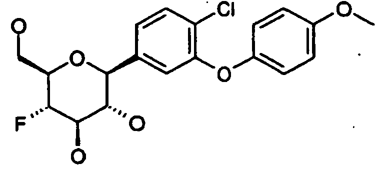 |
| (13) | 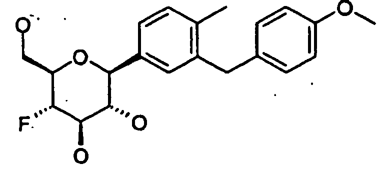 | (14) | 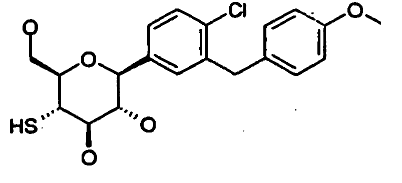 |
| (15) | 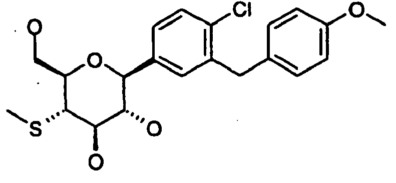 | (16) | 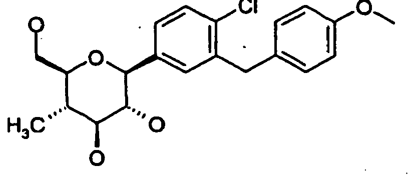 |
| (17) | 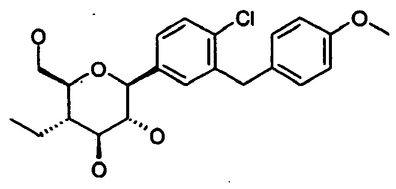 | (18) | 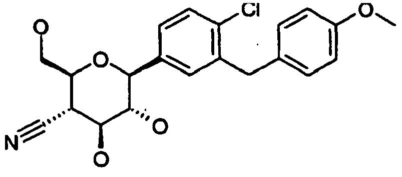 |
| (19) | 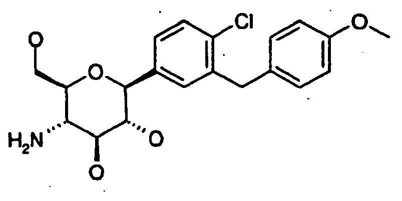 | (20) | 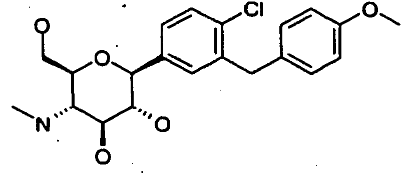 |
| (21) | 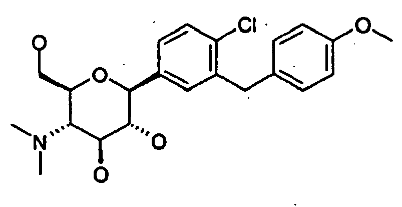 | (22) | 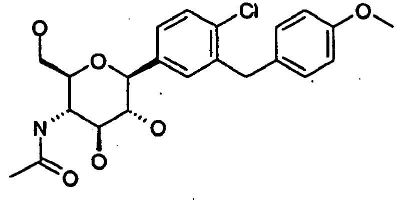 |
| (23) | 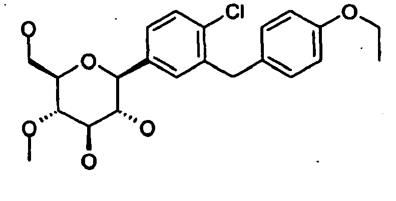 | (24) | 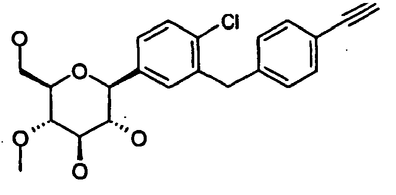 |
| (25) | 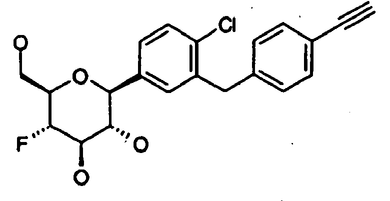 | (26) | 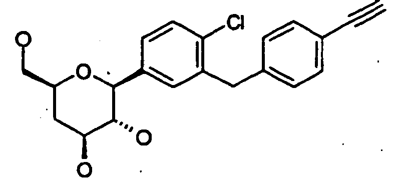 |
| (27) | 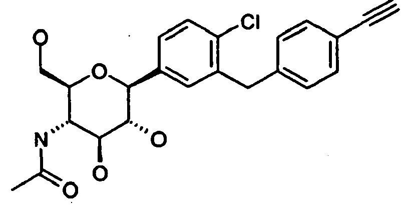 | (28) | 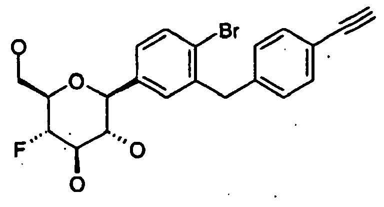 |
| (29) | 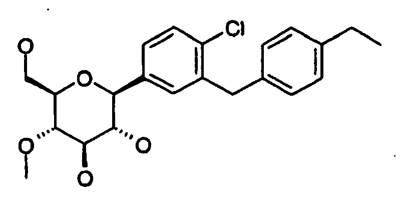 | (30) | 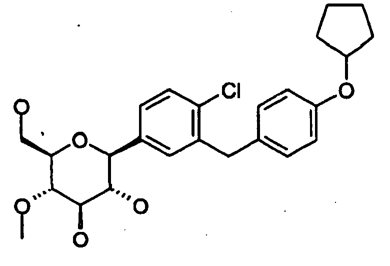 |
| (31) | 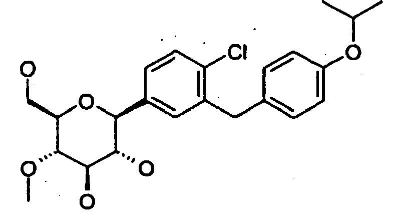 | (32) | 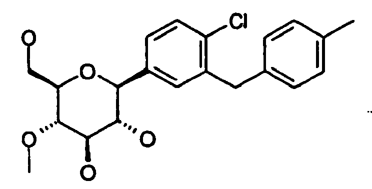 |
| (33) | 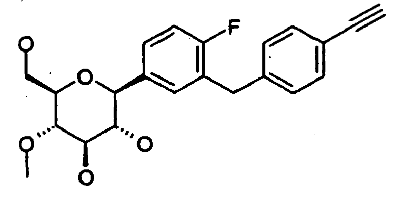 | (34) | 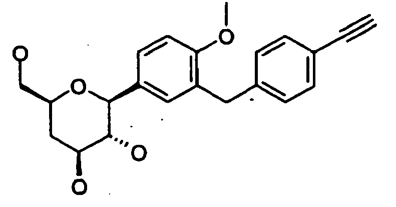 |
| (35) | 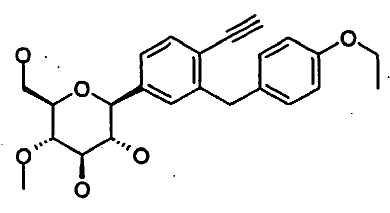 | (36) | 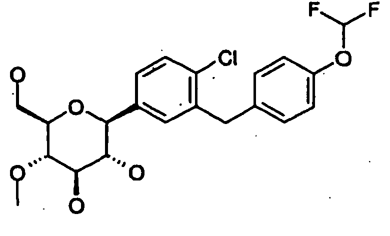 |
| (37) | 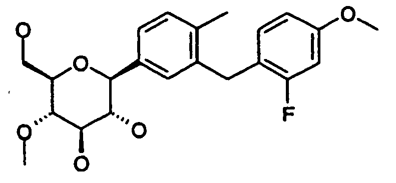 | (38) | 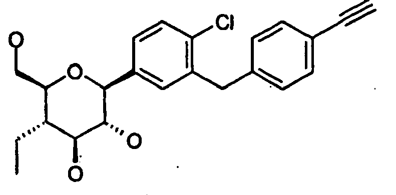 |
| (39) | 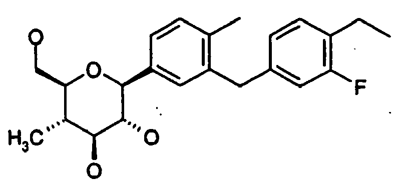 | (40) | 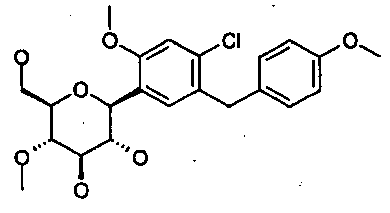 |
| (41) | 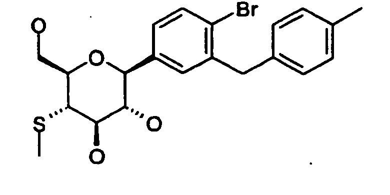 | (42) | 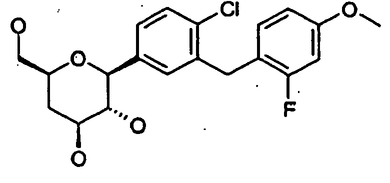 |
| (43) | 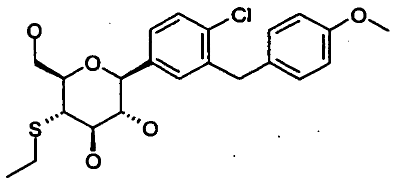 | (44) | 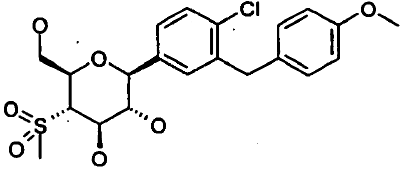 |

Nachfolgend werden Beispiele zu Darreichungsformen beschrieben, worin die Angabe "Wirkstoff" eine oder mehrere erfindungsgemäße Verbindungen, einschließlich deren Salze bedeutet. Im Falle einer der beschriebenen Kombinationen mit einem oder mehreren weiteren Wirksubstanzen umfasst der Begriff "Wirkstoff" auch die weiteren Wirksubstanzen.

### Beispiel A

Tabletten mit 100 mg Wirksubstanz

### Zusammensetzung:

| | | |
|---|---|---|
| 1 | Tablette enthält: | |
| | Wirksubstanz | 100.0 mg |
| | Milchzucker | 80.0 mg |
| | Maisstärke | 34.0 mg |
| | Polyvinylpyrrolidon | 4.0 mg |
| | Magnesiumstearat | 2.0 mg |
| | | 220.0 mg |

### Herstellungverfahren:

Wirkstoff, Milchzucker und Stärke werden gemischt und mit einer wäßrigen Lösung des Polyvinylpyrrolidons gleichmäßig befeuchtet. Nach Siebung der feuchten Masse (2.0 mm-Maschenweite) und Trocknen im Hordentrockenschrank bei 50°C wird erneut gesiebt (1.5 mm-Maschenweite) und das Schmiermittel zugemischt. Die pressfertige Mischung wird zu Tabletten verarbeitet.

| | |
|---|---|
| Tablettengewicht: | 220 mg |
| Durchmesser: | 10 mm, biplan mit beidseitiger Facette und einseitiger Teilkerbe. |

### Beispiel B

Tabletten mit 150 mg Wirksubstanz

### Zusammensetzung:

| | | |
|---|---|---|
| 1 | Tablette enthält: | |
| | Wirksubstanz | 150.0 mg |
| | Milchzucker pulv. | 89.0 mg |
| | Maisstärke | 40.0 mg |
| | Kolloide Kieselgelsäure | 10.0 mg |
| | Polyvinylpyrrolidon | 10.0 mg |
| | Magnesiumstearat | 1.0 mg |
| | | |

### Herstellung:

Die mit Milchzucker, Maisstärke und Kieselsäure gemischte Wirksubstanz wird mit einer 20%igen wäßrigen Polyvinylpyrrolidonlösung befeuchtet und durch ein Sieb mit 1.5 mm-Maschenweite geschlagen.
Das bei 45°C getrocknete Granulat wird nochmals durch dasselbe Sieb gerieben und mit der angegebenen Menge Magnesiumstearat gemischt. Aus der Mischung werden Tabletten gepreßt.

| | |
|---|---|
| Tablettengewicht: | 300 mg |
| Stempel: | 10 mm, flach |

### Beispiel C

Hartgelatine-Kapseln mit 150 mg Wirksubstanz

### Zusammensetzung:

| | | |
|---|---|---|
| 1 | Kapsel enthält: | |
| | Wirkstoff | 150.0 mg |
| | Maisstärke getr. | ca. 180.0 mg |
| | Milchzucker pulv. | ca. 87.0 mg |
| | Magnesiumstearat | 3.0 mg |
| | ca. | 420.0 mg |

### Herstellung:

Der Wirkstoff wird mit den Hilfsstoffen vermengt, durch ein Sieb von 0.75 mm-Maschenweite gegeben und in einem geeigneten Gerät homogen gemischt.
Die Endmischung wird in Hartgelatine-Kapseln der Größe 1 abgefüllt.
Kapselfüllung: ca. 320 mg
Kapselhülle: Hartgelatine-Kapsel Größe 1.

### Beispiel D

Suppositorien mit 150 mg Wirksubstanz

### Zusammensetzung:

| | | |
|---|---|---|
| 1 | Zäpfchen enthält: | |
| | Wirkstoff | 150.0 mg |
| | Polyäthylenglykol 1500 | 550.0 mg |
| | Polyäthylenglykol 6000 | 460.0 mg |
| | Polyoxyäthylensorbitanmonostearat | 840.0 mg |
| | | 2000.0 mg |

### Herstellung:

Nach dem Aufschmelzen der Suppositorienmasse wird der Wirkstoff darin homogen verteilt und die Schmelze in vorgekühlte Formen gegossen.

### Beispiel E

Ampullen mit 10 mg Wirksubstanz

### Zusammensetzung:

| | |
|---|---|
| Wirkstoff | 10.0 mg |
| 0.01 n Salzsäure s.q. | |
| Aqua bidest ad | 2.0 ml |

### Herstellung:

Die Wirksubstanz wird in der erforderlichen Menge 0.01 n HCl gelöst, mit Kochsalz isotonisch gestellt, sterilfiltriert und in 2 ml Ampullen abgefüllt.

### Beispiel F

Ampullen mit 50 mg Wirksubstanz

### Zusammensetzung:

| | |
|---|---|
| Wirkstoff | 50.0 mg |
| 0.01 n Salzsäure s.q. | |
| Aqua bidest | ad 10.0 ml |

### Herstellung:

Die Wirksubstanz wird in der erforderlichen Menge 0.01 n HCl gelöst, mit Kochsalz isotonisch gestellt, sterilfiltriert und in 10 ml Ampullen abgefüllt.

## Patentansprüche

1. D-Pyranosyl-substituierte Phenyle der allgemeinen Formel I 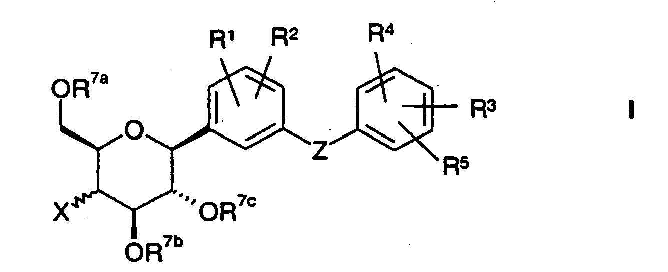 in der
R¹ Wasserstoff, Fluor, Chlor, Brom, Iod, C₁-₆-Alkyl, C₂₋₆-Alkinyl, C₂₋₆-Alkenyl, C₃₋₁₀-Cycloalkyl, C₃₋₁₀-Cycloalkyl-C₁₋₃-alkyl, C₅₋₁₀-Cycloalkenyl, C₅₋₁₀Cycloalkenyl-C₁₋₃-alkyl, C₁₋₄-Alkylcarbonyl, Arylcarbonyl, Heteroarylcarbonyl, Aminocarbonyl, C₁₋₄-Alkylaminocarbonyl, Di-(C₁₋₃-Alkyl)aminocarbonyl, Pyrrolidin-1-ylcarbonyl, Piperidin-1-ylcarbonyl, Morpholin-4-ylcarbonyl, Piperazin-1-ylcarbonyl, 4-(C₁₋₄-Alkyl)piperazin-1-ylcarbonyl, C₁₋₄-Alkoxycarbonyl, Amino, C₁₋₄-Alkylamino, Di-(C₁₋₃-alkyl)amino, Pyrrolidin-1-yl, Piperidin-1-yl, Morpholin-4-yl, Piperazin-1-yl, 4-(C₁₋₄-Alkyl)piperazin-1-yl, C₁₋₄-Alkylcarbonylamino, C₁₋₆-Alkyloxy, C₃₋₁₀-Cycloalkyloxy, C₅₋₁₀-Cycloalkenyloxy, Aryloxy, C₁₋₄-Alkylsulfanyl, C₁₋₄-Alkylsulfinyl, C₁₋₄-Alkylsulfonyl, C₃₋₁₀-Cycloalkylsulfanyl, C₃₋₁₀-Cycloalkylsulfinyl, C₃₋₁₀-Cycloalkylsulfonyl, C₅₋₁₀-Cycloalkenylsulfanyl, C₅₋₁₀-Cycloalkenylsulfinyl, C₅₋₁₀-Cycloalkenylsulfonyl, Arylsulfanyl, Arylsulfinyl, Arylsulfonyl, Hydroxy, Cyan oder Nitro bedeutet,
wobei Alkyl-, Alkenyl-, Alkinyl-, Cycloalkyl- und Cycloalkenyl-Reste teilweise oder vollständig fluoriert oder ein- oder zweifach mit gleichen oder verschiedenen Substituenten ausgewählt aus Chlor, Hydroxy, C₁₋₃-Alkoxy und C₁₋₃-Alkyl substituiert sein können, und
wobei in Cycloalkyl- und Cycloalkenyl-Resten eine oder zwei Methylengruppen unabhängig voneinander durch O, S, CO, SO oder SO₂ ersetzt sein können, und
wobei in N-Heterocycloalkyl-Resten eine Methylengruppe durch CO oder SO₂ ersetzt sein kann, und
R² Wasserstoff, Fluor, Chlor, Brom, Hydroxy, C₁₋₄-Alkyl, C₁₋₄-Alkoxy, Cyan oder Nitro, wobei Alkyl-Reste ein- oder mehrfach mit Fluor substituiert sein können, oder
für den Fall, dass R¹ und R² an zwei miteinander benachbarte C-Atome des Phenylrings gebunden sind, können R¹ und R² derart miteinander verbunden sein, dass R¹ und R² zusammen eine C₃₋₅-Alkylen-, C₃₋₅-Alkenylen- oder Butadienylen-Brücke bilden, die teilweise oder vollständig fluoriert oder ein- oder zweifach mit gleichen oder verschiedenen Substituenten ausgewählt aus Chlor, Hydroxy, C₁₋₃-Alkoxy und C₁₋₃-Alkyl substituiert sein kann, und in der eine oder zwei Methylengruppen unabhängig voneinander durch O, S, CO, SO, SO₂ oder NR^{N} ersetzt sein können, und in der im Falle einer Butadienylen-Brücke eine oder zwei Methingruppen durch ein N-Atom ersetzt sein können,
R³ Wasserstoff, Fluor, Chlor, Brom, Iod, C₁₋₆-Alkyl, C₂₋₆-Alkinyl, C₂₋₆-Alkenyl, C₃₋₁₀-Cycloalkyl, C₃₋₁₀-Cycloalkyl-C₁₋₃-alkyl, C₅₋₁₀-Cycloalkenyl, C₅₋₁₀-Cycloalkenyl-C₁₋₃-alkyl, Aryl, Heteroaryl, C₁₋₄-Alkylcarbonyl, Arylcarbonyl, Heteroarylcarbonyl, Aminocarbonyl, C₁₋₄-Alkylaminocarbonyl, Di-(C₁₋₃-Alkyl)aminocarbonyl, Pyrrolidin-1-ylcarbonyl, Piperidin-1-ylcarbonyl, Morpholin-4-ylcarbonyl, Piperazin-1-ylcarbonyl, 4-(C₁₋₄-Alkyl)piperazin-1-ylcarbonyl, Hydroxycarbonyl, C₁₋₄-Alkoxycarbonyl, C₁₋₄-Alkylamino, Di-(C₁₋₃-alkyl)amino, Pyrrolidin-1-yl, Piperidin-1-yl, Morpholin-4-yl, Piperazin-1-yl, 4-(C₁₋₄-Alkyl)piperazin-1-yl, C₁₋₄-Alkylcarbonylamino, Arylcarbonylamino, Heteroarylcarbonylamino, C₁₋₄-Alkylsulfonylamino, Arylsulfonylamino, C₁₋₆-Alkoxy, C₃₋₇-Cycloalkyloxy, C₅₋₇-Cycloalkenyloxy, Aryloxy, Heteroaryloxy, C₁₋₄-Alkylsulfanyl, C₁₋₄-Alkylsulfinyl, C₁₋₄-Alkylsulfonyl, C₃₋₁₀-Cycloalkylsulfanyl, C₃₋₁₀-Cycloalkylsulfinyl, C₃₋₁₀-Cycloalkylsulfonyl, C₅₋₁₀-Cycloalkenylsulfanyl, C₅₋₁₀-Cycloalkenylsulfinyl, C₅₋₁₀-Cycloalkenylsulfonyl, Arylsulfanyl, Arylsulfinyl, Arylsulfonyl, Amino, Hydroxy, Cyan oder Nitro,
wobei Alkyl-, Alkenyl-, Alkinyl-, Cycloalkyl- und Cycloalkenyl-Reste teilweise oder vollständig fluoriert oder ein- oder zweifach mit gleichen oder verschiedenen Substituenten ausgewählt aus Chlor, Hydroxy, C₁₋₃-Alkoxy und C₁₋₃-Alkyl substituiert sein können, und
wobei in Cycloalkyl- und Cycloalkenyl-Resten eine oder zwei Methylengruppen unabhängig voneinander durch O, S, CO, SO oder SO₂ ersetzt sein können, und
wobei in N-Heterocycloalkyl-Resten eine Methylengruppe durch CO oder SO₂ substituiert sein kann, und
R⁴ Wasserstoff, Fluor, Chlor, Brom, Iod, Cyan, Nitro, C₁₋₃-Alkyl, C₁₋₃-Alkoxy, durch 1 bis 3 Fluoratome substituiertes Methyl- oder Methoxy, oder
für den Fall, dass R³ und R⁴ an zwei miteinander benachbarte C-Atome des Phenylrings gebunden sind, können R³ und R⁴ derart miteinander verbunden sein, dass R³ und R⁴ zusammen eine C₃₋₅-Alkylen-, C₃₋₅-Alkenylen- oder Butadienylen-Brücke bilden, die teilweise oder vollständig fluoriert oder ein- oder zweifach mit gleichen oder verschiedenen Substituenten ausgewählt aus Chlor, Hydroxy, C₁₋₃-Alkoxy und C₁₋₃-Alkyl substituiert sein kann, und in der eine oder zwei Methylengruppen unabhängig voneinander durch O, S, CO, SO, SO₂ oder NR^{N} ersetzt sein können, und in der im Falle einer Butadienylen-Brücke eine oder zwei Methingruppen durch ein N-Atom ersetzt sein können,
R⁵ Wasserstoff, Fluor, Chlor, Brom, Iod, Cyan, Nitro, C₁₋₃-Alkyl, C₁₋₃-Alkoxy, durch 1 bis 3-Fluoratome substituiertes Methyl- oder Methoxy, und
R^{N} H, C₁₋₄-Alkyl oder C₁₋₄-Alkylcarbonyl,
L unabhängig voneinander ausgewählt aus der Gruppe bestehend aus Fluor, Chlor, Brom, Iod, C₁₋₃-Alkyl, Difluormethyl, Trifluormethyl, C₁₋₃-Alkoxy, Difluormethoxy, Trifluormethoxy und Cyan,
R^{7a}, R^{7c} R^{7b}, unabhängig voneinander eine Bedeutung ausgewählt aus der Gruppe Wasserstoff, (C₁₋₁₈-Alkyl)carbonyl, (C₁₋₁₈-Alkyl)oxycarbonyl, Arylcarbonyl und Aryl-(C₁₋₃-alkyl)-carbonyl besitzen,
X Wasserstoff, Fluor, Chlor, Brom, Iod, C₁₋₆-Alkyl, C₂₋₆-Alkinyl, C₂₋₆-Alkenyl, C₃₋₇-Cycloalkyl-C₁₋₃-alkyl, C₅₋₇-Cycloalkenyl-C₁₋₃-alkyl, Aryl-C₁₋₃-alkyl, Heteroaryl-C₁₋₃-alkyl, C₁₋₆-Alkoxy, C₂₋₆-Alkenyloxy, C₂₋₆-Alkinyloxy, C₃₋₇-Cycloalkyloxy, C₅₋₇-Cycloalkenyloxy, Aryloxy, Heteroaryloxy, C₃₋₇-Cycloalkyl-C₁₋₃-alkyloxy, C₅₋₇-Cycloalkenyl-C₁₋₃-alkyloxy, Aryl-C₁₋₃-alkyloxy, Heteroaryl-C₁₋₃-alkyloxy, Aminocarbonyl, C₁₋₄-Alkylaminocarbonyl-, Di-(C₁₋₃-Alkyl)aminocarbonyl, Hydroxycarbonyl, C₁₋₄-Alkoxycarbonyl, Aminocarbonyl-C₁₋₃-alkyl-, C₁₋₄-Alkylaminocarbonyl-C₁₋₃-alkyl, Di-(C₁₋₃-Alkyl)aminocarbonyl-C₁₋₃-alkyl, Hydroxycarbonyl-C₁₋₃-alkyl, C₁₋₄-Alkoxycarbonyl-C₁₋₃-alkyl, C₃₋₇-Cycloalkyloxy-C₁₋₃-alkyl, C₅₋₇-Cycloalkenyloxy-C₁₋₃-alkyl, Aryloxy-C₁₋₃-alkyl, Heteroaryloxy-C₁₋₃-alkyl, C₁₋₄-Alkylsulfonyloxy, Arylsulfonyloxy, Aryl-C₁₋₃-alkyl-sulfonyloxy oder Cyan,
wobei eine unmittelbar mit dem Pyranosering verbundene Methylengruppe durch NR^{N}, S, CO, SO oder SO₂ ersetzt sein kann, und
wobei Alkyl-, Alkenyl-, Alkinyl-, Cycloalkyl- und Cycloalkenyl-Reste teilweise oder vollständig fluoriert oder ein- oder zweifach mit gleichen oder verschiedenen Substituenten ausgewählt aus Chlor, Cyan, Hydroxy, C₁₋₃-Alkoxy, C₁₋₃-Alkylsulfanyl, -NH₂, -NHR^{N}, -NR^{N}(C₁₋₃-Alkyl) und C₁₋₃-Alkyl substituiert sein können, und
wobei in Cycloalkyl- und Cycloalkenyl-Resten eine oder zwei Methylengruppen unabhängig voneinander durch O, S, CO, SO oder SO₂ ersetzt sein können, und
Z Sauerstoff, Methylen, Dimethylmethylen, 1,1-Cyclopropylen, Difluormethylen oder Carbonyl bedeutet;
wobei unter den bei der Definition der vorstehend genannten Reste erwähnten Arylgruppen Phenyl- oder Naphthylgruppen zu verstehen sind, welche unabhängig voneinander ein- oder zweifach mit gleichen oder verschiedenen Resten L substituiert sein können; und
unter den bei der Definition der vorstehend erwähnten Reste erwähnten Heteroarylgruppen eine Pyrrolyl-, Furanyl-, Thienyl-, Pyridyl-, Indolyl-, Benzofuranyl-, Benzothiophenyl-, Chinolinyl- oder Isochinolinylgruppe zu verstehen ist,
oder eine Pyrrolyl-, Furanyl-, Thienyl- oder Pyridylgruppe zu verstehen ist, in der eine oder zwei Methingruppen durch Stickstoffatome ersetzt sind,
oder eine Indolyl-, Benzofuranyl-, Benzothiophenyl-, Chinolinyl- oder Isochinolinylgruppe zu verstehen ist, in der eine bis drei Methingruppen durch Stickstoffatome ersetzt sind,
wobei die vorstehend erwähnten Heteroarylgruppen unabhängig voneinander ein- oder zweifach mit gleichen oder verschiedenen Resten L substituiert sein können;
wobei unter dem bei der Definition der vorstehend erwähnten Reste erwähnten N-Heterocycloalkyl-Rest ein gesättigter carbocyclischer Ring, der eine lmino-Gruppe im Ring aufweist, zu verstehen ist, der eine weitere Imino-Gruppe oder ein O- oder S-Atom im Ring aufweisen kann, und
wobei, soweit nichts anderes erwähnt wurde, die vorstehend erwähnten Alkylgruppen geradkettig oder verzweigt sein können,
deren Tautomere, deren Stereoisomere, deren Gemische und deren Salze, insbesondere deren physiologisch verträglichen Salze.

2. D-Pyranosyl-substituierte Phenyle gemäß Anspruch 1, **gekennzeichnet durch** die Formel IA 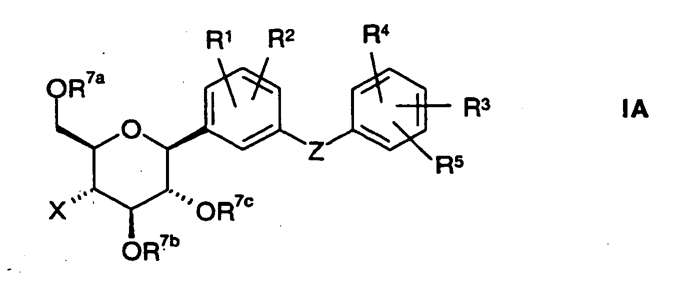 worin R¹ bis R⁵, X, Z, R^{7a}, R^{7b}, R^{7c} die Bedeutungen gemäß Anspruch 1 aufweisen.

3. D-Pyranosyl-substituierte Phenyle gemäß Anspruch 1, **gekennzeichnet durch** die Formel IB 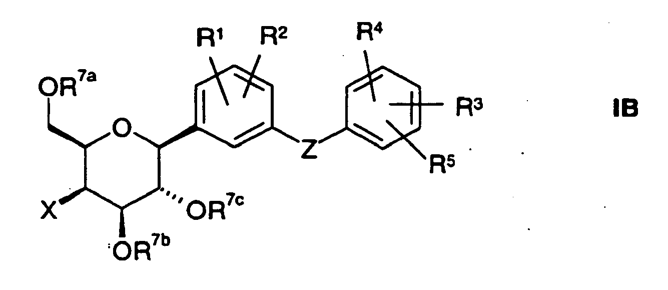 worin R¹ bis R⁵, X, Z, R^{7a}, R^{7b}, R^{7c} die Bedeutungen gemäß Anspruch 1 aufweisen.

4. D-Pyranosyl-substituierte Phenyle gemäß Anspruch 2, **gekennzeichnet durch** die Formel IA.2 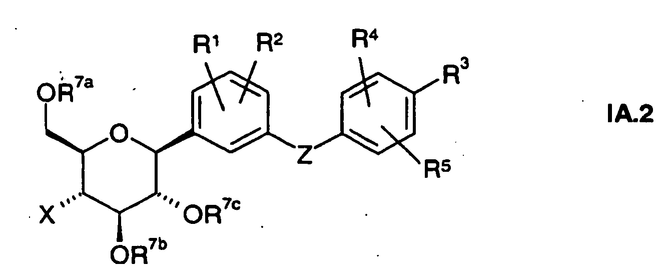 worin R¹ bis R⁵, X, Z, R^{7a}, R^{7b}, R^{7c} die Bedeutungen gemäß Anspruch 1 aufweisen.

5. D-Pyranosyl-substituierte Phenyle gemäß einem oder mehreren der Ansprüche 1 bis 4, **dadurch gekennzeichnet, dass**
R¹ Wasserstoff, Fluor, Chlor, Brom, Iod, C₁₋₆-Alkyl, C₂₋₆-Alkinyl, C₂₋₆-Alkenyl, C₃₋₇-Cycloalkyl, C₅₋₇-Cycloalkenyl, C₁₋₄-Alkylcarbonyl, Aminocarbonyl, C₁₋₄-Alkylaminocarbonyl, Di-(C₁₋₃-Alkyl)aminocarbonyl, C₁₋₄-Alkoxycarbonyl, C₁₋₄-Alkylamino, Di-(C₁₋₃-alkyl)amino, Pyrrolidin-1-yl, Piperidin-1-yl, Morpholin-4-yl, C₁₋₄-Alkylcarbonylamino, C₁₋₆-Alkyloxy, C₃₋₇-Cycloalkyloxy, C₅₋₇-Cycloalkenyloxy, C₁₋₄-Alkylsulfanyl, C₁₋₄-Alkylsulfonyl, C₃₋₇-Cycloalkylsulfanyl, C₃₋₇Cycloalkylsulfonyl, C₅₋₇-Cycloalkenylsulfanyl, C₅₋₇-Cycloalkenylsulfonyl, Hydroxy oder Cyan bedeutet,
wobei Alkyl-, Alkenyl-, Alkinyl-, Cycloalkyl- und Cycloalkenyl-Reste teilweise oder vollständig fluoriert oder ein- oder zweifach mit gleichen oder verschiedenen Substituenten ausgewählt aus Chlor, Hydroxy, C₁₋₃-Alkoxy und C₁₋₃-Alkyl substituiert sein können, und
wobei in Cycloalkyl- und Cycloalkenyl-Resten eine oder zwei Methylengruppen unabhängig voneinander durch O, S, CO, SO oder SO₂ ersetzt sein können, und
wobei in N-Heterocycloalkyl-Resten eine Methylengruppe durch CO oder SO₂ ersetzt sein kann.

6. D-Pyranosyl-substituierte Phenyle gemäß einem oder mehreren der Ansprüche 1 bis 5, **dadurch gekennzeichnet, dass**
R³ Wasserstoff, Fluor, Chlor, Brom, C₁₋₆-Alkyl, C₂₋₆-Alkinyl, C₂₋₆-Alkenyl, C₃₋₇-Cycloalkyl, C₃₋₇-Cycloalkyl-methyl, C₅₋₇-Cycloalkenyl, C₃₋₇-Cycloalkenyl-methyl, Aryl, Heteroaryl, C₁₋₄-Alkylcarbonyl, Aminocarbonyl, C₁₋₄-Alkylaminocarbonyl, Di-(C₁₋₃-Alkyl)aminocarbonyl, C₁₋₄-Alkoxycarbonyl, Di-(C₁₋₃-alkyl)amino, Pyrrolidin-1-yl, Piperidin-1-yl, Morpholin-4-yl, C₁₋₄-Alkylcarbonylamino, C₁₋₆-Alkoxy, C₃₋₇-Cycloalkyloxy, C₅₋₇-Cycloalkenyloxy, Aryloxy, Heteroaryloxy, C₁₋₄-Alkylsulfanyl, C₁₋₄-Alkylsulfonyl, C₃₋₇-Cycloalkylsulfanyl, C₃₋₇Cycloalkylsulfonyl, C₅₋₇-Cycloalkenylsulfanyl, C₅₋₇-Cycloalkenylsulfonyl, Hydroxy oder Cyan bedeutet,
wobei Alkyl-, Alkenyl-, Alkinyl-, Cycloalkyl- und Cycloalkenyl-Reste teilweise oder vollständig fluoriert oder ein- oder zweifach mit gleichen oder verschiedenen Substituenten ausgewählt aus Chlor, Hydroxy, C₁₋₃-Alkoxy und C₁₋₃-Alkyl substituiert sein können, und
wobei in Cycloalkyl- und Cycloalkenyl-Resten eine oder zwei Methylengruppen unabhängig voneinander durch O, S, CO, SO oder SO₂ ersetzt sein können, und
wobei in N-Heterocycloalkyl-Resten eine Methylengruppe durch CO oder SO₂ ersetzt sein kann,
wobei die Begriffe Aryl und Heteroaryl wie zuvor definiert sind und Aryl- und Heteroaryl-Gruppen unabhängig voneinander ein- oder zweifach mit gleichen oder verschiedenen Resten L substituiert sein können, wobei L wie in Anspruch 1 definiert ist.

7. D-Pyranosyl-substituierte Phenyle gemäß einem oder mehreren der Ansprüche 1 bis 6, **dadurch gekennzeichnet, dass**
X Wasserstoff, Fluor, Chlor, C₁₋₆-Alkyl, C₂₋₆-Alkinyl, C₂₋₆-Alkenyl, C₃₋₇-Cycloalkyl-C₁₋₃-alkyl, C₅₋₇-Cycloalkenyl-C₁₋₃-alkyl, Aryl-C₁₋₃-alkyl, Heteroaryl-C₁₋₃-alkyl, C₁₋₆-Alkoxy, C₂₋₆-Alkenyloxy, C₂₋₆-Alkinyloxy, C₃₋₇-Cycloalkyloxy, C₅₋₇-Cycloalkenyloxy, Aryloxy, Heteroaryloxy, Aryl-C₁₋₃-alkyloxy, C₃₋₇-Cycloalkyloxy-C₁₋₃-alkyl, C₅₋₇-Cycloalkenyloxy-C₁₋₃-alkyl, Aryloxy-C₁₋₃-alkyl, Heteroaryloxy-C₁₋₃-alkyl, Aminocarbonyl, C₁₋₄-Alkylaminocarbonyl, C₁₋₄-Alkylcarbonylamino-, N-(C₁₋₅-Alkyl)-N-(C₁₋₄-alkylcarbonyl)-amino, Hydroxycarbonyl, C₁₋₄-Alkoxycarbonyl, C₁₋₆-Alkylsulfonyl, Aminocarbonyl-C₁₋₃-alkyl, C₁₋₄-Alkylaminocarbonyl-C₁₋₃-alkyl, Di-(C₁₋₃-Alkyl)aminocarbonyl-C₁₋₃-alkyl, C₁₋₄-Alkoxycarbonyl-C₁₋₃alkyl, Amino; C₁₋₅-Alkylamino, N-(C₁₋₅-Alkyl)-N-(C₁₋₃-alkyl)-amino, Mercapto oder Cyan bedeutet,
wobei Alkyl-, Alkenyl-, Alkinyl-, Cycloalkyl- und Cycloalkenyl-Reste teilweise oder vollständig fluoriert oder ein- oder zweifach mit gleichen oder verschiedenen Substituenten ausgewählt aus Chlor, Cyan, Hydroxy, C₁₋₃-Alkoxy, C₁₋₃-Alkylsulfanyl, -NH₂, -NHR^{N}, -NR^{N}(C₁₋₃-Alkyl) und C₁₋₃-Alkyl substituiert sein können, und
wobei in den vorstehend genannten Cycloalkyl- und Cycloalkenyl-Resten eine oder zwei Methylengruppen unabhängig voneinander durch O, S, CO, SO oder SO₂ ersetzt sein können, und
wobei die Begriffe Aryl und Heteroaryl wie zuvor definiert sind und Aryl- und Heteroaryl-Gruppen unabhängig voneinander ein- oder zweifach mit gleichen oder verschiedenen Resten L substituiert sein können, wobei L wie in Anspruch 1 definiert ist.

8. D-Pyranosyl-substituierte Phenyle gemäß einem oder mehreren der Ansprüche 1 bis 7, **dadurch gekennzeichnet, dass** die Gruppe X gemäß
einer ersten Ausführungsform Wasserstoff, Chlor, Cyan, C₁₋₆-Alkyl, C₂₋₆-Alkinyl, C₂₋₆-Alkenyl, C₁₋₆-Alkylsulfonyl, Hydroxycarbonyl, C₁₋₄-Alkoxycarbonyl, Aminocarbonyl oder C₁₋₄-Alkylaminocarbonyl bedeutet, wobei Alkyl-Reste ein- oder mehrfach fluoriert oder einfach mit Hydroxy oder Cyan substituiert sein können; oder
gemäß einer zweiten Ausführungsform C₁₋₅-Alkyloxy, C₂₋₅-Alkenyloxy, C₂₋₅-Alkinyloxy, C₃₋₇-Cycloalkyloxy, Aryl-C₁₋₃-alkyloxy oder Aryloxy bedeutet; oder
gemäß einer dritten Ausführungsform Mercapto, C₁₋₅-Alkylsulfanyl, C₂₋₅-Alkenylsulfanyl, C₂₋₅-Alkinylsulfanyl, C₃₋₇-Cycloalkylsulfanyl oder Arylsulfanyl bedeutet; oder
gemäß einer vierten Ausführungsform Amino, C₁₋₅-Alkylamino, N-(C₁₋₅-Alkyl)-N-(C₁₋₃-alkyl)-amino, C₁₋₄-Alkylcarbonylamino, N-(C₁₋₅-Alkyl)-N-(C₁₋₄-alkylcarbonyl)-amino oder Arylamino bedeutet; oder
gemäß einer fünften Ausführungsform Fluor bedeutet;
wobei unter Aryl eine Phenyl- oder Naphthylgruppe, insbesondere Phenyl zu verstehen ist, welche ein- oder zweifach mit gleichen oder verschiedenen Substituenten L substituiert sein kann; und
L wie in Anspruch 1 definiert ist.

9. D-Pyranosyl-substituierte Phenyle gemäß einem oder mehreren der Ansprüche 1 bis 6, **dadurch gekennzeichnet, dass** die Gruppe X Brom, Iod, C₁₋₆-Alkylsulfonyloxy, Arylsulfonyloxy oder Aryl-C₁₋₃-alkyl-sulfonyloxy bedeutet,
wobei die vorstehend genannten Alkyl-Reste teilweise oder vollständig fluoriert oder ein- oder zweifach chloriert sein können und wobei die vorstehend genannten Aryl-Gruppen ein- oder zweifach mit gleichen oder verschiedenen Resten L substituiert sein können und L wie in Anspruch 1 definiert ist.

10. D-Pyranosyl-substituierte Phenyle gemäß einem oder mehreren der Ansprüche 1 bis 9, **dadurch gekennzeichnet, dass**
R² Wasserstoff, Fluor, Chlor, Brom, Methyl, Hydroxy, Methoxy, Ethoxy, Trifluormethoxy, Cyan, Nitro oder durch 1 bis.3 Fluoratome substituiertes Methyl bedeutet.

11. D-Pyranosyl-substituierte Phenyle gemäß einem oder mehreren der Ansprüche 1 bis 10, **dadurch gekennzeichnet, dass**
R⁴ und R⁵ unabhängig voneinander Wasserstoff oder Fluor bedeuten.

12. D-Pyranosyl-substituierte Phenyle gemäß einem oder mehreren der Ansprüche 1 bis 11, **dadurch gekennzeichnet, dass**
Z Sauerstoff oder Methylen bedeutet.

13. D-Pyranosyl-substituierte Phenyle gemäß einem oder mehreren der Ansprüche 1 bis 12, **dadurch gekennzeichnet, dass**
R^{7a}, R^{7b}, R^{7c} unabhängig voneinander Wasserstoff, (C₁₋₆-Alkyl)oxycarbonyl-, (C₁₋₈-Alkyl)carbonyl oder Benzoyl, vorzugsweise Wasserstoff bedeuten.

14. Physiologisch verträgliche Salze der Verbindungen nach einem oder mehreren der Ansprüche 1 bis 13 mit anorganischen oder organischen Säuren.

15. Verwendung einer Verbindung nach einem oder mehreren der Ansprüche 1 bis 13 oder eines physiologisch verträglichen Salzes gemäß Anspruch 14 als Arzneimittel.

16. Arzneimittel, enthaltend eine Verbindung nach einem oder mehreren der Ansprüche 1 bis 13 oder ein physiologisch verträgliches Salz gemäß Anspruch 14 neben gegebenenfalls einem oder mehreren inerten Trägerstoffen und/oder Verdünnungsmitteln.

17. Verwendung mindestens einer Verbindung nach einem oder mehreren der Ansprüche 1 bis 13 oder eines physiologisch verträglichen Salzes gemäß Anspruch 14 zur Herstellung eines Arzneimittels, das zur Behandlung oder Prophylaxe von Erkrankungen oder Zuständen geeignet ist, die durch Inhibierung des natriumabhängigen Glucose-Cotransporters SGLT beeinflussbar sind.

18. Verwendung mindestens einer Verbindung nach einem oder mehreren der Ansprüche 1 bis 13 oder eines physiologisch verträglichen Salzes gemäß Anspruch 14 zur Herstellung eines Arzneimittels, das zur Behandlung oder Prophylaxe von Stoffwechselerkrankungen geeignet ist.

19. Verwendung nach Anspruch 18, **dadurch gekennzeichnet, dass** die Stoffwechserkrankung ausgewählt ist aus der Gruppe bestehend aus Diabetes mellitus Typ 1 und Typ 2, diabetische Komplikationen, metabolische Azidose oder Ketose, reaktiver Hypoglykämie, Hyperinsulinämie, Glukosestoffwechselstörung, Insulinresistenz, Metabolischem Syndrom, Dyslipidämien unterschiedlichster Genese, Atherosklerose und verwandte Erkrankungen, Adipositas, Bluthochdruck, chronisches Herzversagen, Ödeme, Hyperurikämie.

20. Verwendung mindestens einer Verbindung nach mindestens einem der Ansprüche 1 bis 13 oder eines physiologisch verträglichen Salzes gemäß Anspruch 14 zur Herstellung eines Arzneimittels zur Inhibition des natriumabhängigen Glucose-Cotransporters SGLT.

21. Verwendung mindestens einer Verbindung nach mindestens einem der Ansprüche 1 bis 13 oder eines physiologisch verträglichen Salzes gemäß Anspruch 14 zur Herstellung eines Arzneimittels zum Verhindern der Degeneration von pankreatischen beta-Zellen und/oder zum Verbessern und/oder Wiederherstellen der Funktionalität von pankreatischen beta-Zellen.

22. Verwendung mindestens einer Verbindung nach mindestens einem der Ansprüche 1 bis 13 oder eines physiologisch verträglichen Salzes gemäß Anspruch 14 zur Herstellung von Diuretika und/oder Antihypertensiva.

23. Verfahren zur Herstellung eines Arzneimittels gemäß Anspruch 16, **dadurch gekennzeichnet, dass** auf nicht-chemischem Wege eine Verbindung nach mindestens einem der Ansprüche 1 bis 13 oder ein physiologisch verträgliches Salz gemäß Anspruch 14 in einen oder mehrere inerte Trägerstoffe und/oder Verdünnungsmittel eingearbeitet wird.

24. Verfahren zur Herstellung der Verbindungen der allgemeinen Formel I gemäß den Ansprüchen 1 bis 13, **dadurch gekennzeichnet, dass** eine Verbindung der allgemeinen Formel II 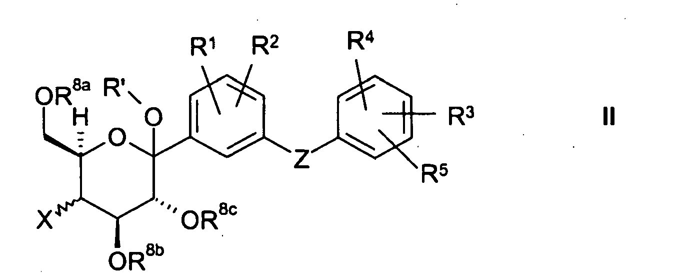 in der
R' H, C₁₋₄-Alkyl, (C₁₋₁₈-Alkyl)carbonyl, (C₁₋₁₈-Alkyl)oxycarbonyl, Arylcarbonyl oder Aryl-(C₁₋₃-alkyl)-carbonyl bedeutet, worin die Alkyl- oder Arylgruppen ein- oder mehrfach mit Halogen substituiert sein können;
R^{8a}, R^{8b}, R^{8c} unabhängig voneinander eine in Anspruch 1 oder 13 für die Reste R^{7a}, R^{7b}, R^{7c} angegebenen Bedeutungen besitzt, oder eine Benzyl-Gruppe, eine R^{a}R^{b}R^{c}Si-Gruppe oder eine Ketal- oder Acetalgruppe bedeutet,
wobei jeweils zwei benachbarte Reste R^{8a}, R^{8b}, R^{8c} eine cyclische Ketal- oder Acetalgruppe oder mit zwei Sauerstoffatomen des Pyranose-Rings einen substituierten 2,3-Oxydioxan-Ring bilden können, und wobei Alkyl-, Aryl- und/oder Benzylgruppen ein- oder mehrfach mit Halogen, C₁₋₃-Alkyl oder C₁₋₃-Alkoxy substituiert sein können und Benzyl-Gruppen auch mit einer Di-(C₁₋₃-alkyl)amino-Gruppe substituiert sein können; und
R^{a}, R^{b}, R^{c} unabhängig voneinander C₁₋₄-Alkyl, Aryl oder Aryl-C₁₋₃-alkyl bedeuten, wobei die Alkyl- oder Arylgruppen ein- oder mehrfach mit Halogen substituiert sein können;
wobei unter den bei der Definition der vorstehend genannten Reste erwähnten Arylgruppen Phenyl- oder Naphthylgruppen, vorzugsweise Phenylgruppen zu verstehen sind;
und X, Z, R¹ bis R⁵ die in den Ansprüchen 1 bis 13 angegebenen Bedeutungen besitzen,
mit einem Reduktionsmittel in Gegenwart einer Säure umgesetzt wird, wobei die eventuell vorhandenen Schutzgruppen gleichzeitig oder nachträglich abgespalten werden;
erforderlichenfalls ein bei den vorstehend beschriebenen Umsetzungen verwendeter Schutzrest wieder abgespalten wird und/oder
gewünschtenfalls eine so erhaltene Verbindung der allgemeinen Formel I selektiv an einer Hydroxygruppe derivatisiert oder diese substituiert wird und/oder
gewünschtenfalls eine so erhaltene Verbindung der allgemeinen Formel I in ihre Stereoisomere aufgetrennt wird und/oder
gewünschtenfalls eine so erhaltene Verbindung der allgemeinen Formel I in ihre physiologisch verträglichen Salze überführt wird.

25. Verfahren zur Herstellung der Verbindungen der allgemeinen Formel I gemäß den Ansprüchen 1 bis 13, in der R^{7a}, R^{7b} und R^{7c} Wasserstoff bedeuten, **dadurch gekennzeichnet, dass** in einer Verbindung der allgemeinen Formel III 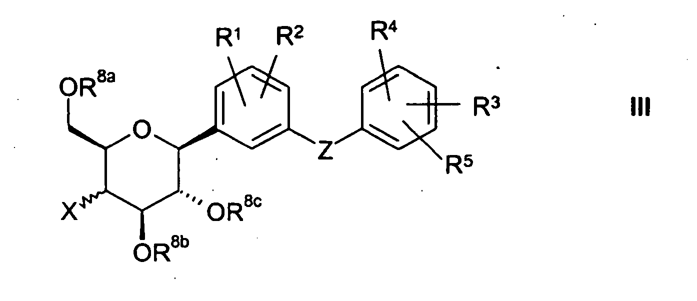 in der
R^{8a}, R^{8b}, R^{8c} unabhängig voneinander eine in Anspruch 1 oder 13 für die Reste R^{7a}, R^{7b}, R^{7c} angegebenen Bedeutungen, jedoch nicht Wasserstoff, besitzen oder eine R^{a}R^{b}R^{c}Si-Gruppe oder eine Ketal- oder Acetalgruppe bedeutet,
wobei jeweils zwei benachbarte Reste R^{8a}, R^{8b}, R^{8c} eine cyclische Ketal- oder Acetylgruppe bilden können, und wobei Alkyl- und/oder Arylgruppen ein- oder mehrfach halogeniert sein können; und
R^{a}, R^{b}, R^{c} unabhängig voneinander C₁₋₄-Alkyl, Aryl oder Aryl-C₁₋₃-alkyl bedeuten, wobei die Alkyl- oder Arylgruppen ein- oder mehrfach mit Halogen substituiert sein können;
wobei unter den bei der Definition der vorstehend genannten Reste erwähnten Arylgruppen Phenyl- oder Naphthylgruppen, vorzugsweise Phenylgruppen zu verstehen sind;
und X, Z, R¹ bis R⁵ die in den Ansprüchen 1 bis 13 angegebenen Bedeutungen besitzen,
die nicht Wasserstoff bedeutenden Reste R^{8a}, R^{8b} bzw. R^{8c} entfernt werden, und
erforderlichenfalls ein bei den vorstehend beschriebenen Umsetzungen verwendeter Schutzrest wieder abgespalten wird und/oder
gewünschtenfalls eine so erhaltene Verbindung der allgemeinen Formel I selektiv an einer Hydroxygruppe derivatisiert oder diese substituiert wird und/oder
gewünschtenfalls eine so erhaltene Verbindung der allgemeinen Formel I in ihre Stereoisomere aufgetrennt wird und/oder
eine so erhaltene Verbindung der allgemeinen Formel I in ihre Salze, insbesondere für die pharmazeutische Anwendung in ihre physiologisch verträglichen Salze, überführt wird.

## Claims

1. D-pyranosyl-substituted phenyls of general formula I 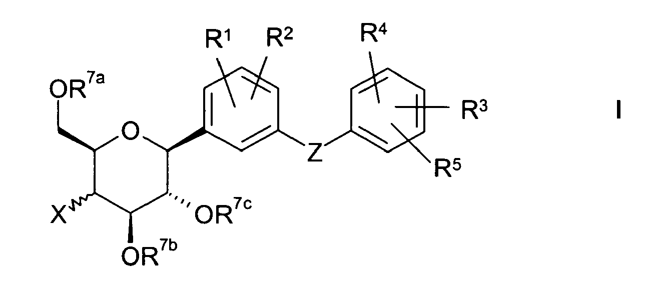 wherein
R¹ denotes hydrogen, fluorine, chlorine, bromine, iodine, C₁₋₆-alkyl, C₂₋₆-alkynyl, C₂₋₆-alkenyl, C₃₋₁₀-cycloalkyl, C₃₋₁₀-cycloalkyl-C₁₋₃-alkyl, C₅₋₁₀-cycloalkenyl, C₅₋₁₀-cycloalkenyl-C₁₋₃-alkyl, C₁₋₄-alkylcarbonyl, arylcarbonyl, heteroarylcarbonyl, aminocarbonyl, C₁₋₄-alkylaminocarbonyl, di-(C₁₋₃-alkyl)aminocarbonyl, pyrrolidin-1-ylcarbonyl, piperidin-1-ylcarbonyl, morpholin-4-ylcarbonyl, piperazin-1-ylcarbonyl, 4-(C₁₋₄-alkyl)piperazin-1-ylcarbonyl, C₁₋₄-alkoxycarbonyl, amino, C₁₋₄-alkylamino, di-(C₁₋₃-alkyl)amino, pyrrolidin-1-yl, piperidin-1-yl, morpholin-4-yl, piperazin-1-yl, 4-(C₁₋₄-alkyl)piperazin-1-yl, C₁₋₄-alkylcarbonylamino, C₁₋₆-alkyloxy, C₃₋₁₀-cycloalkyloxy, C₅₋₁₀-cycloalkenyloxy, aryloxy, C₁₋₄-alkylsulphanyl, C₁₋₄-alkylsulphinyl, C₁₋₄-alkylsulphonyl, C₃₋₁₀-cycloalkylsulphanyl, C₃₋₁₀-cycloalkylsulphinyl, C₃₋₁₀-cycloalkylsulphonyl, C₅₋₁₀-cycloalkenylsulphanyl, C₅₋₁₀-cycloalkenylsulphinyl, C₅₋₁₀-cycloalkenylsulphonyl, arylsulphanyl, arylsulphinyl, arylsulphonyl, hydroxy, cyano or nitro,
wherein alkyl, alkenyl, alkynyl, cycloalkyl and cycloalkenyl groups may be partly or completely fluorinated or may be mono- or disubstituted by identical or different substituents selected from chlorine, hydroxy, C₁₋₃-alkoxy and C₁₋₃-alkyl, and
in cycloalkyl and cycloalkenyl groups one or two methylene groups may be replaced independently of one another by O, S, CO, SO or SO₂, and
in N-heterocycloalkyl groups a methylene group may be replaced by CO or SO₂, and
R² denotes hydrogen, fluorine, chlorine, bromine, hydroxy, C₁₋₄-alkyl, C₁₋₄-alkoxy, cyano or nitro, wherein alkyl groups may be mono- or polysubstituted by fluorine, or
in the event that R¹ and R² are bound to two adjacent C atoms of the phenyl ring, R¹ and R² may be joined together such that R¹ and R² together form a C₃₋₅-alkylene, C₃₋₅-alkenylene or butadienylene bridge, which may be partly or completely fluorinated or may be mono- or disubstituted by identical or different substituents selected from chlorine, hydroxy, C₁₋₃-alkoxy and C₁₋₃-alkyl, and wherein one or two methylene groups may be replaced independently of one another by O, S, CO, SO, SO₂ or NR^{N}, and wherein in the case of a butadienylene bridge one or two methyne groups may be replaced by an N atom,
R³ denotes hydrogen, fluorine, chlorine, bromine, iodine, C₁₋₆-alkyl, C₂₋₆-alkynyl, C₂₋₆-alkenyl, C₃₋₁₀-cycloalkyl, C₃₋₁₀-cycloalkyl-C₁₋₃-alkyl, C₅₋₁₀-cycloalkenyl, C₅₋₁₀-cycloalkenyl-C₁₋₃-alkyl, aryl, heteroaryl, C₁₋₄-alkylcarbonyl, arylcarbonyl, heteroarylcarbonyl, aminocarbonyl, C₁₋₄-alkylaminocarbonyl, di-(C₁₋₃-alkyl)aminocarbonyl, pyrrolidin-1-ylcarbonyl, piperidin-1-ylcarbonyl, morpholin-4-ylcarbonyl, piperazin-1-ylcarbonyl, 4-(C₁₋₄-alkyl)piperazin-1-ylcarbonyl, hydroxycarbonyl, C₁₋₄-alkoxycarbonyl, C₁₋₄-alkylamino, di-(C₁₋₃-alkyl)amino, pyrrolidin-1-yl, piperidin-1-yl, morpholin-4-yl, piperazin-1-yl, 4-(C₁₋₄-alkyl)piperazin-1-yl, C₁₋₄-alkylcarbonylamino, arylcarbonylamino, heteroarylcarbonylamino, C₁₋₄-alkylsulphonylamino, arylsulphonylamino, C₁₋₆-alkoxy, C₃₋₇-cycloalkyloxy, C₅₋₇-cycloalkenyloxy, aryloxy, heteroaryloxy, C₁₋₄-alkylsulphanyl, C₁₋₄-alkylsulphinyl, C₁₋₄-alkylsulphonyl, C₃₋₁₀-cycloalkylsulphanyl, C₃₋₁₀-cycloalkylsulphinyl, C₃₋₁₀-cycloalkylsulphonyl, C₅₋₁₀-cycloalkenylsulphanyl, C₅₋₁₀-cycloalkenylsulphinyl, C₅₋₁₀-cyclo-alkenylsulphonyl, arylsulphanyl, arylsulphinyl, arylsulphonyl, amino, hydroxy, cyano or nitro,
wherein alkyl, alkenyl, alkynyl, cycloalkyl- and cycloalkenyl groups may be partly or completely fluorinated or mono- or disubstituted by identical or different substituents selected from chlorine, hydroxy, C₁₋₃-alkoxy and C₁₋₃-alkyl, and
in cycloalkyl and cycloalkenyl groups one or two methylene groups may be replaced independently of one another by O, S, CO, SO or SO₂, and
in N-heterocycloalkyl groups a methylene group may be substituted by CO or SO₂, and
R⁴ denotes hydrogen, fluorine, chlorine, bromine, iodine, cyano, nitro, C₁₋₃-alkyl, C₁₋₃-alkoxy or methyl or methoxy substituted by 1 to 3 fluorine atoms, or
if R³ and R⁴ are bound to two adjacent C atoms of the phenyl ring, R³ and R⁴ may be joined together such that R³ and R⁴ together form a C₃₋₅-alkylene, C₃₋₅-alkenylene or butadienylene bridge, which may be partly or completely fluorinated or mono- or disubstituted by identical or different substituents selected from chlorine, hydroxy, C₁₋₃-alkoxy and C₁₋₃-alkyl, and wherein one or two methylene groups may be replaced independently of one another by O, S, CO, SO, SO₂ or NR^{N}, and wherein in the case of a butadienylene bridge one or two methyne groups may be replaced by an N atom,
R⁵ denotes hydrogen, fluorine, chlorine, bromine, iodine, cyano, nitro, C₁₋₃-alkyl, C₁₋₃-alkoxy or methyl or methoxy substituted by 1 to 3 fluorine atoms, and
R^{N} denotes H, C₁₋₄-alkyl or C₁₋₄-alkylcarbonyl,
L are selected independently of one another from among fluorine, chlorine, bromine, iodine, C₁₋₃-alkyl, difluoromethyl, trifluoromethyl, C₁₋₃-alkoxy, difluoromethoxy, trifluoromethoxy and cyano,
R^{7a}, R^{7b}, R^{7c} independently of one another have a meaning selected from among hydrogen, (C₁₋₁₈-alkyl)carbonyl, (C₁₋₁₈-alkyl)oxycarbonyl, arylcarbonyl and aryl-(C₁₋₃-alkyl)-carbonyl,
X denotes hydrogen, fluorine, chlorine, bromine, iodine, C₁₋₆-alkyl, C₂₋₆-alkynyl, C₂₋₆-alkenyl, C₃₋₇-cycloalkyl-C₁₋₃-alkyl, C₅₋₇-cycloalkenyl-C₁₋₃-alkyl, aryl-C₁₋₃-alkyl, heteroaryl-C₁₋₃-alkyl, C₁₋₆-alkoxy, C₂₋₆-alkenyloxy, C₂₋₆-alkynyloxy, C₃₋₇-cycloalkyloxy, C₅₋₇-cycloalkenyloxy, aryloxy, heteroaryloxy, C₃₋₇-cycloalkyl-C₁₋₃-alkyloxy, C₅₋₇-cycloalkenyl-C₁₋₃-alkyloxy, aryl-C₁₋₃-alkyloxy, heteroaryl-C₁₋₃-alkyloxy, aminocarbonyl, C₁₋₄-alkylaminocarbonyl, di-(C₁₋₃-alkyl)aminocarbonyl, hydroxycarbonyl, C₁₋₄-alkoxycarbonyl, aminocarbonyl-C₁₋₃-alkyl, C₁₋₄-alkylaminocarbonyl-C₁₋₃-alkyl, di-(C₁₋₃-alkyl)aminocarbonyl-C₁₋₃-alkyl, hydroxycarbonyl-C₁₋₃-alkyl, C₁₋₄-alkoxycarbonyl-C₁₋₃-alkyl, C₃₋₇-cycloalkyloxy-C₁₋₃-alkyl, C₅₋₇-cycloalkenyloxy-C₁₋₃-alkyl, aryloxy-C₁₋₃-alkyl, heteroaryloxy-C₁₋₃-alkyl, C₁₋₄-alkylsulphonyloxy, arylsulphonyloxy, aryl-C₁₋₃-alkylsulphonyloxy or cyano,
wherein a methylene group directly linked to the pyranose ring may be replaced by NR^{N}, S, CO, SO or SO₂, and
alkyl, alkenyl, alkynyl, cycloalkyl and cycloalkenyl groups may be partly or completely fluorinated or mono- or disubstituted by identical or different substituents selected from chlorine, cyano, hydroxy, C₁₋₃-alkoxy, C₁₋₃-alkylsulphanyl, -NH₂, -NHR^{N}, -NR^{N}(C₁₋₃-alkyl) and C₁₋₃-alkyl, and
in cycloalkyl and cycloalkenyl groups one or two methylene groups may be replaced independently of one another by O, S, CO, SO or SO₂, and
Z denotes oxygen, methylene, dimethylmethylene, 1,1-cyclopropylene, difluoromethylene or carbonyl;
while by the aryl groups mentioned in the definition of the above-mentioned groups are meant phenyl or naphthyl groups, which may be mono- or disubstituted independently of one another by identical or different groups L; and
by the heteroaryl groups mentioned in the definition of the above-mentioned groups are meant a pyrrolyl, furanyl, thienyl, pyridyl, indolyl, benzofuranyl, benzothiophenyl, quinolinyl or isoquinolinyl group,
or a pyrrolyl, furanyl, thienyl or pyridyl group, wherein one or two methyne groups are replaced by nitrogen atoms,
or an indolyl, benzofuranyl, benzothiophenyl, quinolinyl or isoquinolinyl group,
wherein one to three methyne groups are replaced by nitrogen atoms,
while the above-mentioned heteroaryl groups may independently of one another be mono- or disubstituted by identical or different groups L;
while by the N-heterocycloalkyl group mentioned in the definition of the above-mentioned groups is meant a saturated carbocyclic ring which comprises an imino group in the ring, which may comprise a further imino group or an O or S atom in the ring, and
unless otherwise stated, the above-mentioned alkyl groups may be straight-chain or branched,
the tautomers, the stereoisomers, the mixtures thereof and the salts thereof, particularly the physiologically acceptable salts thereof.

2. D-pyranosyl-substituted phenyls according to claim 1, **characterised by** the formula IA 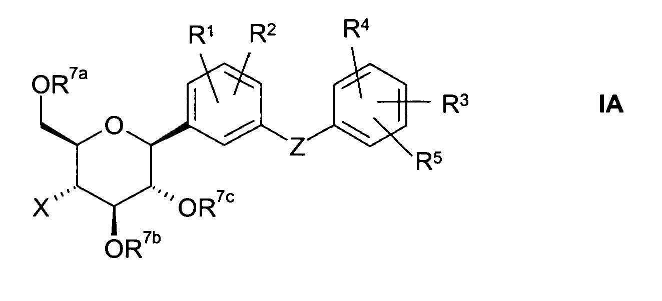 wherein R¹ to R⁵, X, Z, R^{7a}, R^{7b}, R^{7c} have the meanings according to claim 1.

3. D-pyranosyl-substituted phenyls according to claim 1, **characterised by** the formula IB 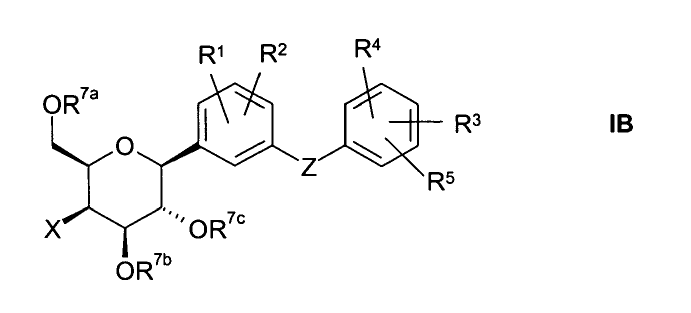 wherein R¹ to R⁵, X, Z, R^{7a}, R^{7b}, R^{7c} have the meanings according to claim 1.

4. D-pyranosyl-substituted phenyls according to claim 2, **characterised by** the formula IA.2 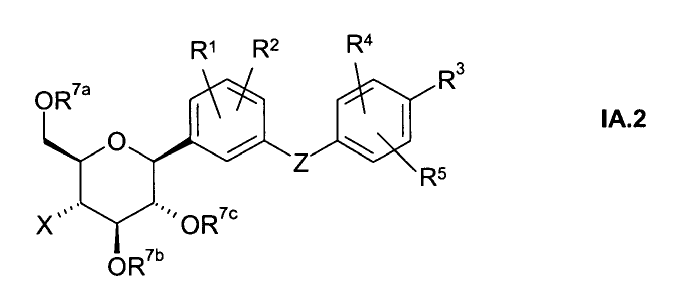 wherein R¹ to R⁵, X, Z, R^{7a}, R^{7b}, R^{7c} have the meanings according to claim 1.

5. D-pyranosyl-substituted phenyls according to one or more of claims 1 to 4, **characterised in that**
R¹ denotes hydrogen, fluorine, chlorine, bromine, iodine, C₁₋₆-alkyl, C₂₋₆-alkynyl, C₂₋₆-alkenyl, C₃₋₇-cycloalkyl, C₅₋₇-cycloalkenyl, C₁₋₄-alkylcarbonyl, aminocarbonyl, C₁₋₄-alkylaminocarbonyl, di-(C₁₋₃-alkyl)aminocarbonyl, C₁₋₄-alkoxycarbonyl, C₁₋₄-alkylamino, di-(C₁₋₃-alkyl)amino, pyrrolidin-1-yl, piperidin-1-yl, morpholin-4-yl, C₁₋₄-alkylcarbonylamino, C₁₋₆-alkyloxy, C₃₋₇-cycloalkyloxy, C₅₋₇-cycloalkenyloxy, C₁₋₄-alkylsulphanyl, C₁₋₄-alkylsulphonyl, C₃₋₇-cycloalkylsulphanyl, C₃₋₇-cycloalkylsulphonyl, C₅₋₇-cycloalkenylsulphanyl, C₅₋₇-cycloalkenylsulphonyl, hydroxy or cyano,
wherein alkyl, alkenyl, alkynyl, cycloalkyl and cycloalkenyl groups may be partly or completely fluorinated or mono- or disubstituted by identical or different substituents selected from chlorine, hydroxy, C₁₋₃-alkoxy and C₁₋₃-alkyl, and
in cycloalkyl and cycloalkenyl groups one or two methylene groups may be replaced independently of one another by O, S, CO, SO or SO₂, and
in N-heterocycloalkyl groups a methylene group may be replaced by CO or SO₂.

6. D-pyranosyl-substituted phenyls according to one or more of claims 1 to 5, **characterised in that**
R³ denotes hydrogen, fluorine, chlorine, bromine, C₁₋₆-alkyl, C₂₋₆-alkynyl, C₂₋₆-alkenyl, C₃₋₇-cycloalkyl, C₃₋₇-cycloalkyl-methyl, C₅₋₇-cycloalkenyl, C₃₋₇-cycloalkenyl-methyl, aryl, heteroaryl, C₁₋₄-alkylcarbonyl, aminocarbonyl, C₁₋₄-alkylaminocarbonyl, di-(C₁₋₃-alkyl)aminocarbonyl, C₁₋₄-alkoxycarbonyl, di-(C₁₋₃-alkyl)amino, pyrrolidin-1-yl, piperidin-1-yl, morpholin-4-yl, C₁₋₄-alkylcarbonylamino, C₁₋₆-alkoxy, C₃₋₇-cyclo-alkyloxy, C₅₋₇-cycloalkenyloxy, aryloxy, heteroaryloxy, C₁₋₄-alkylsulphanyl, C₁₋₄-alkylsulphonyl, C₃₋₇-cycloalkylsulphanyl, C₃₋₇cycloalkylsulphonyl, C₅₋₇-cycloalkenylsulphanyl, C₅₋₇-cycloalkenylsulphonyl, hydroxy or cyano,
wherein alkyl, alkenyl, alkynyl, cycloalkyl and cycloalkenyl groups may be partly or completely fluorinated or mono- or disubstituted by identical or different substituents selected from chlorine, hydroxy, C₁₋₃-alkoxy and C₁₋₃-alkyl, and
in cycloalkyl and cycloalkenyl groups one or two methylene groups may be replaced independently of one another by O, S, CO, SO or SO₂, and
in N-heterocycloalkyl groups a methylene group may be replaced by CO or SO₂,
the terms aryl and heteroaryl are as hereinbefore defined and aryl and heteroaryl groups may independently of one another be mono- or disubstituted by identical or different groups L, where L is defined as in claim 1.

7. D-pyranosyl-substituted phenyls according to one or more of claims 1 to 6, **characterised in that**
X denotes hydrogen, fluorine, chlorine, C₁₋₆-alkyl, C₂₋₆-alkynyl, C₂₋₆-alkenyl, C₃₋₇-cyoloalkyl-C₁₋₃-alkyl, C₅₋₇-cycloalkenyl-C₁₋₃-alkyl, aryl-C₁₋₃-alkyl, heteroaryl-C₁₋₃-alkyl, C₁₋₆-alkoxy, C₂₋₆-alkenyloxy, C₂₋₆-alkynyloxy, C₃₋₇-cycloalkyloxy, C₅₋₇-cycloalkenyloxy, aryloxy, heteroaryloxy, aryl-C₁₋₃-alkyloxy, C₃₋₇-cycloalkyloxy-C₁₋₃-alkyl, C₅₋₇-cycloalkenyloxy-C₁₋₃-alkyl, aryloxy-C₁₋₃-alkyl, heteroaryloxy-C₁₋₃-alkyl, aminocarbonyl, C₁₋₄-alkylaminocarbonyl, C₁₋₄-alkylcarbonylamino, N-(C₁₋₅-alkyl)-N-(C₁₋₄-alkylcarbonyl)-amino, hydroxycarbonyl, C₁₋₄-alkoxycarbonyl, C₁₋₆-alkylsulphonyl, aminocarbonyl-C₁₋₃-alkyl, C₁₋₄-alkylaminocarbonyl-C₁₋₃-alkyl, di-(C₁₋₃-alkyl)aminocarbonyl-C₁₋₃-alkyl, C₁₋₄-alkoxycarbonyl-C₁₋₃-alkyl, amino, C₁₋₅-alkylamino, N-(C₁₋₅-alkyl)-N-(C₁₋₃-alkyl)-amino, mercapto or cyano,
wherein alkyl, alkenyl, alkynyl, cycloalkyl and cycloalkenyl groups may be partly or completely fluorinated or mono- or disubstituted by identical or different substituents selected from chlorine, cyano, hydroxy, C₁₋₃-alkoxy, C₁₋₃-alkylsulphanyl, -NH₂, -NHR^{N}, -NR^{N}(C₁₋₃-alkyl) and C₁₋₃-alkyl_{,} and
in the above-mentioned cycloalkyl and cycloalkenyl groups one or two methylene groups may be replaced independently of one another by O, S, CO, SO or SO₂, and
the terms aryl and heteroaryl are as hereinbefore defined and aryl and heteroaryl groups may independently of one another be mono- or disubstituted by identical or different groups L, where L is defined as in claim 1.

8. D-pyranosyl-substituted phenyls according to one or more of claims 1 to 7, **characterised in that** the group X
according to a first embodiment denotes hydrogen, chlorine, cyano, C₁₋₆-alkyl, C₂₋₆-alkynyl, C₂₋₆-alkenyl, C₁₋₆-alkylsulphonyl, hydroxycarbonyl, C₁₋₄-alkoxycarbonyl, aminocarbonyl or C₁₋₄-alkylaminocarbonyl, while alkyl groups may be mono- or polyfluorinated or monosubstituted by hydroxy or cyano; or
according to a second embodiment denotes C₁₋₅-alkyloxy, C₂₋₅-alkenyloxy, C₂₋₅-alkynyloxy, C₃₋₇-cycloalkyloxy, aryl-C₁₋₃-alkyloxy or aryloxy; or
according to a third embodiment denotes mercapto, C₁₋₅-alkylsulphanyl, C₂₋₅-alkenylsulphanyl, C₂₋₅-alkynylsulphanyl, C₃₋₇-cycloalkylsulphanyl or arylsulphanyl; or
according to a fourth embodiment denotes amino, C₁₋₅-alkylamino, N-(C₁₋₅-alkyl)-N-(C₁₋₃-alkyl)-amino, C₁₋₄-alkylcarbonylamino, N-(C₁₋₅-alkyl)-N-(C₁₋₄-alkylcarbonyl)-amino or arylamino; or
according to a fifth embodiment denotes fluorine;
while by aryl is meant a phenyl or naphthyl group, particularly phenyl, which may be mono- or disubstituted by identical or different substituents L; and
L is defined as in claim 1.

9. D-pyranosyl-substituted phenyls according to one or more of claims 1 to 6, **characterised in that** the group X denotes bromine, iodine, C₁₋₆-alkylsulphonyloxy, arylsulphonyloxy or aryl-C₁₋₃-alkyl-sulphonyloxy,
wherein the above-mentioned alkyl groups may be partly or completely fluorinated or mono- or dichlorinated and the above-mentioned aryl groups may be mono- or disubstituted by identical or different groups L and L is defined as in claim 1.

10. D-pyranosyl-substituted phenyls according to one or more of claims 1 to 9, **characterised in that**
R² denotes hydrogen, fluorine, chlorine, bromine, methyl, hydroxy, methoxy, ethoxy, trifluoromethoxy, cyano, nitro or methyl substituted by 1 to 3 fluorine atoms.

11. D-pyranosyl-substituted phenyls according to one or more of claims 1 to 10, **characterised in that**
R⁴ and R⁵ independently of one another denote hydrogen or fluorine.

12. D-pyranosyl-substituted phenyls according to one or more of claims 1 to 11, **characterised in that**
Z denotes oxygen or methylene.

13. D-pyranosyl-substituted phenyls according to one or more of claims 1 to 12, **characterised in that**
R^{7a}, R^{7b}, R^{7c} independently of one another denote hydrogen, (C₁₋₆-alkyl)oxycarbonyl, (C₁₋₈-alkyl)carbonyl or benzoyl, preferably hydrogen.

14. Physiologically acceptable salts of the compounds according to one or more of claims 1 to 13 with inorganic or organic acids.

15. Use of a compound according to one or more of claims 1 to 13 or a physiologically acceptable salt according to claim 14 as a pharmaceutical composition.

16. Pharmaceutical composition, containing a compound according to one or more of claims 1 to 13 or a physiologically acceptable salt according to claim 14 optionally together with one or more inert carriers and/or diluents.

17. Use of at least one compound according to one or more of claims 1 to 13 or a physiologically acceptable salt according to claim 14 for preparing a pharmaceutical composition which is suitable for the treatment or prevention of diseases or conditions which can be influenced by inhibiting the sodium-dependent glucose cotransporter SGLT.

18. Use of at least one compound according to one or more of claims 1 to 13 or a physiologically acceptable salt according to claim 14 for preparing a pharmaceutical composition which is suitable for the treatment or prevention of metabolic disorders.

19. Use according to claim 18, **characterised in that** the metabolic disorder is selected from among type 1 and type 2 diabetes mellitus, complications of diabetes, metabolic acidosis or ketosis, reactive hypoglycaemia, hyperinsulinaemia, glucose metabolic disorder, insulin resistance, metabolic syndrome, dyslipidaemias of different origins, atherosclerosis and related diseases, obesity, high blood pressure, chronic heart failure, oedema and hyperuricaemia.

20. Use of at least one compound according to at least one of claims 1 to 13 or a physiologically acceptable salt according to claim 14 for preparing a pharmaceutical composition for inhibiting the sodium-dependent glucose cotransporter SGLT.

21. Use of at least one compound according to at least one of claims 1 to 13 or a physiologically acceptable salt according to claim 14 for preparing a pharmaceutical composition for preventing the degeneration of pancreatic beta cells and/or for improving and/or restoring the functionality of pancreatic beta cells.

22. Use of at least one compound according to at least one of claims 1 to 13 or a physiologically acceptable salt according to claim 14 for preparing diuretics and/or antihypertensives.

23. Process for preparing a pharmaceutical composition according to claim 16, **characterised in that** a compound according to at least one of claims 1 to 13 or a physiologically acceptable salt according to claim 14 is incorporated in one or more inert carriers and/or diluents by a non-chemical method.

24. Process for preparing the compounds of general formula I according to claims 1 to 13, **characterised in that** a compound of general formula II 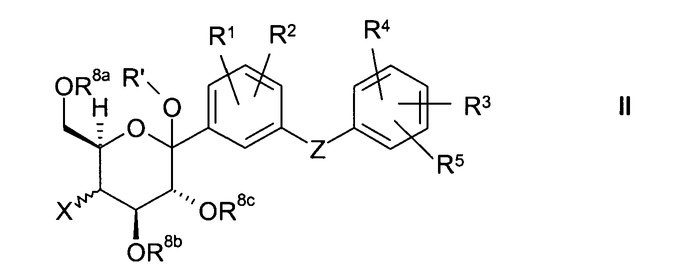 wherein
R' denotes H, C₁₋₄-alkyl, (C₁₋₁₈-alkyl)carbonyl, (C₁₋₁₈-alkyl)oxycarbonyl, arylcarbonyl or aryl-(C₁₋₃-alkyl)-carbonyl, wherein the alkyl or aryl groups may be mono- or polysubstituted by halogen;
R^{8a}, R^{8b}, R^{8c} independently of one another have one of the meanings given in claim 1 or 13 for the groups R^{7a}, R^{7b}, R^{7c}, or denote a benzyl group, a R^{a}R^{b}R^{c}Si group or a ketal or acetal group, while in each case two adjacent groups R^{8a}, R^{8b}, R^{8c} may form a cyclic ketal or acetal group or with two oxygen atoms of the pyranose ring may form a substituted 2,3-oxydioxan ring , and alkyl, aryl and/or benzyl groups may be mono- or polysubstituted by halogen, C₁₋₃-alkyl or C₁₋₃-alkoxy and benzyl groups may also be substituted by a di-(C₁₋₃-alkyl)amino group; and
R^{a}, R^{b}, R^{c} independently of one another represent C₁₋₄-alkyl, aryl or aryl-C₁₋₃-alkyl, wherein the alkyl or aryl groups may be mono- or polysubstituted by halogen;
while by the aryl groups mentioned in the definition of the above-mentioned groups are meant phenyl or naphthyl groups, preferably phenyl groups;
and X, Z, R¹ to R⁵ have the meanings given in claims 1 to 13,
is reacted with a reducing agent in the presence of an acid, while any protecting groups present are cleaved at the same time or subsequently;
any protecting group used during the reactions described above is cleaved and/or
if desired a compound of general formula I thus obtained is selectively derivatised at a hydroxy group or the latter is substituted and/or
if desired a compound of general formula I thus obtained is resolved into its stereoisomers and/or
if desired a compound of general formula I thus obtained is converted into the physiologically acceptable salts thereof.

25. Process for preparing the compounds of general formula I according to claims 1 to 13, wherein R^{7a}, R^{7b} and R^{7c} represent hydrogen, **characterised in that** in a compound of general formula III 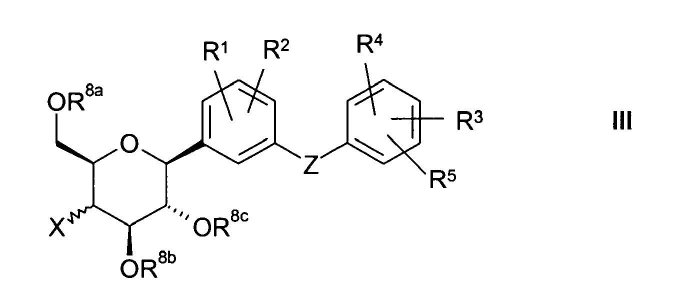 wherein
R^{8a}, R^{8b}, R^{8c} independently of one another have one of the meanings given in claim 1 or 13 for the groups R^{7a}, R^{7b}, R^{7c}, but not hydrogen, or denote an R^{a}R^{b}R^{c}Si group or a ketal or acetal group, while in each case two adjacent groups R^{8a}, R^{8b}, R^{8c} may form a cyclic ketal or acetyl group, and alkyl and/or aryl groups may be mono- or polyhalogenated; and
R^{a}, R^{b}, R^{c} independently of one another represent C₁₋₄-alkyl, aryl or aryl-C₁₋₃-alkyl, wherein the alkyl or aryl groups may be mono- or polysubstituted by halogen;
while by the aryl groups mentioned in the definition of the above-mentioned groups are meant phenyl or naphthyl groups, preferably phenyl groups;
and X, Z, R¹ to R⁵ have the meanings given in claims 1 to 13,
the groups R^{8a}, R^{8b} or R^{8c} which do not represent hydrogen are removed, and any protecting group used during the reactions described above is cleaved and/or
if desired a compound of general formula I thus obtained is selectively derivatised at a hydroxy group or the latter is substituted and/or
if desired a compound of general formula I thus obtained is resolved into its stereoisomers and/or
a compound of general formula I thus obtained is converted into the salts thereof, particularly for pharmaceutical use into the physiologically acceptable salts thereof.

## Revendications

1. Phényles à substituant D-pyranosyle de formule générale I 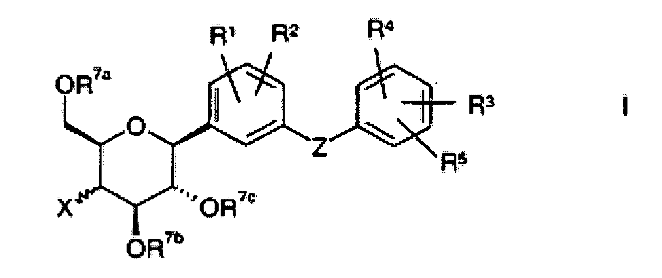 où
R¹ représente un hydrogène, un fluor, un chlore, un brome, un iode, un alkyle en C₁₋₆, un alcinyle en C₂₋₆, un alcényle en C₂₋₆, un cycloalkyle en C₃₋₁₀, un C₃₋₁₀-cycloalkyl-C₁₋₃-alkyle, un cycloalcényle en C₅₋₁₀, un C₅₋₁₀-cycloalcényle-C₁₋₃-alkyle, un alkylcarbonyle en C₁₋₄, un arylcarbonyle, un hétéroarylcarbonyle, un aminocarbonyle, un alkylaminocarbonyle en C₁₋₄, un di-(C₁₋₃-alkyle)-aminocarbonyle, un pyrrolidin-1-ylcarbonyle, un pipéridin-1-ylcarbonyle, un morpholin-4-ylcarbonyle, un pipérazin-1-ylcarbonyle, un 4-(C₁₋₄-alkyl)pipérazin-1-ylcarbonyle, un alkoxycarbonyle en C₁₋₄, un amino, un alkylamino en C₁₋₄, un di-(C₁₋₃-alkyl)amino, un pyrrolidin-1-yle, un pipéridin-1-yle, un morpholin-4-yle, un pipérazin-1-yle, un 4-(C₁₋₄-alkyl)pipérazin-1-yle, un alkylcarbonylamino en C₁₋₄, un alkyloxy en C₁₋₆, un cycloalkyloxy C₃₋₁₀, un cycloalcényloxy en C₅₋₁₀, un aryloxy, un alkylsulfanyle en C₁₋₄, un alkylsulfinyle en C₁₋₄, un alkylsulfonyle en C₁₋₄, un cycloalkylsulfanyle en C₃₋₁₀, un cycloalkylsulfinyle en C₃₋₁₀, un cycloalkylsulfonyle en C₃₋₁₀, un cycloalcénylsulfanyle en C₅₋₁₀, un cycloalcénylsulfinyle en C₅₋₁₀, un cycloalcénylsulfonyle en C₅₋₁₀, un arylsulfanyle, un arylsulfinyle, un arylsulfonyle, un hydroxy, un cyano ou un nitro,
les résidus alkyle, alcényle, alcinyle, cycloalkyle et cycloalcényle pouvant être en partie ou entièrement fluorés ou substitués une ou deux fois avec les mêmes ou différents substituants sélectionnés parmi chlore, hydroxy, alkoxy en C₁₋₃ et alkyle en C₁₋₃, et
dans les résidus cycloalkyle et cycloalcényle, un ou deux groupes méthyle peuvant être remplacés indépendamment les uns des autres par O, S, CO, SO ou SO₂, et
dans les résidus N-hétérocycloalkyle un groupe méthyle pouvant être remplacé par CO ou SO₂, et
R² représente un hydrogène, un fluor, un chlore, un brome, un hydroxy, un alkyle en C₁₋₄, un alkoxy en C₁₋₄, un cyano ou un nitro, les résidus alkyle pouvant être substitués une ou plusieurs fois avec du fluor, ou
dans le cas où R¹ et R² sont liés à deux atomes C adjacents l'un à l'autre du cycle phényle, R¹ et R² peuvent être liés l'un à l'autre de telle manière que R¹ et R² constituent ensemble un pont alkyle en C₃₋₅, alcénylène en C₃₋₅ ou butadiénylène, qui peuvent être en partie ou entièrement fluorés ou substitués une ou deux fois avec les mêmes ou différents substituants sélectionnés parmi chlore, hydroxy, alkoxy en C₁₋₃ et alkyle en C₁₋₃, et dans lequel un ou deux groupes méthyle peuvent être remplacés indépendamment les uns des autres par O, S, CO, SO, SO₂ ou NR^{N}, et dans le cas d'un pont butadiénylène un ou deux groupes méthine peuvent être remplacés par un atome d'azote,
R³ représente un hydrogène, un fluor, un chlore, un brome, un iode, un alkyle en C₁₋₆, un alcinyle en C₂₋₆, un alcényle en C₂₋₆, un cycloalkyle en C₃₋₁₀, un C₃₋₁₀-cycloalkyle-C₁₋₃-alkyle, un cycloalcényle en C₅₋₁₀, un C₅₋₁₀-cycloalcényl-C₁₋₃-alkyle, un aryle, un hétéroaryle, un alkylcarbonyle en C₁₋₄, un arylcarbonyle, un hétéroarylcarbonyle, un aminocarbonyle, un alkylaminocarbonyle en C₁₋₄, un di-(C₁₋₃-alkyl)aminocarbonyle, un pyrrolidin-1-ylcarbonyle, un pipéridin-1-yl-carbonyle, un morpholin-4-ylcarbonyle, un pipérazin-1-ylcarbonyle, un 4-(C₁₋₄-alkyl)-pipérazin-1-ylcarbonyle, un hydroxycarbonyle, un alkoxycarbonyle en C₁₋₄, un alkylamino en C₁₋₄, un di-(C₁₋₃-alkyl)amino, un pyrrolidin-1-yle, un pipéridin-1-yle, un morpholin-4-yle, un pipérazin-1-yle, un 4-(C₁₋₄-alkyl)pipérazin-1-yle, un alkylcarbonylamino en C₁₋₄, un arylcarbonylamino, un hétéroarylcarbonylamino, un alkylsulfonylamino en C₁₋₄, un arylsulfonylamino, un alkoxy en C₁₋₆, un cycloalkyloxy en C₃₋₇, un cycloalcényloxy en C₅₋₇, un aryloxy, un hétéroaryloxy, un alkylsulfanyle en C₁₋₄, un alkylsulfinyle en C₁₋₄, un alkylsulfonyle en C₁₋₄, un cycloalkylsulfanyle en C₃₋₁₀, un cycloalkylsulfinyle en C₃₋₁₀, un cycloalkylsulfonyle en C₃₋₁₀, un cycloalcénylsulfanyle en C₅₋₁₀, un cycloalcénylsulfinyle en C₅₋₁₀, un cycloalcénylsulfonyle en C₅₋₁₀, un arylsulfanyle, un arylsulfinyle, un arylsulfonyle, un amino, un hydroxy, un cyano ou nitro,
les résidus alkyle, alcényle, alcinyle, cycloalkyle et cycloalcényle pouvant être en partie ou entièrement fluorés ou substitués une ou deux fois avec les mêmes ou différents substituants sélectionnés parmi chlore, hydroxy, alkoxy en C₁₋₃ et alkyle en C₁₋₃, dans les résidus cycloalkyle et cycloalcényle un ou deux groupes méthyle pouvant être remplacés indépendamment les uns des autres par O, S, CO, SO ou SO₂, dans les résidus N-hétérocycloalkyle un groupe méthyle pouvant être substitué par CO ou SO₂, et
R⁴ représentant un hydrogène, un fluor, un chlore, un brome, un iode, un cyano, un nitro, un alkyle en C₁₋₃, un alkoxy en C₁₋₃, un méthyle ou méthoxy substitué par 1 à 3 atomes de fluor, ou
dans le cas où R³ et R⁴ sont liés à deux atomes C adjacents l'un à l'autre du cycle phényle, R³ et R⁴ sont liés l'un à l'autre de telle manière que R³ et R⁴ constituent ensemble un pont alkylène en C₃₋₅, alcénylène en C₃₋₅ ou butadiénylène, qui peuvent être en partie ou entièrement fluorés ou substitués une ou deux fois avec les mêmes ou différents substituants sélectionnés parmi chlore, hydroxy, alkoxy en C₁₋₃ et alkyle en C₁₋₃, et dans lequel un ou deux groupes méthyle peuvent être remplacés indépendamment l'un de l'autre par O, S, CO, SO, SO₂ ou NR^{N}, et dans le cas d'un pont butadiénylène un ou deux groupes méthine peuvent être remplacés par un atome d'azote,
R⁵ représentant un hydrogène, un fluor, un chlore, un brome, un iode, un cyano, un nitro, un alkyle en C₁₋₃, un alkoxy en C₁₋₃, un méthyle ou méthoxy substitué par 1 à 3 atomes de fluor, et
R^{N} représentant H, un alkyle en C₁₋₄ ou un alkylcarbonyle en C₁₋₄,
chaque L étant indépendamment choisi dans le groupe constitué par le fluor, le chlore, le brome, l'iode, l'alkyle en C₁₋₃, le difluorométhyle, le trifluorométhyle, l'alkoxy en C₁₋₃, le difluorométhoxy, le trifluorométhoxy et le cyano,
R^{7a}, R^{7b},
R^{7c} représentant, indépendamment les uns des autres, un atome d'hydrogène, (C₁₋₁₈-alkyl)carbonyle, (C₁₋₁₈-alkyl)oxycarbonyle, arylcarbonyle et aryl-(C₁₋₃-alkyl)-carbonyle,
X représentant un hydrogène, un fluor, un chlore, un brome, un iode, un alkyle en C₁₋₆, un alcinyle en C₂₋₆, un alcényle en C₂₋₆, un C₃₋₇-cycloalkyl-C₁₋₃-alkyle, un C₅₋₇-cycloalcényl-C₁₋₃-alkyle, un aryl-C₁₋₃-alkyle, un hétéroaryl-C₁₋₃-alkyle, un alkoxy en C₁₋₆, un alcényloxy en C₂₋₆, un alcinyloxy en C₂₋₆, un cycloalkyloxy en C₃₋₇, un cycloalcényloxy en C₅₋₇, un aryloxy, un hétéroaryloxy, un C₃₋₇-cycloalkyl-C₁₋₃-alkyloxy, un C₅₋₇-cycloalcényl-C₁₋₃-alkyloxy, un aryl-C₁₋₃-alkyloxy, un hétéroaryl-C₁₋₃-alkyloxy, un aminocarbonyle, un alkylaminocarbonyle en C₁₋₄, un di-(C₁₋₃-alkyl)aminocarbonyle, un hydroxycarbonyle, un alkoxycarbonyle en C₁₋₄, un aminocarbonyl-C₁₋₃-alkyle, un C₁₋₄-alkylaminocarbonyl-C₁₋₃-alkyle, un di-(C₁₋₃-alkyl)aminocarbonyl-C₁₋₃-alkyle, un hydroxycarbonyl-C₁₋₃-alkyle, un C₁₋₄-alkoxycarbonyl-C₁₋₃-alkyle, un C₃₋₇-cycloalkyloxy-C₁₋₃-alkyle, un C₅₋₇-cycloalcényloxy-C₁₋₃-alkyle, un aryloxy-C₁₋₃-alkyle, un hétéroaryloxy-C₁₋₃-alkyle, un alkylsulfonyloxy en C₁₋₄, un arylsulfonyloxy, un aryl-C₁₋₃-alkyl-sulfonyloxy ou un cyano,
un groupe méthylène lié directement au cycle pyranose pouvant être remplacé par NR^{N}, S, CO, SO ou SO², et
les résidus alkyle, alcényle, alcinyle, cycloalkyle et cycloalcényle pouvant être en partie ou entièrement fluorés ou substitués une ou deux fois avec les mêmes ou différents substituants sélectionnés parmi chlore, cyano, hydroxy, alkoxy en C₁₋₃, alkylsulfanyle en C₁₋₃, -NH₂, -NHR^{N}, -NR^{N}(C₁₋₃-alkyle) et alkyle en C₁₋₃, et
dans les résidus cycloalkyle et cycloalcényle, un ou deux groupes méthylène pouvant être remplacés indépendamment les uns des autres par O, S, CO, SO ou SO₂, et
Z représentant un atome d'oxygène, un méthylène, un diméthylméthylène, un 1,1-cyclopropylène, un difluorméthylène ou un carbonyle ;
les groupes aryle mentionnés dans la définition des résidus ci-devant étant des groupes phényle ou naphtyle qui peuvent être indépendamment substitués une ou deux fois avec les mêmes ou différents résidus L ; et
les groupes hétéroaryle mentionnés dans la définition des résidus ci-devant étant chacun un groupe pyrrolyle, furanyle, thiényle, pyridyle, indolyle, benzofuranyle, benzothiophényle, quinoléinyle ou isoquinoléinyle,
ou un groupe pyrrolyle, furanyle, thienyle ou pyridyle, dans lequel un ou plusieurs groupes méthines sont remplacés par des atomes d'azote,
ou un groupe indolyle, benzofuranyle, benzothiophényle, quinoléinyle ou isoquinoléinyle, dans lequel un à trois groupes méthines sont remplacés par des atomes d'azote,
les groupes hétéroaryle précédemment cités pouvant être indépendamment les uns des autres substitués une ou deux fois par les mêmes ou différents résidus L ;
les résidus N-hétérocycloalkyle mentionnés dans la définition des résidus ci-devant étant un cycle carbocyclique saturé, qui comporte un groupe imino dans le cycle, pouvant comporter un autre groupe imino ou un atome O ou S dans le cycle, et
dans la mesure où rien d'autre n'a été précisé, les groupes alkyle précédemment mentionnés pouvant être linéaires ou ramifiés,
et leurs tautomères, leurs stéréoisomères, leurs mélanges et leurs sels, en particulier les sels physiologiquement acceptables.

2. Phényles à substituant D-pyranosyle selon la revendication 1, **caractérisés par** la formule IA 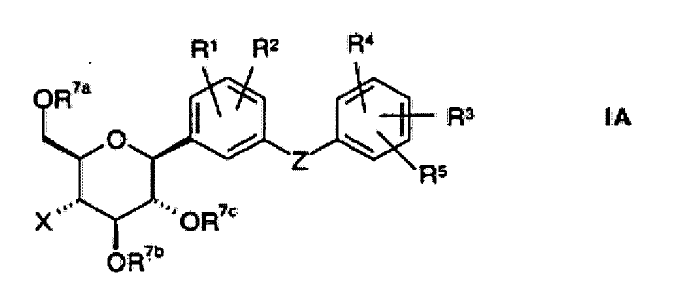 dans laquelle R¹ à R⁵, X, Z, R^{7a}, R^{7b}, R^{7c} ont les significations données dans la revendication 1.

3. Phényles à substituant D-pyranosyle selon la revendication 1, **caractérisés par** la formule IB 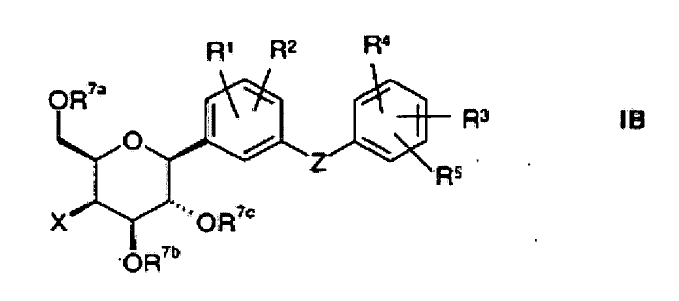 dans laquelle R¹ à R⁵, X, Z, R^{7a}, R^{7b}, R^{7c} ont les significations données dans la revendication 1.

4. Phényles à substituant D-pyranosyle selon la revendication 2, **caractérisés par** la formule IA.2 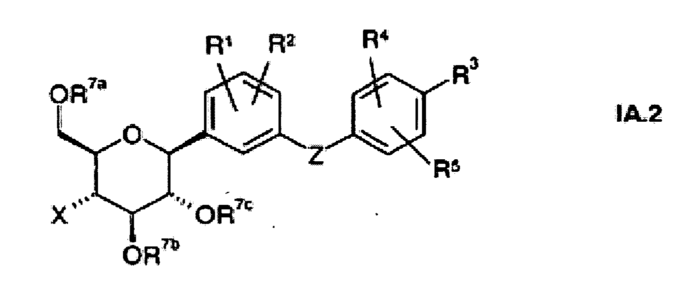 dans laquelle R¹ à R⁵, X, Z, R^{7a}, R^{7b}, R^{7c} ont les significations données dans la revendication 1.

5. Phényles à substituant D-pyranosyle selon l'une quelconque ou plusieurs des revendications 1 à 4, **caractérisés en ce que**
R¹ représente un hydrogène, un fluor, un chlore, un brome, un iode, un alkyle en C₁₋₆, un alcinyle en C₂₋₆, un alcényle en C₂₋₆, un cycloalkyle en C₃₋₇, un cycloalcényle en C₅₋₇, un alkylcarbonyle en C₁₋₄, un aminocarbonyle, un alkylaminocarbonyle en C₁₋₄, un di-(C₁₋₃-alkyle)-aminocarbonyle, un alkoxycarbonyle en C₁₋₄, un alkylamino en C₁₋₄, un di-(C₁₋₃-alkyl)amino, un pyrrolidin-1-yle, un pipéridin-1-yle, un morpholin-4-yle, un alkylcarbonylamino en C₁₋₄, un alkyloxy en C₁₋₆, un cycloalkyloxy C₃₋₇, un cycloalcényloxy en C₅₋₇, un alkylsulfanyle en C₁₋₄, un alkylsulfonyle en C₁₋₄, un cycloalkylsulfanyle en C₃₋₇, un cycloalkylsulfonyle en C₃₋₇, un cycloalcénylsulfanyle en C₅₋₇, un cycloalcénylsulfonyle en C₅₋₇, un hydroxy ou un cyano,
les résidus alkyle, alcényle, alcinyle, cycloalkyle et cycloalcényle pouvant être en partie ou entièrement fluorés ou substitués une ou deux fois avec les mêmes ou différents substituants sélectionnés parmi chlore, hydroxy, alkoxy en C₁₋₃ et alkyle en C₁₋₃, et
dans les résidus cycloalkyle et cycloalcényle, un ou deux groupes méthyle pouvant être remplacés indépendamment les uns des autres par O, S, CO, SO ou SO₂, et
dans les résidus N-hétérocycloalkyle un groupe méthylène pouvant être remplacé par CO ou SO₂.

6. Phényles à substituant D-pyranosyle selon l'une quelconque ou plusieurs des revendications 1 à 5, **caractérisés en ce que**
R³ représente un hydrogène, un fluor, un chlore, un brome, un alkyle en C₁₋₆, un alcinyle en C₂₋₆, un alcényle en C₂₋₆, un cycloalkyle en C₃₋₇, un C₃₋₇-cycloalkyl-méthyle, un cycloalcényle en C₅₋₇, un C₃₋₇-cycloalcényl-méthyle, un aryle, un hétéroaryle, un alkylcarbonyle en C₁₋₄, un aminocarbonyle, un alkylaminocarbonyle en C₁₋₄, un di-(C₁₋₃-alkyl)aminocarbonyle, un alkoxycarbonyle en C₁₋₄, un di-(C₁₋₃-alkyl)amino, un pyrrolidin-1-yle, un pipéridin-1-yle, un morpholin-4-yle, un alkylcarbonylamino en C₁₋₄, un alkoxy en C₁₋₆, un cycloalkyloxy en C₃₋₇, un cycloalcényloxy en C₁₋₇, un aryloxy, un hétéroaryloxy, un alkylsulfanyle en C₁₋₄, un alkylsulfonyle en C₁₋₄, un cycloalkylsulfanyle en C₃₋₇, un cycloalkylsulfonyle en C₃₋₇, un cycloalcénylsulfanyle en C₅₋₇, un cycloalcénylsulfonyle en C₅₋₇, un hydroxy ou un cyano,
les résidus alkyle, alcényle, alcinyle, cycloalkyle et cycloalcényle pouvant être en partie ou entièrement fluorés ou substitués une ou deux fois avec les mêmes ou différents substituants sélectionnés parmi chlore, hydroxy, alkoxy en C₁₋₃ et alkyle en C₁₋₃, et
dans les résidus cycloalkyle et cycloalcényle un ou deux groupes méthyle pouvant être remplacés indépendamment les uns des autres par O, S, CO, SO ou SO₂, et
dans les résidus N-hétérocycloalkyle un groupe méthyle pouvant être substitué par CO ou SO₂,
les termes aryle et hétéroaryle étant tels que définis ci-avant et les groupes aryle et hétéroaryle pouvant être indépendamment les uns des autres substitués une ou deux fois avec les mêmes ou différents résidus L, L étant défini comme dans la revendication 1.

7. Phényles à substituant D-pyranosyle selon l'une quelconque ou plusieurs des revendications 1 à 6, **caractérisés en ce que**
X représente un hydrogène, un fluor, un chlore, un alkyle en C₁₋₆, un alcinyle en C₂₋₆, un alcényle en C₂₋₆, un C₃₋₇-cycloalkyl-C₁₋₃-alkyle, un C₅₋₇-cycloalcényl-C₁₋₃-alkyle, un aryl-C₁₋₃-alkyle, un hétéroaryl-C₁₋₃-alkyle, un alkoxy en C₁₋₆, un alcényloxy en C₂₋₆,
un alcinyloxy en C₂₋₆, un cycloalkyloxy en C₃₋₇, un cycloalcényloxy en C₅₋₇, un 6, aryloxy, un hétéroaryloxy, un aryl-C₁₋₃-alkyloxy, un C₃₋₇-cycloalkyloxy-C₁₋₃-alkyle, un C₅₋₇-cycloalcényloxy-C₁₋₃-alkyl, un aryloxy-C₁₋₃-alkyle, un hétéroaryloxy-C₁₋₃-alkyle, un aminocarbonyle, un C₁₋₄-alkyl-aminocarbonyle, un C₁₋₄-alkylcarbonylamino-, un N-(C₁₋₅-alkyl)-N-(C₁₋₄-alkylcarbonyl)-amino, un hydroxycarbonyle, un alkoxycarbonyle en C₁₋₄, un alkylsulfonyle en C₁₋₆, un aminocarbonyl-C₁₋₃-alkyle, un C₁₋₄-alkylaminocarbonyl-C₁₋₃-alkyle, un di-(C₁₋₃-alkyl)aminocarbonyl-C₁₋₃-alkyle, un C₁₋₄-alcoxycarbonyl-C₁₋₃-alkyle, un amino, un alkylamino en C₁₋₅, un N-(C₁₋₅-alkyl)-N-(C₁₋₃-alkyl)-amino, un mercapto ou un cyano, les résidus alkyle, alcényle, alkinyle, cycloalkyle et cycloalcényle pouvant être en partie ou entièrement fluorés ou substitués une ou deux fois avec les mêmes ou différents substituants sélectionnés parmi chlore, cyano, hydroxy, alcoxy en C₁₋₃, alkylsulfanyle en C₁₋₃, -NH₂, -NHR^{N}, -NR^{N}(C₁₋₃-alkyl) et l'alkyle en C₁₋₃, et
dans les résidus cycloalkyle et cycloalcényle précédemment cités, un ou deux groupes méthylène pouvant être remplacés indépendamment les uns des autres par O, S, CO, SO ou SO₂, et
les termes aryle et hétéroaryle étant tels que définis ci-avant et les groupes aryle et hétéroaryle pouvant être indépendamment les uns des autres substitués une ou deux fois avec les mêmes ou différents résidus L, L étant défini comme dans la revendication 1.

8. Phényles à substituant D-pyranosyle selon l'une quelconque ou plusieurs des revendications 1 à 7, **caractérisés en ce que** le groupe X représente
selon un premier mode de réalisation un hydrogène, un chlore, un groupe cyano, alkyle en C₁₋₆, alcinyle en C₂₋₆, alcényle en C₂₋₆, alkylsulfonyle en C₁₋₆, hydroxycarbonyle, alcoxycarbonyle en C₁₋₄, aminocarbonyle ou alkylaminocarbonyle en C₁₋₄, les résidus alkyle pouvant être fluorés une ou plusieurs fois ou substitués une fois avec un hydroxy ou cyano ; ou
selon un deuxième mode de réalisation, un groupe alkyloxy en C₁₋₅, alcényloxy en C₂₋₅, alcinyloxy en C₂₋₅, cycloalkyloxy en C₃₋₇, aryl-C₁₋₃-alkyloxy ou un aryloxy ; ou
selon un troisième mode de réalisation, un groupe mercapto, alkylsulfanyle en C₁₋₅, alcénylsulfanyle en C₂₋₅, un alcinylsulfanyle en C₂₋₅, cycloalkylsulfanyle en C₃₋₇ ou un arylsulfanyle ; ou
selon un quatrième mode de réalisation, un groupe amino, alkylamino en C₁₋₅, un N-(C₁₋₅-alkyl)-N-(C₁₋₃-alkyl)-amino, C₁₋₄-alkylcarbonylamino, N-(C₁₋₅-alkyl)-N-(C₁₋₄-alkylcarbonyl)-amino ou arylamino ; ou
selon un cinquième mode de réalisation, un atome de fluor ;
devant être entendu par aryle un groupe phényle ou naphtyle, en particulier phényle, pouvant être substitué une ou deux fois avec les mêmes ou différents substituts L ; et
L étant défini comme dans la revendication 1.

9. Phényles à substituant D-pyranosyle selon l'une quelconque ou plusieurs des revendications 1 à 6, **caractérisés en ce que** le groupe X représente un brome, un iode, un alkylsulfonyloxy en C₁₋₆, un arylsulfonyloxy ou un aryl-C₁₋₃-alkyl-sulfonyloxy, les résidus alkyle précédemment cités pouvant être en partie ou entièrement fluorés ou chlorés une ou deux fois et les groupes aryle précédemment cités pouvant être substitués une ou deux fois avec les mêmes ou différents résidus L et L étant défini comme dans la revendication 1.

10. Phényles à substituant D-pyranosyle selon l'une quelconque ou plusieurs des revendications 1 à 9, **caractérisés en ce que**
R² représente un hydrogène, un fluor, un chlore, un brome, un méthyle, un hydroxy, un méthoxy, un éthoxy, un trifluorméthoxy, un cyano, un nitro ou un méthyle substitué par 1 à 3 atomes de fluor.

11. Phényles à substituant D-pyranosyle selon l'une quelconque ou plusieurs des revendications 1 à 10, **caractérisés en ce que**
R⁴ et R⁵ représentent indépendamment l'un de l'autre un hydrogène ou un fluor.

12. Phényles à substituant D-pyranosyle selon l'une quelconque ou plusieurs des revendications 1 à 11, **caractérisés en ce que**
Z représente un oxygène ou un méthyle.

13. Phényles à substituant D-pyranosyle selon l'une quelconque ou plusieurs des revendications 1 à 12, **caractérisés en ce que**
R^{7a}, R^{7b}, R^{7c} représentent, chacun indépendamment les uns des autres, un hydrogène, un (C₁₋₆-alkyl)oxycarbonyle, un (C₁₋₈-alkyl)carbonyle ou un benzoyle, de préférence un hydrogène.

14. Sels physiologiquement acceptables des composés selon l'une quelconque ou plusieurs des revendications 1 à 13 avec des acides minéraux ou organiques.

15. Utilisation d'un composé selon l'une quelconque ou plusieurs des revendications 1 à 13 ou d'un sel physiologiquement acceptable selon la revendication 14, comme médicament.

16. Médicament, contenant un composé selon l'une quelconque ou plusieurs des revendications 1 à 13 ou un sel physiologiquement acceptable selon la revendication 14, avec éventuellement un ou plusieurs excipients et/ou diluants inertes.

17. Utilisation d'au moins un composé selon l'une quelconque ou plusieurs des revendications 1 à 13 ou d'un sel physiologiquement acceptable selon la revendication 14, pour la fabrication d'un médicament adapté au traitement ou à la prophylaxie de maladies ou d'états qui peuvent être influencés par l'inhibition des cotransporteurs glucose-sodium SGLT.

18. Utilisation d'au moins un composé selon l'une quelconque ou plusieurs des revendications 1 à 13 ou d'un sel physiologiquement acceptable selon la revendication 14 pour la fabrication d'un médicament adapté au traitement ou à la prophylaxie des maladies du métabolisme.

19. Utilisation selon la revendication 18, **caractérisée en ce que** la maladie du métabolisme est choisie dans le groupe formé par le diabète de type 1 et de type 2, les complications diabétiques, l'acidose métabolique ou la cétose, l'hypoglycémie réactive, l'hyperinsulinémie, les troubles du métabolisme du glucose, l'insulinorésistance, le syndrome métabolique, les dyslipidémies de différentes origines, l'athérosclérose et les maladies liées, l'adipose, l'hypertension, l'insuffisance cardiaque chronique, les oedèmes, l'hyperuricémie.

20. Utilisation d'au moins un composé selon au moins l'une quelconque des revendications 1 à 13 ou d'un sel physiologiquement acceptable selon la revendication 14 pour la fabrication d'un médicament destiné à l'inhibition des cotransporteurs glucose-sodium SGLT.

21. Utilisation d'au moins un composé selon au moins l'une quelconque des revendications 1 à 13 ou d'un sel physiologiquement acceptable selon la revendication 14 pour la fabrication d'un médicament destiné à freiner la dégénerescence des cellules-bêta du pancréas et/ou à améliorer et/ou à rétablir la fonction des cellules-bêta du pancréas.

22. Utilisation d'au moins un composé selon au moins l'une quelconque des revendications 1 à 13 ou d'un sel physiologiquement acceptable selon la revendication 14 pour la fabrication de diurétiques et/ou d'anti-hypertenseurs.

23. Procédé de fabrication d'un médicament selon la revendication 16, **caractérisé en ce qu'**un composé selon au moins l'une quelconque des revendications 1 à 13 ou un sel physiologiquement acceptable selon la revendication 14 est incorporé d'une manière non chimique dans un ou plusieurs excipients et/ou diluants inertes.

24. Procédé de préparation d'un composé de formule générale I selon les revendications 1 à 13, **caractérisé en ce que** l'on fait réagir un composé de formule générale II 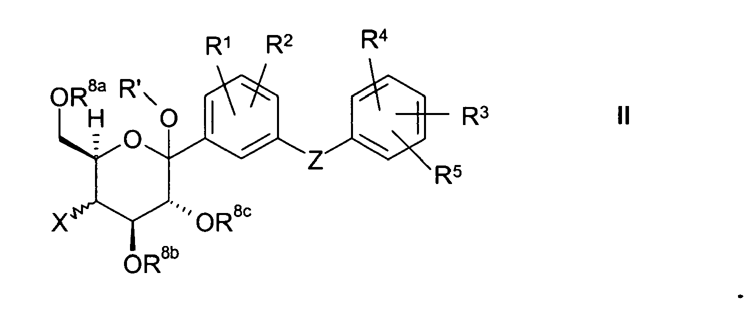 où
R' représente H, un alkyle en C₁₋₄, un (C₁₋₁₈-alkyl)carbonyle, un (C₁₋₁₈-alkyl)oxycarbonyle, un arylcarbonyle ou un aryl-(C₁₋₃-alkyl)-carbonyle, les groupes alkyle ou aryle pouvant être substitués une ou plusieurs fois par un halogène ;
R^{8a}, R^{8b},
R^{8c} ont indépendamment les uns des autres l'un des sens donnés dans la revendication 1 ou 13 pour les résidus R^{7a}, R^{7b}, R^{7c} ou désignent un groupe benzyle, un groupe R^{a}R^{b}R^{c}Si ou un groupe cétal ou acétal, deux résidus R^{8a}, R^{8b}, R^{8c} respectivement adjacents pouvant constituer un groupe cétal ou acétal cyclique ou, avec deux atomes d'oxygène du cycle pyranose un cycle 2,3-oxydioxane substitué, et les groupes alkyle, aryle et/ou benzyle pouvant être substitués une ou plusieurs fois avec un halogène, un alkyle en C₁₋₃ ou un alkoxy en C₁₋₃ et les groupes benzyle pouvant aussi être substitués avec un groupe di-(C₁₋₃-alkyl)amino ; et
R^{a}, R^{b}, R^{c} représentent, chacun indépendamment les uns des autres, un groupe alkyle en C₁₋₄, aryle ou aryl-C₁₋₃-alkyle, les groupes alkyle ou aryle pouvant être substitués une ou plusieurs fois avec des halogènes ;
devant être entendus par les groupes aryle mentionnés dans la définition des résidus précédemment cités des groupes phényle ou naphthyle, de préférence des groupes phényle ;
et X, Z, R¹ à R⁵ ayant la signification donnée dans les revendications 1 à 13,
avec un agent réducteur en présence d'un acide, les éventuels groupes protecteurs présents étant séparés simultanément ou ultérieurement ;
si nécessaire, un résidu protecteur utilisé dans la réaction précédemment décrite est à nouveau séparé et/ou
si on le souhaite, on prépare un dérivé du composé ainsi obtenu de formule générale I de façon ciblée au niveau d'un groupe hydroxy ou celui-ci est substitué et/ou
si on le souhaite, on sépare un composé ainsi obtenu de formule générale I en ses stéréoisomères et/ou
si on le souhaite, on convertit un composé ainsi obtenu de formule générale I en son sel physiologiquement acceptable.

25. Procédé de préparation d'un composé de formule générale I selon les revendications 1 à 13, dans lequel R^{7a}, R^{7b} et R^{7c} désignent un hydrogène, **caractérisé en ce que**, dans un composé de formule générale III 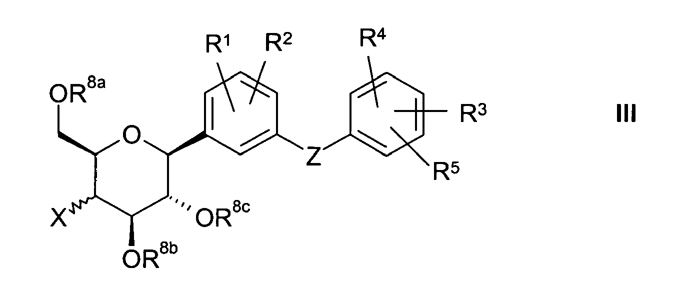 où
R^{8a}, R^{8b},
R^{8c} ont indépendamment les uns des autres l'un des sens donnés dans la revendication 1 ou 13 pour les résidus R^{7a}, R^{7b}, R^{7c}, à l'exception d'un atome d'hydrogène, ou désignent un groupe R^{a}R^{b}R^{c}Si ou un groupe cétal ou acétal, deux résidus R^{8a}, R^{8b}, R^{8c} respectivement adjacents pouvant constituer un groupe cétal ou acétal cyclique, et les groupes alkyle et/ou aryle pouvant être halogénés une ou plusieurs fois ; et
R^{a}, R^{b}, R^{c} représentent, chacun indépendamment les uns des autres, un groupe alkyle en C₁₋₄, aryle ou aryl-C₁₋₃-alkyle, les groupes alkyle ou aryle pouvant être substitués une ou plusieurs fois avec des atomes halogènes ;
les groupes aryle mentionnés dans la définition des résidus étant des groupes phényle
ou naphtyle, de préférence des groupes phényle ;
et X, Z, R¹ à R⁵ ayant la signification donnée dans les revendications 1 à 13,
les résidus R^{8a}, R^{8b} ou R^{8c} ne désignant pas un atome d'hydrogène sont éliminés, et
si nécessaire, un résidu protecteur utilisé dans la réaction précédemment décrite est à nouveau séparé et/ou
si on le souhaite, on prépare un dérivé du composé ainsi obtenu de formule générale I de façon ciblée au niveau d'un groupe hydroxy ou celui-ci est substitué et/ou
si on le souhaite, on sépare un composé ainsi obtenu de formule générale I en ses stéréoisomères et/ou
on convertit un composé ainsi obtenu de formule générale I en son sel, en particulier pour l'utilisation pharmaceutique en son sel physiologiquement acceptable.
